# EUROPEAN PATENT APPLICATION

(11) **EP 4 296 274 A1**
(43) Date of publication of application: **27.12.2023**
(21) Application number: 22382596.9
(22) Date of filing: 23.06.2022
(51) Int. Cl.: C07K 7/06, C07K 7/08, C07K 14/00, C07K 19/00, A61K 47/00, A61K 38/00

(54) **PEPTIDES FOR INTRACELLULAR DELIVERY**

(71) Applicant: Universidade de Santiago de Compostela, 15782 Santiago de Compostela (ES)
(72) Inventor: MONTENEGRO GARCÍA, Javier, 15782 Santiago de Compostela (ES); JUANES CARRASCO, María Luisa, 15782 Santiago de Compostela (ES); LOSTALÉ SEIJO, Irene, 15782 Santiago de Compostela (ES); FUERTES GARCÍA, Alberto, 15782 Santiago de Compostela (ES); LOZA GARCÍA, Mabel, 15782 Santiago de Compostela (ES); MOREIRA ALVAREZ, David, 15782 Santiago de Compostela (ES)
(74) Representative: Hoffmann Eitle

(57) **Abstract**

The present invention relates to short peptides or salts thereof which can be modified with appropriate hydrophobic tails to generate amphiphilic molecules useful for the intracellular delivery of molecules of biological interest. Thus, the invention also relates to the amphiphilic molecules and to the complexes between said amphiphilic molecules and the molecules of biological interest. In addition, the invention refers to the use of said peptides, said amphiphilic molecules and said complexes for the delivery of molecules of biological interest. Finally, the invention relates to said peptides or salts thereof, said amphiphilic molecules and said complexes for use in medicine, particularly for use as a vaccine. Methods for the preparation of the amphiphilic molecules and the complexes of the invention are also contemplated.

## Description

### FIELD OF THE INVENTION

The present invention relates to short peptides or salts thereof which can be modified with appropriate hydrophobic tails to generate amphiphilic compounds useful for the intracellular delivery of molecules of biological interest.

### BACKGROUND ART

Delivery of functional nucleic acids and/or proteins into cells continues to be a major challenge in chemistry, biology and materials science. New methodologies and recent breakthroughs have renewed the interest in the discovery and development of new tools for efficient intracellular delivery, such as functional nucleic acids and/or protein delivery. However, despite all the advances that have already been achieved in the last few years, currently used techniques suffer from important limitations. These could be related to the nucleic acid or protein cargo, or to the delivery vehicle. The nucleic acid cargo suffers from either potential permanent recombination of plasmids, from the sensitivity towards nuclease degradation of nucleic acids such as ssRNA and from the problematic immunological response that can be triggered by any kind of nucleic acid. The delivery vehicle can be either a viral or a non-viral vector. Although very efficient and in some cases also selective, viral vectors have important limitations related to their potential biosafety concerns, their immunogenicity and the low levels of the DNA packaging capacity. As an alternative, different non-viral vectors have been developed to achieve and improve nucleic acid and protein delivery, which include lipids, peptides, nanoparticles, polymers and supramolecular systems. However, non-viral vectors still present important barriers and limitations such as the poor packaging and protection of the cargo, the low stability of the resulting complex, the immune response, the escape from the endocytic route and, most importantly, the low efficiency and the high cytotoxicity. In this context, short cationic peptides have been described in the literature as suitable non-viral vectors when modified with tails of an appropriate chemistry. For example, I. Louzao, R. Garcia-Fandino and J. Montenegro, Hydrazone-modulated peptides for efficient gene transfection, J. Mater. Chem. B, 2017, describes hydrazone formation to modulate the transfection activity of a parent linear peptide in combination with a plasmid DNA cargo. Similarly, I. Lostalé-Seijo, et al., Peptide/Cas9 nanostructures for ribonucleoprotein cell membrane transport and gene edition, Chem. Sci., 2017, reports a supramolecular strategy for the direct delivery of Cas9 ribonucleoprotein by an amphiphilic penetrating peptide that was prepared by hydrazone bond formation between a cationic peptide scaffold and a hydrophobic aldehyde tail. These contributions suggest that there is a need in the art for the development of peptide based non-viral vectors for a wide range of applications in the intracellular delivery of cargos of biological interest.

### SUMMARY OF THE INVENTION

The inventors have found peptides or salts thereof which can be modified with hydrophobic tails to generate amphiphilic compounds useful for the intracellular delivery of molecules of biological interest.

In a first aspect the invention relates to a peptide or a salt thereof
a) wherein the peptide or salt thereof has a length of 7 to 21 amino acids,
b) wherein the peptide or salt thereof comprises basic amino acid residues selected from the group consisting of (i) arginine (R), (ii) histidine (H) and (iii) reactive basic amino acid residues containing a reactive group on its side chain, and optionally hydrophobic amino acid residues selected from the group consisting of alanine (A), valine (V), leucine (L) and isoleucine (I), wherein, if present, the hydrophobic amino acids are less than 50% of the total number of amino acids in the peptide,
c) wherein the number of arginines in the peptide or salt thereof is from 2 to 10,
d) wherein the number of histidines in the peptide or salt thereof is from 0 to 3,
e) wherein the number of reactive basic amino acids residues containing a reactive residue in its side chain in the peptide or salt thereof is 3,
f) wherein the number of hydrophobic amino acids in the peptide or salt thereof is 0 to 8,
g) wherein in the peptide amino acid sequence there are not more than two consecutive amino acids of the same residue, except for the reactive basic amino acid residue containing a reactive group in its side chain and the amino acid arginine, in which cases there can be a maximum of three consecutive arginines and/or a maximum of three consecutive reactive basic amino acid residues containing a reactive group in its side chain, and
h) wherein the amino acids comprised in the peptide or salt thereof are L-amino acids and/or D-amino acids, preferably wherein all of the amino acids comprised in the peptide are L-amino acids or all of the amino acids comprised in the peptide are D-amino acids.

Preferably, the peptide of the invention or salt thereof is a cationic peptide. The peptide of the present invention or salt thereof is suitable for forming the amphiphiles of the present invention, as described below. In a preferred embodiment, the amino acid in the *N*-terminal end of the peptide is arginine (R).

Preferably, the reactive basic amino acid residue containing a reactive group on its side chain is an amino acid as defined in Formula (I):

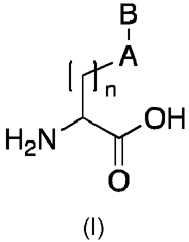

Wherein, if B is not present, A is a reactive group, preferably a primary amine. If B is present, A is preferably an amide bond, but could also be a secondary amine or any other functional group, such as a functional group arising from an originally basic amino acid. B, if present, is a spacer linked to a reactive group, as described herein, and n is 1, 2, 3, 4, 5, 6, 7, 8, or more, preferably n is 1, 2, 3 or 4, more preferably 3 or 4.

In a second aspect, the invention relates to an amphiphilic molecule comprising or, alternatively, consisting of:
a) A peptide or salt thereof as defined in the first aspect of the invention and
b) Three hydrophobic tails,
wherein each hydrophobic tail is connected to the peptide or salt thereof by a covalent bond formed by a reaction between (i) the reactive group in the side chain of the reactive basic amino acid residue in the peptide or salt thereof and (ii) a precursor of the hydrophobic tail, wherein the precursor of the hydrophobic tail comprises the hydrophobic tail and a second reactive group which can react with the reactive group in the side chain of the reactive basic amino acid residue of the peptide or salt thereof.

In a third aspect, the invention refers to a complex comprising
a) at least one amphiphilic molecule as defined in the second aspect of the invention, and
b) at least one molecule of biological interest.

In a further aspect, the invention refers to the use of the peptide of the present invention or salt thereof, the amphiphilic molecule of the present invention, or the complex of the present invention, for the delivery of a molecule of biological interest.

In still a further aspect, the invention refers to the peptide of the present invention or salt thereof, the amphiphilic molecule of the present invention, or the complex of the present invention for use in medicine (as a medicament).

In yet a further aspect, the invention refers to the peptide of the present invention or salt thereof, the amphiphilic molecule of the present invention, or the complex of the present invention, for use as a vaccine, or for use as an immunogenic composition. The present invention further provides a vaccine comprising the peptide of the present invention or salt thereof, the amphiphilic molecule of the present invention or the complex of the present invention. Hence, the molecule of biological interest may be capable of generating an immune response in a subject.

In another aspect, the invention refers to a method for the preparation of the amphiphilic molecule according to the invention, wherein the method comprises contacting a solution of the peptide of the invention in an organic solvent with a solution of a precursor of the hydrophobic tail, wherein the precursor of the hydrophobic tail comprises a reactive group which reacts with the reactive group in the side chain of the reactive basic amino acid residues in the peptide or salt thereof under conditions adequate for the formation of a covalent bond between the reactive group of the precursor of the hydrophobic tail and the reactive group of the peptide or salt thereof, wherein said conditions consist of:
- acidic media, when the reactive group in the hydrophobic tail precursor is an aldehyde, or
- basic media and in the presence of a carboxylic acid activator reagent, when the reactive group in the hydrophobic tail precursor is an alcohol or a carboxylic acid.

In a further aspect, the invention refers to a method for the preparation of a complex according to the invention comprising contacting a solution of the amphiphilic molecule of the invention with a solution of at least one molecule of biological interest, in a suitable medium, under conditions adequate for the formation of a complex between the amphiphilic molecule and the molecule of biological interest.

In a further aspect, the invention relates to a method for the *in vitro* delivery of a molecule of biological interest to a cell population comprising
(i) contacting a first solution of the amphiphilic molecule of the invention in a suitable solvent and a second solution comprising the molecule of biological interest in a suitable solvent under conditions adequate for the formation of a complex between the amphiphilic molecule and the at least one molecule of biological interest; and
(ii) adding the complex obtained in step (i) to the cell population under conditions adequate for the delivery of the molecule of biological interest to the cell population.

In another aspect, the invention relates to a method for obtaining a library comprising at least two different amphiphilic molecules according to the present invention, the method comprising contacting at least "n" peptides of the present invention or salts thereof in an organic solvent with at least "3n" hydrophobic tail precursors, each precursor comprising a hydrophobic tail and a reactive group which reacts with the reactive group in the side chain of the basic amino acids in the peptide or salt thereof, wherein the contacting is carried out under conditions adequate for the formation of a covalent bond between the hydrophobic tail precursors and the reactive groups in the side chain of the basic amino acids in the peptide or salt thereof. "n" corresponds to a natural number, such as 1, 2, 3, 4, 5, 6, 7, 8, 9, 10, etc. Preferably, the contacting step of the method for obtaining a library of amphiphilic molecules is performed by contacting at least one type of peptide or salts thereof with at least one type of hydrophobic tail precursor, separately. For instance, in the method for obtaining a library comprising at least two different amphiphilic molecules according to the present invention, at least 2 peptides or salts thereof as defined in the present invention are contacted in an organic solvent with at least 6 hydrophobic tail precursors, as described above.

In addition, the invention relates to a library comprising at least two different amphiphilic molecules, each amphiphilic molecule comprising
(i) a peptide of the invention or salt thereof and
(ii) three hydrophobic tails,
wherein the hydrophobic tails are connected to the peptide by a bond formed by a reaction between the reactive group in the side chain of the reactive basic amino acid residues of the peptide or salt thereof and a precursor of the hydrophobic tails, wherein each precursor of the hydrophobic tail contains said hydrophobic tail and a second reactive group which can react with the reactive group in the side chain of the reactive basic amino acid residue in the peptide or salt thereof, and wherein one amphiphilic molecule differs at least from other amphiphilic molecule in the peptide and/or in the hydrophobic tails.

Finally, the invention relates to a method for the identification of an amphiphilic molecule suitable for the delivery of a molecule of biological interest into a cell, the method comprising:
(i) contacting the molecule of biological interest with the library of the present invention under conditions adequate for the binding of the amphiphilic molecule and the molecule of biological interest;
(ii) optionally selecting the amphiphilic molecules that are capable of binding to the molecule of biological interest from those amphiphilic molecules which do not bind to the molecule of biological interest, and
(iii) screening for an amphiphilic molecule suitable for the delivery of the molecule of biological interest into a cell.

### BRIEF DESCRIPTION OF THE FIGURES

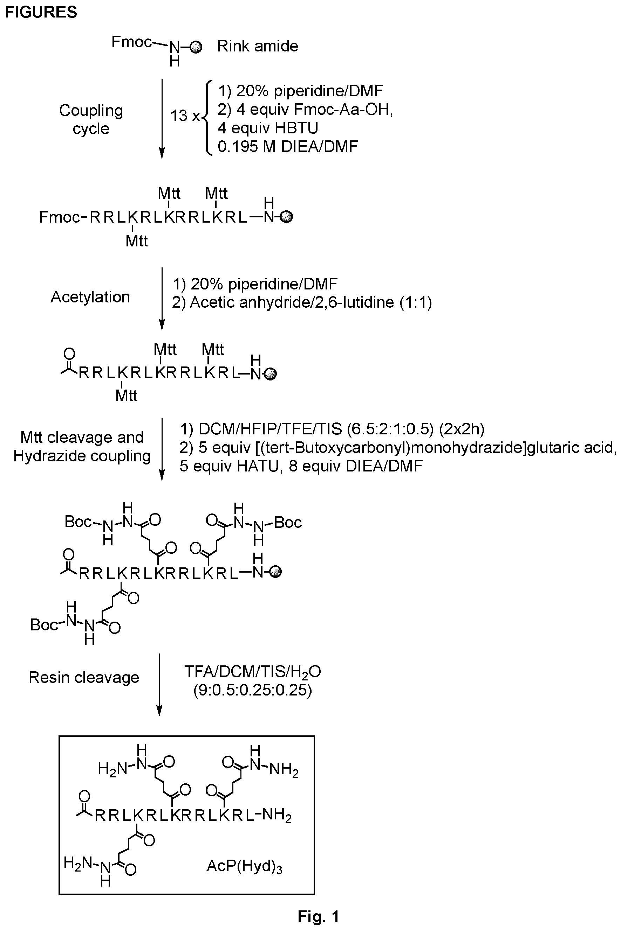

**Figure 1****.** Schematic overview of the peptide synthesis procedure.

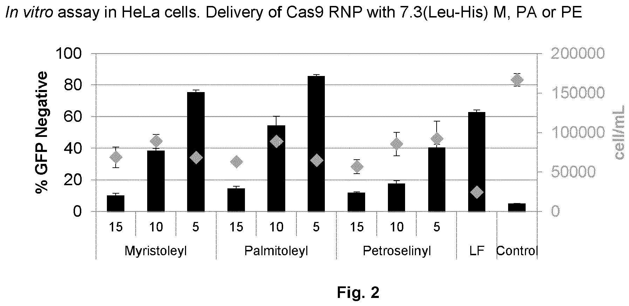

**Figure 2****.** Percentage of editing (in bars, left axis) in HeLa-EGFP cells incubated with Cas9 ribonucleoprotein with an EGFP-targeting gRNA using the amphiphiles comprising 7.3(Leu-His) peptide with myristoleyl, palmitoleyl or petroselinyl aldehydes. After 3 days, EGFP negative cells (bars; left axis) and cell concentration (dots; right axis) were quantified by flow cytometry. ([Cas9] = 50 nM, amphiphiles concentration in µM).

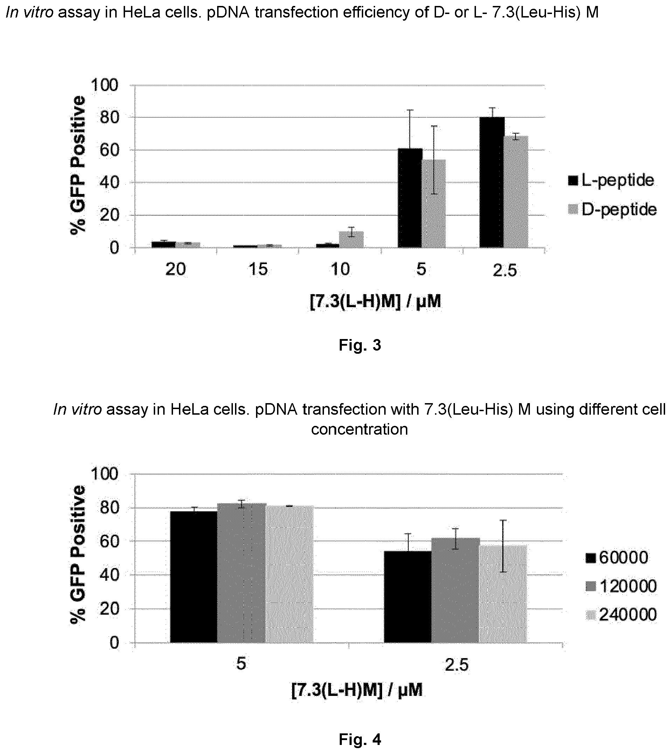

**Figure** 3. EGFP pDNA transfection efficacy with amphiphile comprising 7.3(Leu-His) peptide synthesized using D- and L-amino acids and myristoleyl aldehyde (7.3(L-H) M) in HeLa cells.
**Figure 4****.** EGFP pDNA transfection efficacy with amphiphile comprising 7.3(Leu-His) peptide and myristoleyl aldehyde (7.3(L-H) M) in HeLa cells at different concentrations (60,000, 120,000 and 240,000 cell/mL).

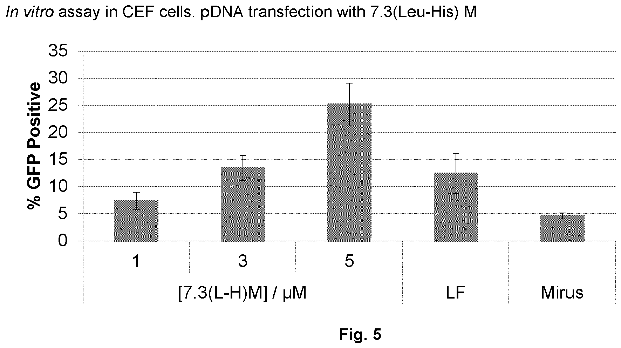

**Figure 5****.** EGFP pDNA transfection efficacy (% GFP-positive cells) with amphiphile comprising 7.3(Leu-His) peptide and myristoleyl aldehyde (7.3(L-H) M) at different concentrations, compared with Lipofectamine 2000 (LF) and *Trans*IT-LT1 Transfection Reagent (Mirus) in CEF (chicken embryo fibroblasts).

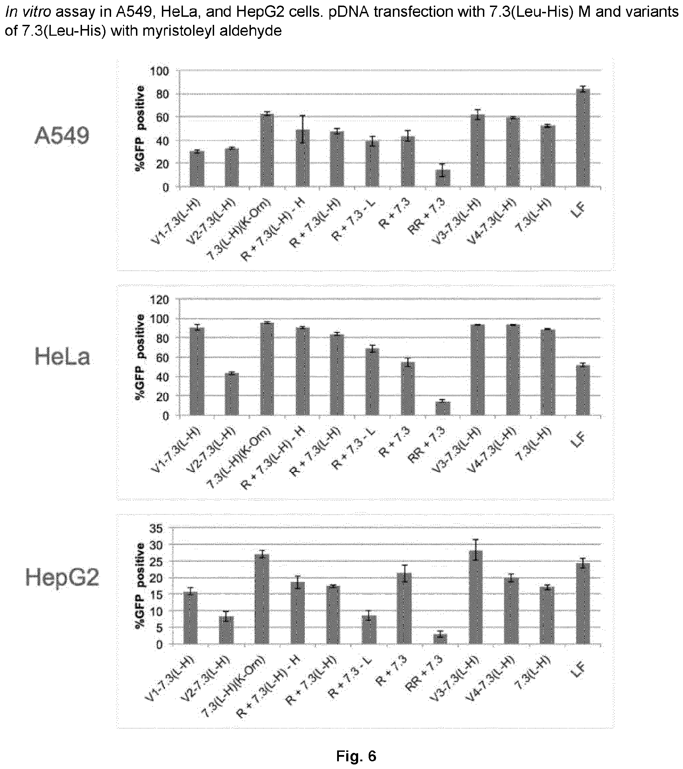

**Figure 6****.** EGFP pDNA transfection efficacy with amphiphiles (myristoleyl aldehyde hydrophobic tails were used in all cases) comprising peptide 7.3(L-H) and other short peptides structurally-derived from 7.3(L-H), in A549, HeLa and HepG2 cell lines. 5 µM of amphiphile and 2 ng/µL pDNA per well were used in the transfection experiments. "LF" = Lipofectamine^{®} 2000 commercial reagent.

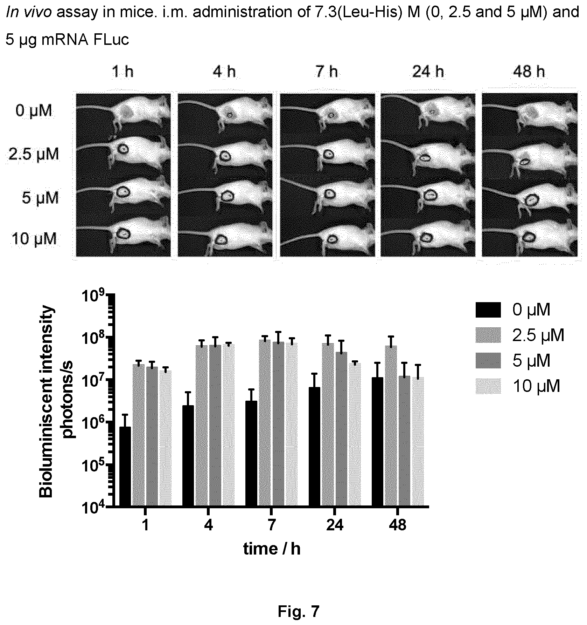

**Figure 7****.** *In vivo* mRNA transfection. Representative bioluminescence images and quantification following i.m. (intramuscular) injection of naked FLuc mRNA (0 µM) or 7.3(Leu-His) M amphiphile/mRNA complexes (2.5, 5 and 10 µM) at different times (1 h, 4 h, 7 h, 24 h and 48 h). 5 µg FLuc mRNA were used in all conditions. 7.3(Leu-His) M refers to a 7.3(Leu-His) cationic peptide linked to myristoleyl aldehyde.

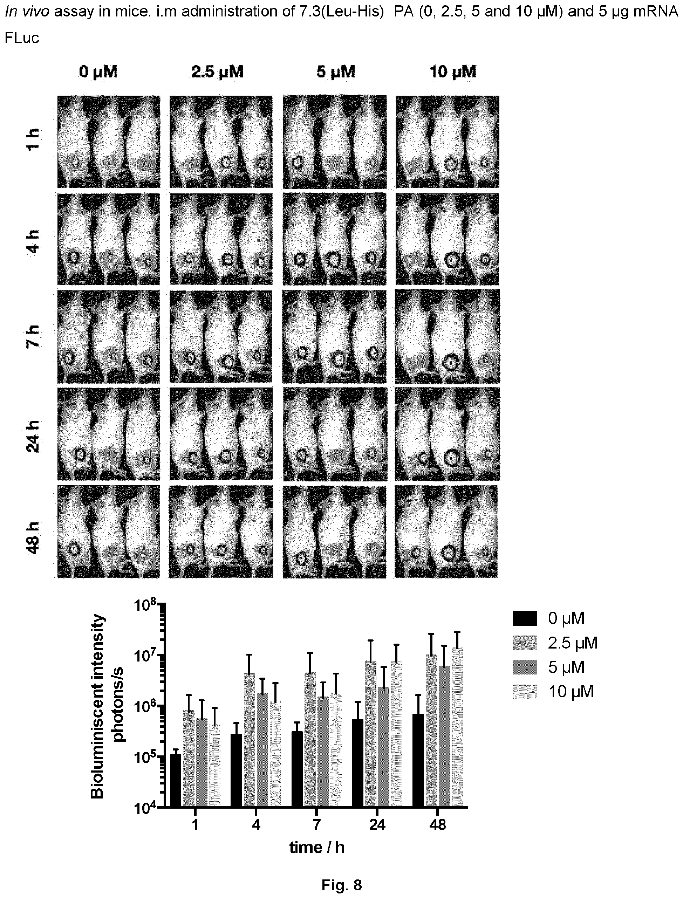

**Figure 8**. *In vivo* mRNA transfection. Representative bioluminescence images and quantification following i.m. (intramuscular) injection of naked FLuc mRNA (0 µM) or 7.3(Leu-His) PA amphiphile/mRNA complexes (2.5, 5 and 10 µM) at different times (1 h, 4 h, 7 h, 24 h and 48 h). 5 µg FLuc mRNA were used in all conditions. 7.3(Leu-His) PA refers to a 7.3(Leu-His) cationic peptide linked to palmitoleyl aldehyde.

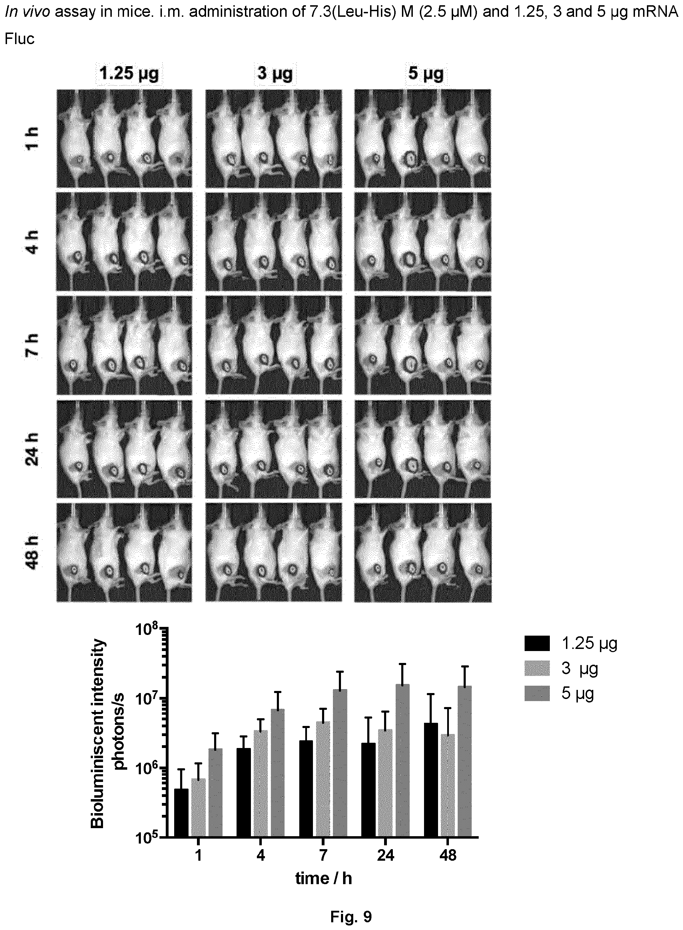

**Figure 9**. *In vivo* mRNA transfection. Representative bioluminescence images and quantification following i.m. (intramuscular) injection of FLuc mRNA/amphiphile complexes at different times (1 h, 4 h, 7 h, 24 h and 48 h). 1.25, 3 and 5 µg FLuc mRNA were used as indicated. 2.5 µM of 7.3(Leu-His) M were used in all conditions.

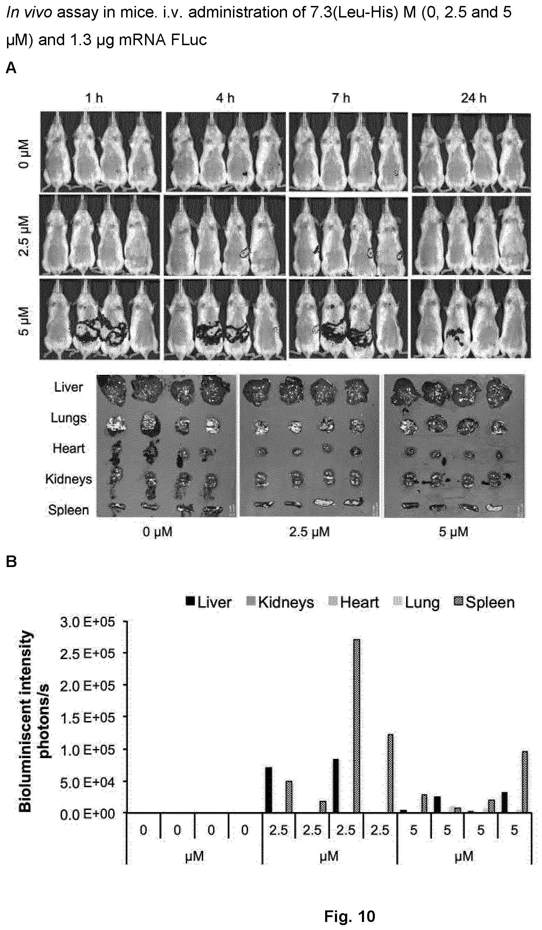

**Figure 10**. A) *In vivo* mRNA transfection. Representative bioluminescence images of animals and extracted organs following i.v. (intravenous) injection of FLuc mRNA/ amphiphile complexes (0, 2.5 and 5 µM of 7.3(Leu-His) M) at different times (1 h, 4 h, 7 h and 24 h). 1.3 µg FLuc mRNA were used in all conditions. B) Quantification of the organs' bioluminescence

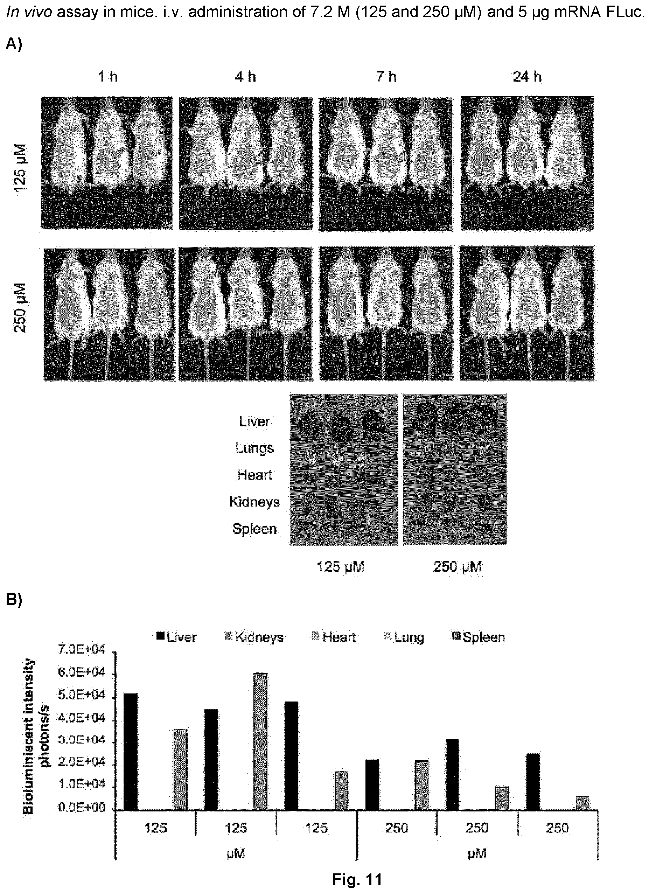

**Figure 11**. A) *In vivo* mRNA transfection. Representative bioluminescence images of mice and extracted organs following i.v. (intravenous) injection of FLuc mRNA/ amphiphile complexes (125 and 250 µM of 7.2 M) at different times (1 h, 4 h, 7 h and 24 h). 5 µg FLuc mRNA/mouse were used. B) Quantification of the organs' bioluminescence.

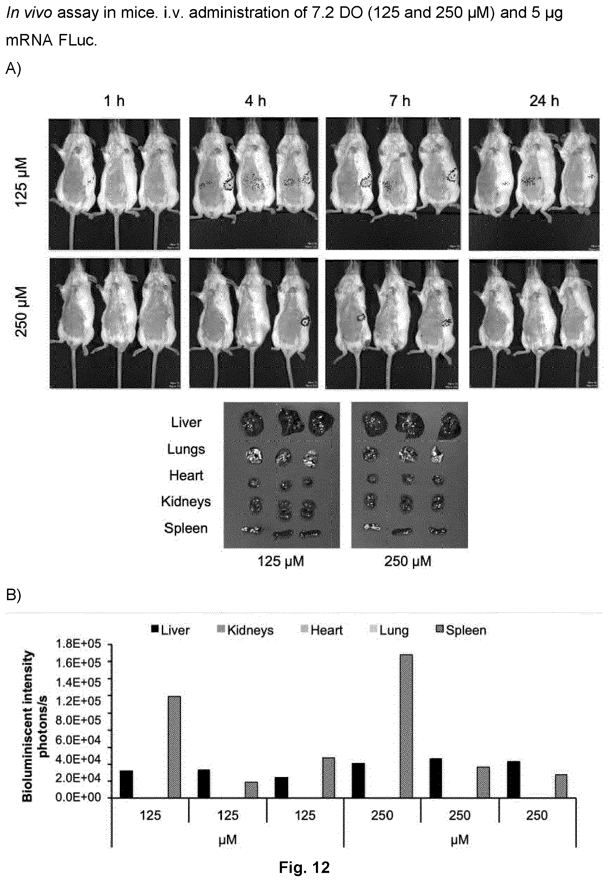

**Figure 12**. A) *In vivo* mRNA transfection. Representative bioluminescence images of mice and extracted organs following i.v. (intravenous) injection of FLuc mRNA/ amphiphile complexes (125 and 250 µM of 7.2 DO) at different times (1 h, 4 h, 7 h and 24 h). 5 µg FLuc mRNA/mouse were used. B) Quantification of the organs' bioluminescence

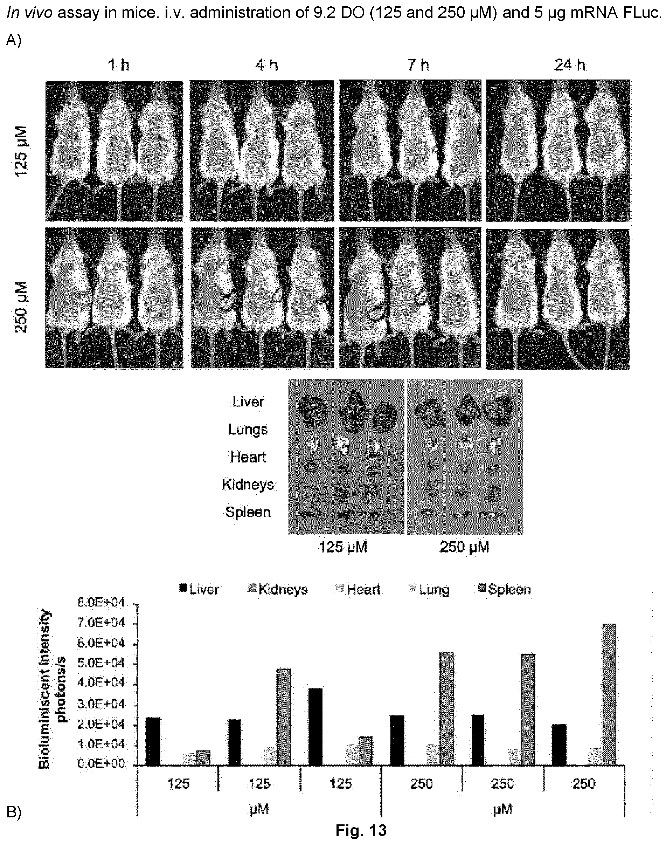

**Figure 13**. A) *In vivo* mRNA transfection. Representative bioluminescence images of mice and extracted organs following i.v. (intravenous) injection of FLuc mRNA/ amphiphile complexes (125 and 250 µM of 9.2 DO) at different times (1 h, 4 h, 7 h and 24 h). 5 µg FLuc mRNA/mouse were used. B) Quantification of the organs' bioluminescence.

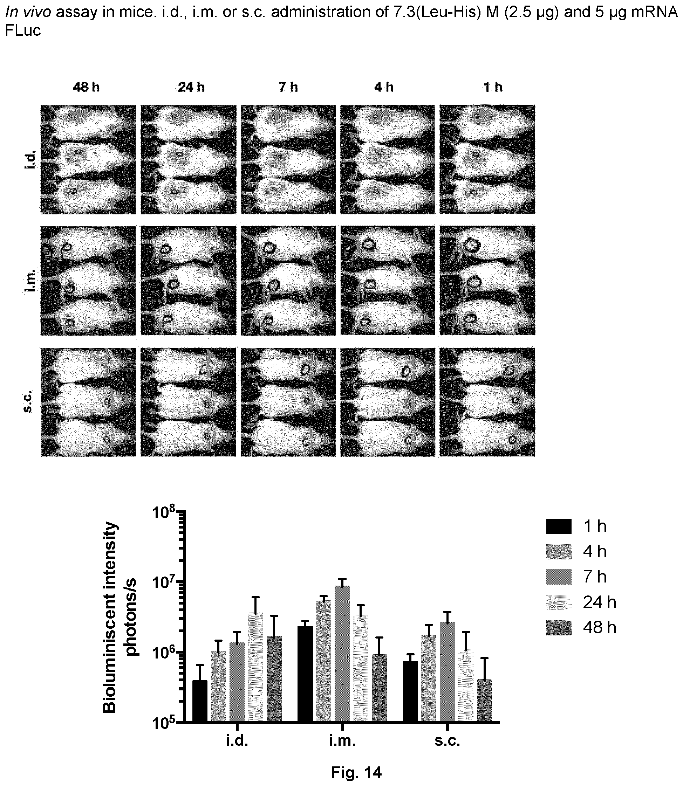

**Figure 14****.** *In vivo* mRNA transfection. Representative bioluminescence images and quantification following i.d. (intradermal), i.m. (intramuscular), or s.c. (subcutaneous) injection of FLuc mRNA/amphiphile complexes at different times (1 h, 4 h, 7 h, 24 h and 48 h). 2.5 µM of 7.3(Leu-His) M and 5 µg FLuc mRNA were used in all conditions.

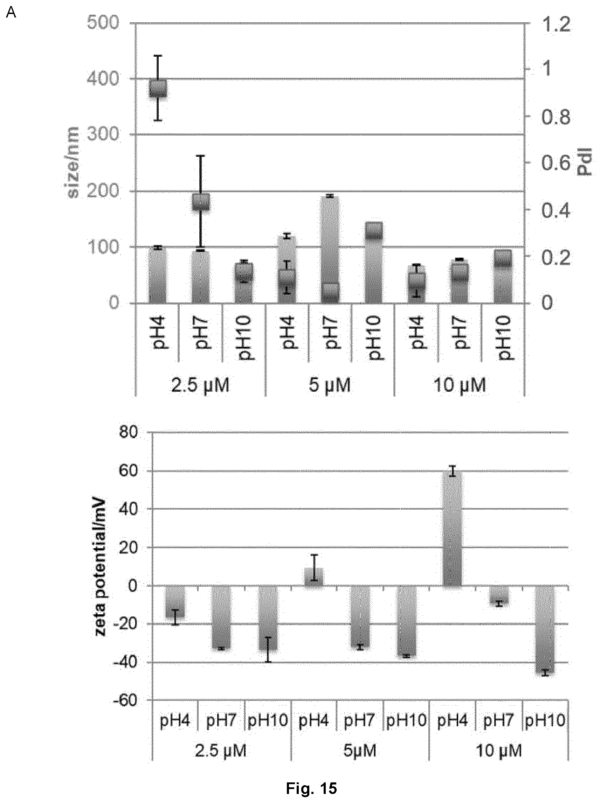

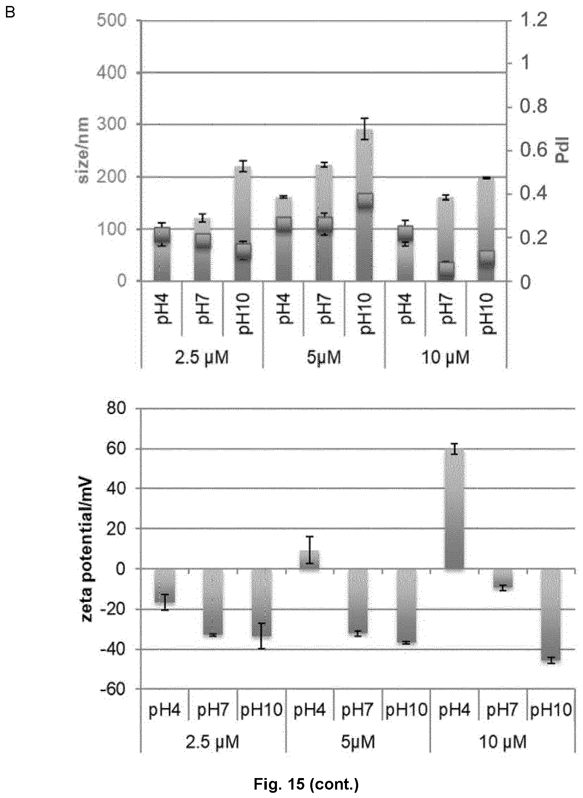

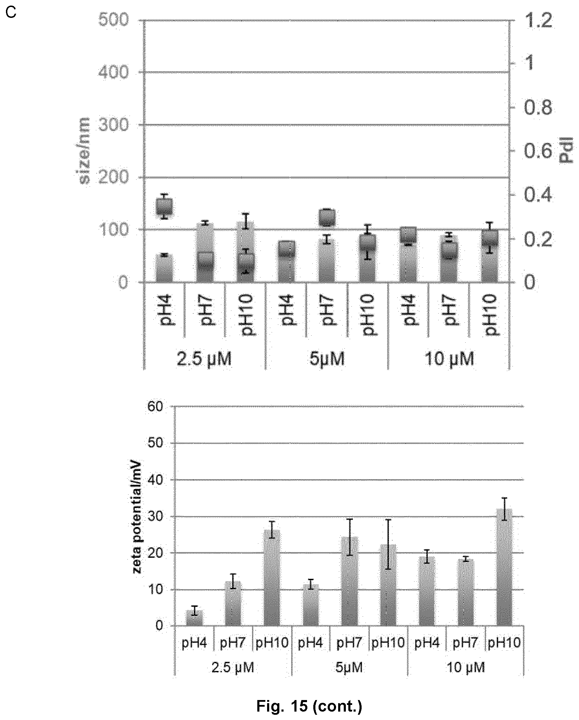

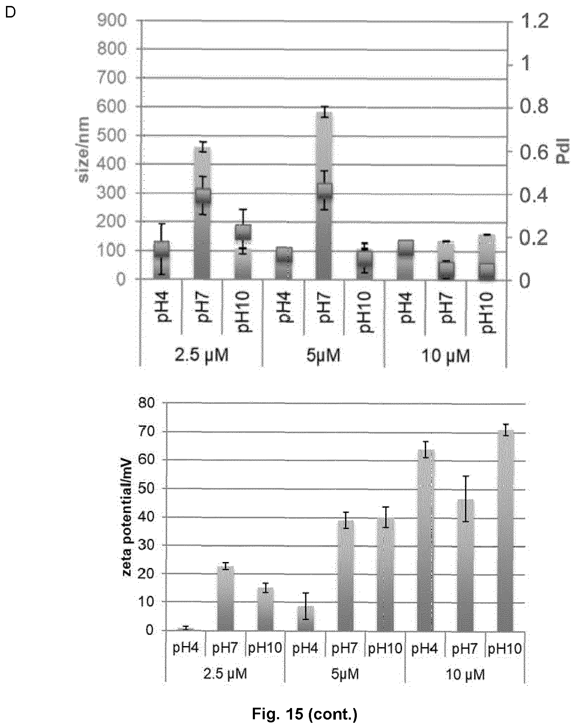

**Figure 15****. A-** DLS results for amphiphile complex 7.3 M + pDNA (100 ng, *in vitro* conditions). Complexes were prepared by mixing freshly prepared amphiphilic molecule 7.3 M at different concentrations (x axis) with 100 ng of EGFP pDNA in water for 30 minutes. Then, pH was adjusted to different values (4-10) and particle size (upper panel: bars represent mean diameter, left axis; square dots represent PDI, right axis) and zeta potential (lower panel) measurements were recorded. **B-** DLS results for amphiphile complex 7.3(L-H) M + pDNA (100 ng, *in vitro* conditions). Complexes were prepared by mixing freshly prepared amphiphilic molecule 7.3(L-H) M at different concentrations (x axis) with 100 ng of EGFP pDNA in water for 30 minutes. Then, pH was adjusted to different values (4-10) and particle size (upper panel: bars represent mean diameter, left axis; square dots represent PDI, right axis) and zeta potential (lower panel) measurements were recorded. C- DLS results for amphiphile complex 7.3 M + mRNA (50 ng, *in vitro* conditions). Complexes were prepared by mixing freshly prepared amphiphilic molecule 7.3 M at different concentrations (x axis) with 50 ng of EGFP mRNA in water for 30 minutes. Then, pH was adjusted to different values (4-10) and particle size (upper panel: bars represent mean diameter, left axis; square dots represent PDI, right axis) and zeta potential (lower panel) measurements were recorded. D- DLS results for amphiphile complex 7.3(L-H) M + mRNA (50 ng, *in vitro* conditions). Complexes were prepared by mixing freshly prepared amphiphilic molecule 7.3(L-H) M at different concentrations (x axis) with 50 ng of EGFP mRNA in water for 30 minutes. Then, pH was adjusted to different values (4-10) and particle size (upper panel: bars represent mean diameter, left axis; square dots represent PDI, right axis) and zeta potential (lower panel) measurements were recorded.

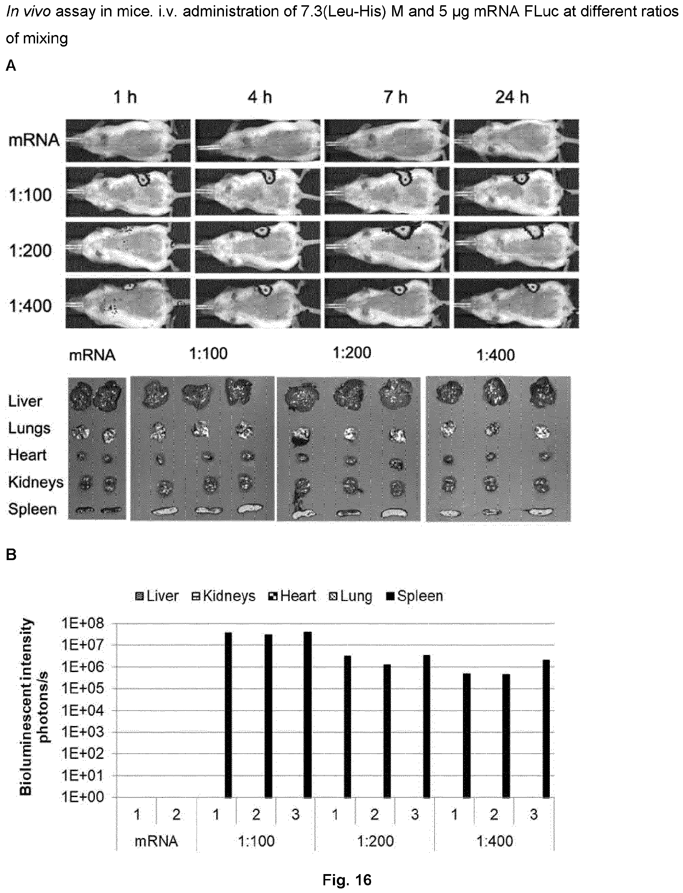

**Figure 16****.** *In vivo* mRNA transfection. **A-** Representative bioluminescence images of mice and extracted organs following i.v. (intravenous) injection of FLuc mRNA/ amphiphile complexes (125 µM of 7.3(Leu-His) M) at different times (1 h, 4 h, 7 h and 24 h). 5 µg FLuc mRNA/mouse were used. The ratios 1:100, 1:200 and 1:400 represent the volume ratio between the DMSO phase containing the amphiphile molecule and the aqueous buffer containing the mRNA cargo during the formulation step. "mRNA" labelled mice were injected with mRNA cargo without any carrier. **B-** Quantification of the organs' bioluminescence.

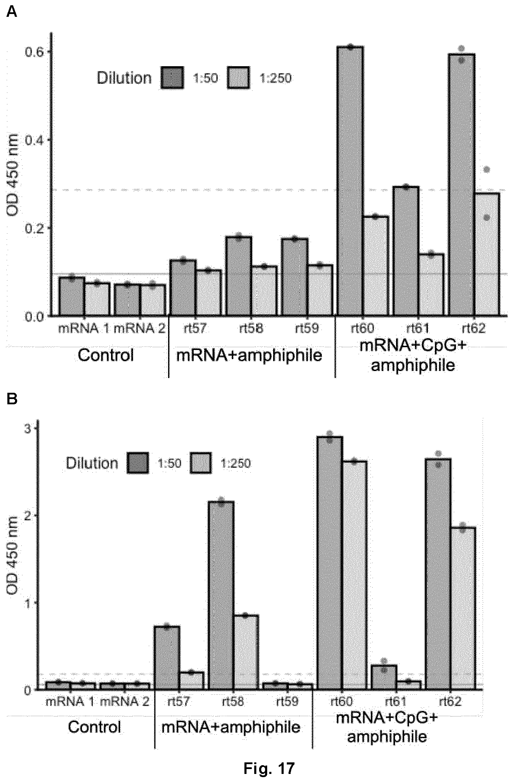

**Figure 17****.** Mice were immunised i.v. with 7.3(Leu-His) M/SARS-CoV-2 spike mRNA complexes at day 0 and day 21, and the serum levels of S1-specific IgG determined by ELISA, 14 days (Figure 17 A) and 35 days (2 weeks after boost) (Figure 17 B). Data shown as optical densities (duplicates) of two dilutions of serum from mice immunised with two different amphiphile/mRNA formulations (rt57-62) and control mice only treated with naked mRNA (mRNA1-2). Limit of detection (dashed line) was established as three times the blank value. rt57-rt62 refer to the specific mice included in the group (arbitrary).

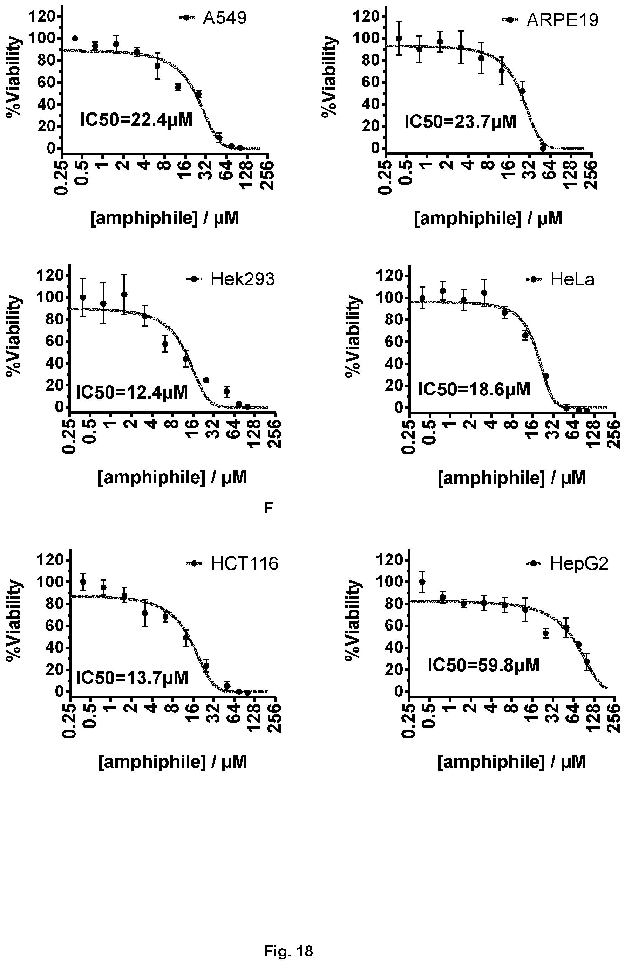

**Figure 18****.** MTT cell viability assay in different cell lines after incubation in the presence of increasing concentrations (0-100 µM) of amphiphile 7.3(L-H) M.

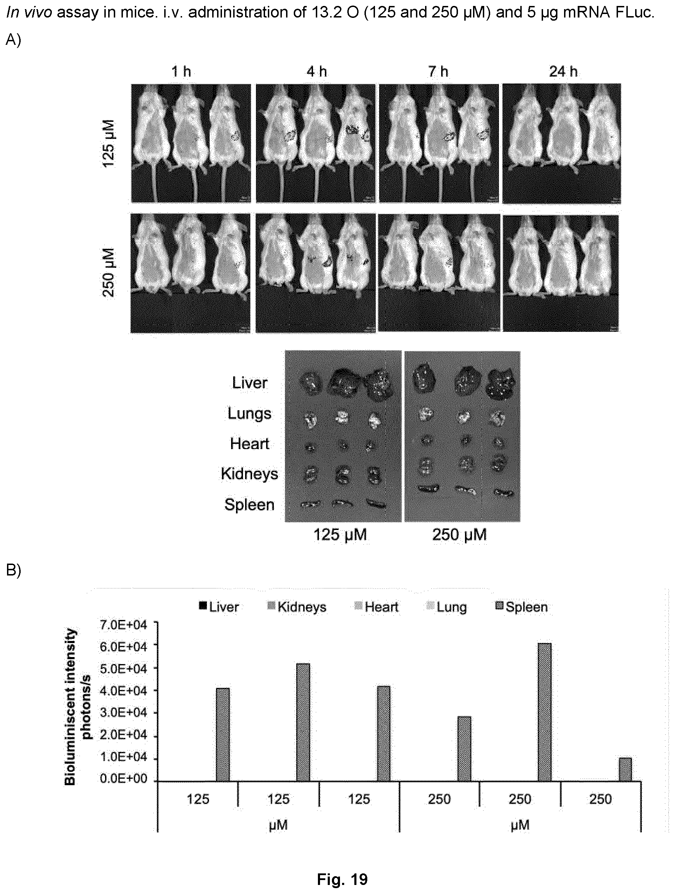

**Figure 19**. A) *In vivo* mRNA transfection. Representative bioluminescence images of mice and extracted organs following i.v. (intravenous) injection of FLuc mRNA/amphiphile complexes (125 and 250 µM of amphiphile 13.2 O, complexed with 1.3 µg FLuc mRNA in all cases) at different times (1 h, 4 h, 7 h and 24 h). B) Quantification of the organs' bioluminescence.

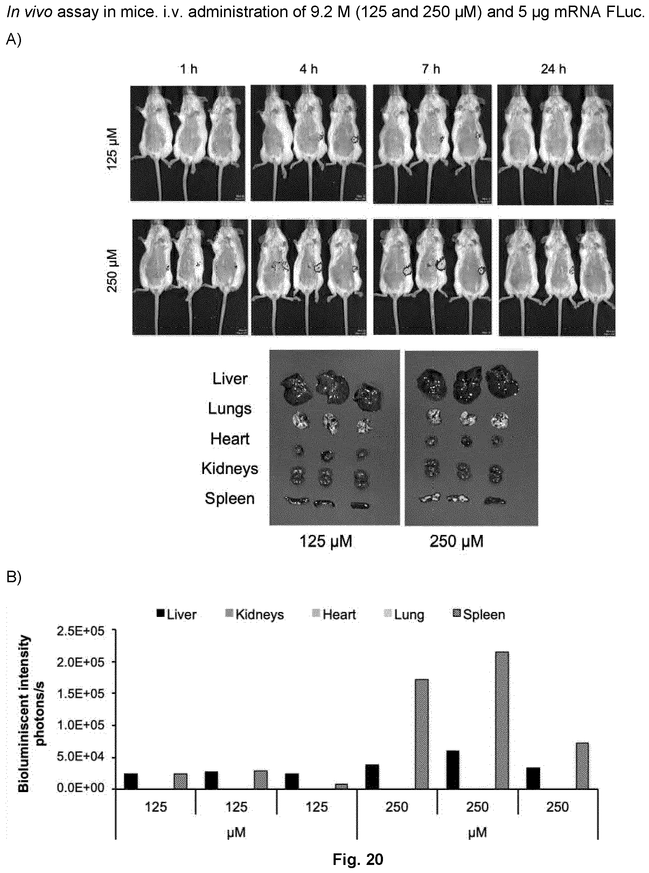

**Figure 20**. A) *In vivo* mRNA transfection. Representative bioluminescence images of mice and extracted organs following i.v. (intravenous) injection of Fluc mRNA/ amphiphile complexes (125 and 250 µM of 9.2 M) at different times (1 h, 4 h, 7 h and 24 h). 5 µg Fluc mRNA/mouse were used. B) Quantification of the organs' bioluminescence.

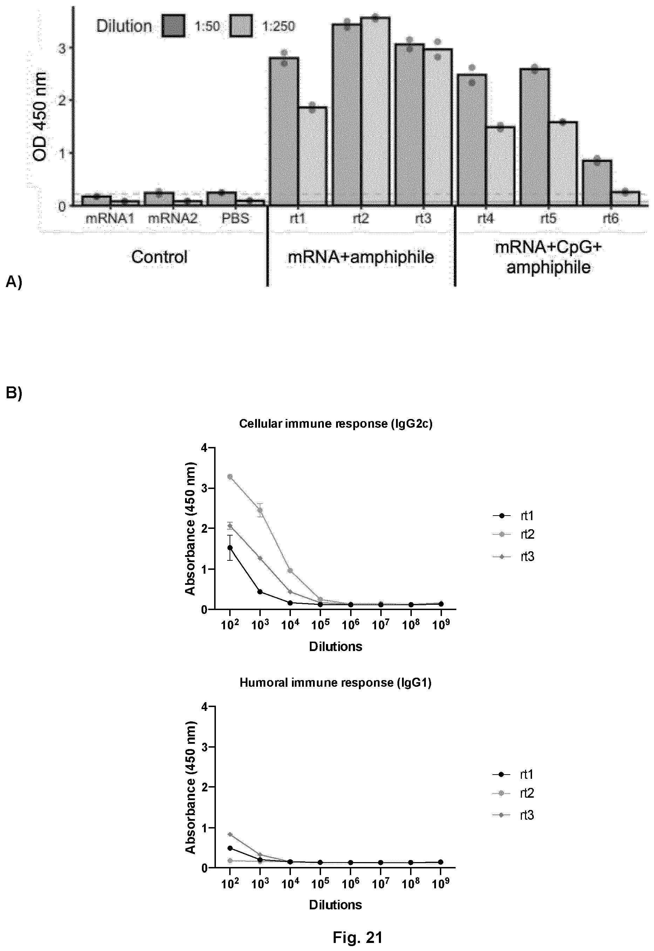

**Figure 21****.** Mice were immunised i.v. with 7.3(Leu-His) M/OVA mRNA complexes at day 0 and day 21, and the serum levels of OVA-specific IgG determined by ELISA at day 35 (2 weeks after boost) (Figure 21 A). Data shown as optical densities (duplicates) of two dilutions of serum from mice immunised with two different formulations: amphiphile/mRNA alone (rt1-3) or in the additional presence of CpG ODN adjuvant (rt4-6) and control mice treated only with naked mRNA (mRNA1-2) or PBS (PBS). Limit of detection (dashed line) was established as three times the blank value. Rt1-6 refer to the specific mice included in the group (arbitrary). B) Serial dilutions of sera obtained at day 35 (dilution factors ranging from 10² to 10⁹) were analysed by ELISA using secondary anti-mouse IgG1 or IgG2c antibodies (1:2,000) for titer determination of antibody isotypes and characterization of the type of immune response.

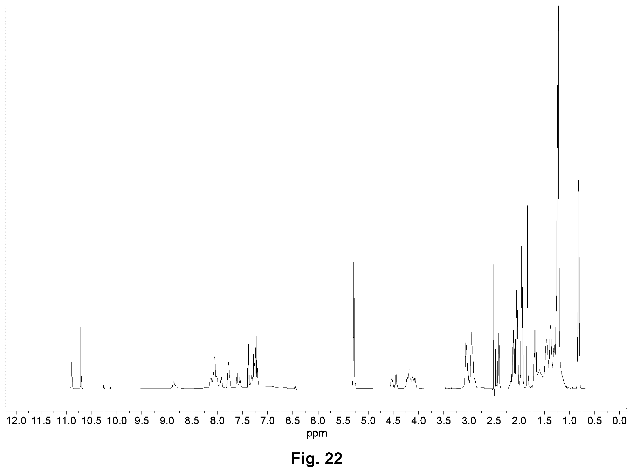

**Figure 22****.** ¹H NMR spectrum of amphiphile 7.3(L-H) M recorded in DMSO-d6.

### DETAILED DESCRIPTION OF THE INVENTION

The inventors have found peptides which can be modified with appropriate hydrophobic tails to generate amphiphilic compounds useful for the intracellular delivery of molecules of biological interest.

### The peptide

In a first aspect, the invention relates to a peptide or a salt thereof
- wherein the peptide or salt thereof has a length of 7 to 21 amino acids,
- wherein the peptide or salt thereof comprises basic amino acids selected from the group consisting of arginine (R), histidine (H) and reactive basic amino acid residues containing a reactive group in its side chain,
- wherein the peptide or salt thereof optionally comprises hydrophobic amino acids selected from the group consisting of alanine (A), valine (V), leucine (L) and isoleucine (I), wherein, if present, the hydrophobic amino acids are less than 50% of the total number of amino acids in the peptide or salt thereof,
- wherein the number of arginines in the peptide is from 2 to 10,
- wherein the number of histidines in the peptide is from 0 to 3,
- wherein the number of reactive basic amino acid residues containing a reactive group in its side chain in the peptide or salt thereof is three,
- wherein the number of hydrophobic amino acids in the peptide or salt thereof is from 0 to 8,
- wherein in the peptide amino acid sequence there are not more than two consecutive amino acids of the same residue, except for the reactive basic amino acid residue containing a reactive group in its side chain and the amino acid arginine, in which cases there can be a maximum of three consecutive arginines and/or a maximum of three consecutive reactive basic amino acid residues containing a reactive group in its side chain, and
- wherein the amino acids comprised in the peptide or salt thereof are L-amino acids and/or D-amino acids, preferably wherein all of the amino acids comprised in the peptide or salt thereof are L-amino acids or wherein all of the amino acids comprised in the peptide or salt thereof are D-amino acids.

Preferably, the peptide or salt thereof of the present invention is a cationic peptide. Preferably, the amino acid in the *N*-terminal end of the peptide of the present invention or salt thereof is arginine.

Preferably, in the peptide amino acid sequence, there are not more than two consecutive hydrophobic amino acids.

The peptide of the present invention or salt thereof is suitable for forming the amphiphilic molecules of the present invention, as described below.

The term "peptide", as used herein, refers to a sequence of amino acids, analogues or mimetics having substantially similar or identical functionality, wherein the one or more amino acids, analogues or mimetics are linked to each other by means of a peptide bond. The term "peptide" also includes analogues having synthetic and natural amino acids linked by peptide bonds. A peptide, as used herein and in the claims, is also intended to include analogues, derivatives, salts, retro-inverso isomers, mimics, mimetics, or peptidomimetics thereof. For example, a peptidic structure of a modulator of the invention may be further modified to increase its stability, bioavailability, solubility, etc. "Analog", "derivative" and "mimetic" include molecules which mimic the chemical structure of a peptidic structure and retain the functional properties of the peptidic structure. Approaches to designing peptide analogues, derivatives and mimetics are known in the art. For example, see Farmer, P. S. in Drug Design (E. J. Ariens, ed.) Academic Press, New York, 1980, vol. 10, pp. 119-143; Ball, J. B. and Alewood, P. F. (1990) J. Mol. Recognition 3:55; Morgan, B. A. and Gainor, J. A. (1989) Ann. Rep. Med. Chem. 24:243; and Freidinger, R. M. (1989) Trends Pharmacol. Sci. 10:270. See also Sawyer, T. K. (1995) Peptidomimetic Design and Chemical Approaches to Peptide Metabolism in Taylor, M. D. and Amidon, G. L. (eds.) Peptide-Based Drug Design: Controlling Transport and Metabolism, Chapter 17; Smith, A. B 3rd, et al. (1995) J. Am. Chem. Soc. 117:11113-11123; Smith, A. B 3rd, etal. (1994) J. Am. Chem. Soc. 116:9947-9962; and Hirschmann, R., et al. (1993) J. Am. Chem. Soc. 115:12550-12568. A "derivatve" (e.g., a peptide or amino acid) includes forms in which one or more reactive groups on the compound have been derivatised with a substituent group. Examples of peptide derivatives include peptides in which an amino acid side chain, the peptide backbone, or the amino- or carboxy-terminus has been derivatised (e.g., peptidic compounds with methylated amide linkages). An "analogue" of a compound X includes compounds which retain chemical structures necessary for functional activity, yet which also contains certain chemical structures which differ. An example of an analogue of a naturally-occurring peptide is a peptide which includes one or more non-naturally-occurring amino acids. A "mimetic" of a compound includes compounds in which chemical structures of the compound necessary for functional activity have been replaced with other chemical structures which mimic the conformation of the compound. Examples of peptidomimetics include peptidic compounds in which the peptide backbone is substituted with one or more benzodiazepine molecules (see e.g., James, G. L. et al. (1993) Science 260:1937-1942). In a particular embodiment, the *N*-terminal end of the peptide is terminated by an acyl group and the C-terminal end of the peptide is terminated by an amine group. In another particular embodiment, the acyl group is an acetyl group and the amine group is a primary amine group.

The salts of the peptide of the present invention include salts with physiologically acceptable acid addition salt. Examples of such salts are peptide salts with inorganic acids (e.g., hydrochloric acid, phosphoric acid, hydrobromic acid or sulfuric acid, etc.) and peptide salts with organic acids (e.g., acetic acid, trifluoroacetic acid, formic acid, propionic acid, fumaric acid, maleic acid, succinic acid, tartaric acid, citric acid, malic acid, oxalic acid, benzoic acid, methanesulfonic acid or benzenesulfonic acid, etc.).

Amino acids are organic compounds that contain amino (-NH₃⁺) and carboxylate (-CO₂⁻) functional groups, along with a side chain (R group) specific to each amino acid. Hence, technically, any organic compound with an amine (-NH₂) and a carboxylic acid (-COOH) functional group is an amino acid. The term "amino acid" refers to naturally occurring and synthetic amino acids, as well as amino acid analogues and amino acid mimetics that function in a manner similar to the naturally occurring amino acids. Amino acids may be referred to herein by either their commonly known three letter symbols or by the one-letter symbols recommended by the IUPAC-IUB Biochemical Nomenclature Commission. Nucleotides, likewise, may be referred to by their commonly accepted single-letter codes. The term "amino acid" refers to proteogenic (i.e., amino acids that are incorporated biosynthetically into proteins during translation) and non-proteinogenic amino acids (such as ornithine, (Orn, O), which has two basic groups). The genetic code encodes 20 standard amino acids for incorporation into proteins during translation. In addition, there are two extra proteinogenic amino acids: selenocysteine and pyrrolysine. The term "amino acid" includes naturally occurring amino acids (Ala, Arg, Asn, Asp, Cys, Gin, Glu, Gly, His, Ile, Leu, Lys, Met, Phe, Pro, Ser, Thr, Trp, Tyr, Val), and non-natural or unusual amino acids. The amino acids are preferably in the L configuration, but also D configuration, or mixtures of amino acids in the D and L configurations. The term "natural amino acids" comprises aliphatic amino acids (e.g., glycine, alanine, valine, leucine and isoleucine), hydroxylated amino acids (e.g., serine and threonine), sulphured amino acids (e.g., cysteine and methionine), dicarboxylic amino acids and their amides (e.g., aspartic acid, asparagine, glutamic acid and glutamine), amino acids having two basic groups (e.g., lysine, arginine, and histidine), aromatic amino acids (e.g., phenylalanine, tyrosine and tryptophan) and cyclic amino acids (e.g., proline).

As used herein the term "non-natural or unusual amino acid" refers to amino acids that are not naturally encoded amino acids, i.e., non-proteinogenic amino acids which can occur naturally in plant or bacteria post-translationally or which are chemically synthesized. Illustrative non-limiting examples of modified or uncommon amino acids include 2-aminoadipic acid, 3-aminoadipic acid, beta-alanine, 2-aminobutyric acid, 4-aminobutyric acid, 6-aminocaproic acid, 2-aminoheptanoic acid, 2-aminoisobutyric acid, 3-aminoisobutyric acid, 2-aminopimelic acid, 2,4-diaminobutyric acid (Dab), desmosine, 2,2'-diaminopimelic acid, 2,3-diaminopropionic acid (Dap), N-ethylglycine, N-ethylasparagine, hydroxylysine, allohydroxylysine, 3-hydroxyproline, 4-hydroxyproline, isodesmosine, alloisoleucine, N-methylglycine, N-methylisoleucine, 6-N-methyllysine, N-methylvaline, norvaline, norleucine, ornithine, and the like.

The peptide of the present invention or salt thereof comprises basic amino acids selected from the group consisting of arginine (R), histidine (H) and 3 reactive basic amino acid residues containing a reactive group in its side chain.

In the context of the present application, "a reactive basic amino acid residue containing a reactive group in its side chain" refers to any originally basic amino acid (e.g., Lys, Orn, Dab, Dap) which contains a group in its side chain capable of forming a covalent bond (see below).

A "reactive basic amino acid residue" refers preferably to basic amino acid residues comprising a primary amine on its side chain, such as, e.g., lysine, ornithine, 2,4-diaminobutyric acid, 2,3-diaminopropionic acid or any other basic amino acid which has a primary amine on its side chain. A primary amine is an amine in which the amino group (the -NH₂ moiety) is directly bonded to only one carbon, which cannot be a carbonyl group carbon. In these cases, the reactive basic amino acid residue originally has a positive net charge at neutral pH, since the reactive group in its side chain is the primary amine of the amino acid.

A primary amine is a reactive group and, as such, a basic amino acid comprising a primary amine on its side chain is a reactive basic amino acid which contains a reactive group on its side chain.

Hence, the reactive basic amino acid residue containing a reactive group in its side chain may be a basic amino acid residue comprising a primary amine on its side chain, wherein the primary amine is the reactive group in the side chain. This would be the case of, e.g., a native Lys or a native Orn, as described above, and the reactive basic amino acid residue containing a reactive group in its side chain would be positively charged at neutral pH. In addition, the reactive group in the side chain may be different from a primary amine. In this case, the reactive group may be, for example, covalently linked (directly or by means of a spacer) to the primary amine which is preferably present in the reactive basic amino acid residue. This would be the case, e.g., of a modified Lys or a modified Orn, i.e, a Lys or an Orn wherein a reactive group is covalently linked (directly or by means of a spacer) to their primary amine. This is the preferred embodiment.

Preferably, the spacer is a linear carbon chain which comprises from 1 to 10 carbon atoms, such as from 2 to 10 carbon atoms, more preferably from 2 to 6 carbon atoms, even more preferably 4 carbon atoms. In a preferred embodiment the spacer comprises, on one side, e.g., a carboxylic acid suitable for forming an amide bond with a primary amine in the side chain of the basic reactive amino acid. In addition, on the other side, the spacer is linked to the reactive group (the "first reactive group") as described herein. In a preferred embodiment, the spacer is linked to a reactive group selected from a hydrazide group, an amino group, a carboxylic acid, or an aminooxy-carboxylic acid group, as described herein.

Therefore, the spacer (which is linked to the first reactive group) reacts with the side chain of the reactive basic amino acid residue (which comprises preferably a primary amine), e.g., by forming an amide bond. Hence, in this case, the reactive basic amino acid comprises, bonded to its side chain, through the spacer, a reactive group which is preferably a hydrazide or an aminooxy-carboxylic acid, as described herein. In this scenario, it may be that the original positive charge at neutral pH of the reactive basic amino acid (e.g., Lys or Orn) is lost because of the reaction with the spacer. Nevertheless, in the context of the present invention, as also described above, such a modified amino acid is also referred to as "reactive basic amino acid", because it was originally (before the reaction with the spacer linked to the reactive group) a basic amino acid and because it has a reactive group in its side chain.

For the purposes of the present application, when one amino acid in the peptide of the present invention or salt thereof comprises a reactive group on its side chain, the amino acid may be referred to with its 3- or 1 -letter symbols and an asterisk (*) (e.g., K* for a native or modified lysine, i.e., a native lysine or a lysine with a reactive group on its side chain which reactive group is not the primary amine of the lysine).

Hence, as described above, in preferred embodiments, the reactive group covalently linked (directly or by means of a spacer) to the primary amine of the basic amino acid may not necessarily be an amine, but can be any reactive group suitable for forming covalent bonds with other reactive groups, such as other reactive groups present in precursors of hydrophobic tails, as defined below in detail.

In a particular embodiment, the reactive group present in the side chain of the reactive basic amino acid is selected from the group consisting of a hydrazide group, an amino group, a carboxylic acid, and an aminooxy-carboxylic acid group. Preferably, the reactive group present in the side chain of the reactive basic amino acid is a hydrazide group or an aminooxy-carboxylic acid group. More preferably, the reactive group present in the side chain of the reactive basic amino acid is a hydrazide group.

In a preferred embodiment, the reactive basic amino acid residue containing a reactive group on its side chain is an amino acid as defined in Formula (I): 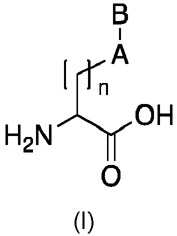 wherein:
If B is not present, A is a reactive group, preferably a primary amine. If B is present, A is preferably an amide bond, but could also be a secondary amine or any other functional group, such as a functional group arising from an originally basic amino acid. B, if present, is a spacer linked to a reactive group, as described herein, and n is 1, 2, 3, 4, 5, 6, 7, 8, or more, preferably n is 1, 2, 3 or 4, more preferably, n is 3 or 4.

Formula (II) represents the reactive basic amino acid residue containing a reactive group on its side chain when it is part of the amino acid sequence in the peptide of the invention: 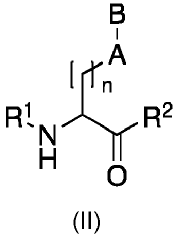 wherein:
R¹ is an amino acid residue comprised in the peptide, as defined herein, an acyl group, preferably an acetyl group, a H or salts thereof;
R² may be an amino acid residue comprised in the peptide, as defined herein, NH₂,OH, or salts thereof. When R² is NH₂, OH, or a salt thereof, the reactive basic amino acid residue is located at the C-terminus of the peptide of the invention;
B may be present or not. If B is not present, A is a reactive group as described herein, preferably a primary amine; if B is present, A is preferably an amide bond, but could also be a secondary amine or any other functional group, such as a functional group arising from an originally basic amino acid;
If present, B is a spacer linked to a reactive group, as described herein; and
n is 1, 2, 3, 4, 5, 6, 7, 8, or more, preferably n is 1, 2, 3, or 4, and more preferably, n is 3 or 4.

In a preferred embodiment, if B is not present, A is a primary amine.

In a preferred embodiment, B is present, and it is preferably a spacer linked to a hydrazide group, an amino group, a carboxylic acid or an aminooxy-carboxylic acid group, as described herein. More preferably, B is present, and it is a spacer linked to a hydrazide group or an aminooxy-carboxylic acid group. Even more preferably, B is present, and it is a spacer linked to a hydrazide group. In preferred embodiments, B is present, and it is a spacer comprising from 1 to 10 carbon atoms, such as from 2 to 10 carbon atoms, more preferably from 2 to 6 carbon atoms, even more preferably 4 carbon atoms, linked to a hydrazide group, an amino group, a carboxylic acid or an aminooxy-carboxylic acid group, more preferably linked to a hydrazide group or an aminooxy-carboxylic acid group, even more preferably linked to a hydrazide group. In preferred embodiments, B is a spacer comprising from 2 to 6 carbon atoms linked to a hydrazide group or an aminooxy-carboxylic acid group. More preferably, B is a spacer comprising 4 carbon atoms linked to a hydrazide group.

In a preferred embodiment B is present and A is an amide bond.

Preferably, the spacer is a linear carbon chain which comprises from 1 to 10 carbon atoms, such as from 2 to 10 carbon atoms, more preferably from 2 to 6 carbon atoms, even more preferably 4 carbon atoms, as described herein. In a preferred embodiment the spacer has, on one side, e.g., a carboxylic acid suitable for forming an amide bond with a primary amine in the side chain of the basic reactive amino acid (A). In addition, on the other side, the spacer is linked to the reactive group (the "first reactive group"), as described herein. In a preferred embodiment, the spacer is linked to a reactive group selected from a hydrazide group, an amino group, a carboxylic acid or an aminooxy-carboxylic acid group, as described herein. Hence, in a preferred embodiment, B is present, and it is a spacer which comprises from 1 to 10 carbon atoms, such as from 2 to 10 carbon atoms, more preferably from 2 to 6 carbon atoms, even more preferably 4 carbon atoms and it is linked to a reactive group as described herein (the "first reactive group"), which is preferably a hydrazide group, an amino group, a carboxylic acid or an aminooxy-carboxylic acid group.

In a further preferred embodiment, the three reactive basic amino acid residues containing a reactive group in its side chain are reactive lysines (K*) or reactive ornithines (O*), or mixtures of them.

The term "reactive lysine", "K*" or "chemically available lysine" refers to the amino acid lysine, C₆H₁₄N₂O₂, which possesses a free side chain amino group (primary amine). In the context of the present invention, as explained in detail above, the primary amine of the side chain of the native lysine may be the reactive group present in the reactive lysine (the "first reactive group"). In addition, in the context of the present invention, as explained in detail above, a reactive group may be covalently linked (directly or by means of a spacer) to the side chain of the native lysine (i.e., to the ε-amino group present in the lysine), which reactive group is preferably a hydrazide group or an aminooxy-carboxylic acid group.

The term "reactive ornithine" or "O*" refers to the non-proteinogenic amino acid with molecular formula C₅H₁₂N₂O₂, which possesses a free side chain amino group (primary amine). In the context of the present invention, as explained in detail above, the primary amine of the native ornithine may be the reactive group present in the reactive ornithine (the "first reactive group"). In addition, in the context of the present invention, as explained in detail above, a reactive group may be covalently linked (directly or by means of a spacer) to the side chain of the native ornithine (i.e., to the δ-amino group present in the ornithine), which reactive group is preferably a hydrazide group or an aminooxy-carboxylic acid group.

Hence, the reactive lysine K* in the peptide of the invention or salt thereof may be a native lysine or a modified lysine, wherein the modified lysine is characterized in that the first reactive group is connected to the ε-amino group in the lysine by an amide bond, said first reactive group and amide bond being preferably connected by a spacer. Preferably, the reactive lysine K* in the peptide of the invention is a modified lysine, as described above.

Hence, the reactive ornithine O* may be a native ornithine or a modified ornithine, wherein the modified ornithine is characterized in that the first reactive group is connected to the δ-amino group in the ornithine by an amide bond, said first reactive group and amide bond being preferably connected by a spacer. Preferably, the reactive ornithine O* in the peptide of the invention is a modified ornithine, as described above.

Preferably, the reactive group present in the side chain of the reactive basic amino acid (i.e., the first reactive group) is hydrazide and it is connected to the ε-amino group in the lysine and/or to the δ-amino group in the ornithine by an amide bond, said hydrazide group and said amide bond being connected by a spacer which is a carbon chain which preferably comprises from 1 to 10 carbon atoms, such as from 2 to 10 carbon atoms, more preferably from 2 to 6 carbon atoms, even more preferably 4 carbon atoms. For instance, and as described in the examples below, the lysine and/or ornithine in the peptide of the invention can be preferably modified with glutaric acid monohydrazide so that an amide bond is formed between the ε-amino group if the amino acid is lysine (or δ-amino group, if the amino acid is ornithine) in the lysine/ornithine and the carboxylic acid in the glutaric acid monohydrazide. Hence, the hydrazide group is linked to the lysine and/or ornithine in the peptide of the invention via an acyl spacer comprising 4 C atoms.

In a particular embodiment, the reactive group (the "first reactive group") present in the side chain of the reactive basic amino acid (which may be linked to a spacer, as described above) is any group that allows bioconjugation by means of click chemistry. In a particular embodiment, the reactive group present in the side chain of the reactive basic amino acid is any group suitable for developing a specific and controllable bioorthogonal reaction. The terms "click chemistry" or "click reaction" are used interchangeably herein and are intended to be consistent with their use in the art. They refer to the class of reactions that are very efficient and selective and can be used to stitch molecules together in high yields, as originally described by Sharpless and co-workers in 2001 (Kolb, H. C., Finn, M. G., & Sharpless, K. B. (2001), Angewandte Chemie International Edition, 40(11), 2004-2021). In the context of the present invention, a bioorthogonal reaction refers to any chemical reaction that can occur inside of living systems without interfering with native biochemical processes. A number of chemical ligation strategies have been developed that fulfil the requirements of bioorthogonality, including without limitation the 1,3-dipolar cycloaddition between azides and cyclooctynes (also termed copper-free click chemistry), between nitrones and cyclooctynes, oxime/hydrazone formation, the tetrazine ligation, the isocyanide-based click reaction, and the quadricyclane ligation.

In a particular embodiment, the reactive group present in the side chain of the reactive basic amino acid is selected from the group consisting of a hydrazide, a linear alkyl amino acid, a carboxylic acid, and an aminooxy-carboxylic acid. Preferably, the reactive group present in the side chain of the reactive basic amino acid is selected from a hydrazide and an aminooxy-carboxylic acid. Even more preferably, the reactive group present in the side chain of the reactive basic amino acid is a hydrazide. In another particular embodiment, the reactive group is linked to a spacer, which is a carbon chain which preferably comprises from 1 to 10 carbon atoms, such as from 2 to 10 carbon atoms, more preferably from 2 to 6 carbon atoms, even more preferably 4 carbon atoms. In this embodiment, the spacer has on one side, e.g., a carboxylic acid suitable for forming an amide bond between the ε-amino group if the amino acid is lysine, or δ-amino group, if the amino acid is ornithine. In addition, on the other side, the spacer is linked to the reactive group, which is selected from the group consisting of a hydrazide, a linear alkyl amino acid, a carboxylic acid and an aminooxy-carboxylic acid. Preferably, the reactive group present in the side chain of the reactive basic amino acid is selected from a hydrazide and an aminooxy-carboxylic acid. Even more preferably, the reactive group is a hydrazide.

Hence, in a preferred embodiment, the first reactive group (which is preferably a hydrazide) is linked to a spacer comprising 5 carbon atoms. In another embodiment, the reactive group is linked to a spacer comprising 4 carbon atoms. For instance, if the reactive group is a carboxylic acid, the reactive group is present, linked to the spacer, as glutaric acid. In a further embodiment, if the reactive group is an aminooxy-carboxylic acid, the reactive group is present, linked to the spacer, as aminooxy-acetic acid. Preferably, the reactive group is hydrazide and it is present, linked to the spacer, as glutaric acid monohydrazide. Hence, the spacer linked to the reactive group is preferably in the form of glutaric acid monohydrazide, glutaric acid or aminooxy-acetic acid, more preferably in the form of glutaric acid monohydrazide.

In an embodiment, the modified lysine or the modified ornithine is obtained by reacting the peptide with a hydrazide carboxylic acid, preferably glutaric acid monohydrazide, or with an aminooxy-carboxylic acid, preferably aminooxy-acetic acid, thereby resulting in the modified lysine or the modified ornithine wherein the reactive group is, respectively, hydrazide and an aminooxy-carboxylic acid group. See "Hydrazone-modulated peptides for efficient gene transfection" (J. Mater. Chem. B (2017), 5:4426-4434) for more details. In a more preferred embodiment, the modified lysine or the modified ornithine is obtained by reacting the peptide with a hydrazide carboxylic acid, preferably glutaric acid monohydrazide, thereby resulting in the modified lysine or the modified ornithine, wherein the reactive group is hydrazide.

The peptide of the invention or salt thereof optionally further comprises at least one hydrophobic amino acid selected from the group consisting of alanine (A), valine (V), leucine (L) and isoleucine (I), preferably leucine (L). If hydrophobic amino acids are present in the peptide of the invention, the hydrophobic amino acids are less than 50%, less than 40%, less than 30%, less than 20%, or less than 10% of the total number of amino acids in the peptide or salt thereof.

In a preferred embodiment, the peptide of the invention or salt thereof has a minimum length of 7 amino acids and a maximum length of 21 amino acids. For instance, the peptide of the invention or salt thereof has a length of 7, 8, 9, 10, 11, 12, 13, 14, 15, 16, 17, 18, 19, 20 or 21 amino acids. More preferably, the peptide of the invention or salt thereof has from 7 to 17 amino acids, even more preferably from 7 to 13 amino acids, such as 7, 9 or 13 amino acids, or from 7 to 9 amino acids. In a preferred embodiment, the peptide of the invention or salt thereof has 7, 9, 13, 17 or 21 amino acids.

The number of R amino acids (arginines) in the peptide or salt thereof is preferably from 2 to 10. Thus, the number of arginines in the peptide or salt thereof is preferably 2, 3, 4, 5, 6, 7, 8, 9 or 10. Preferably, the number of arginines is from 2 to 4.

The number of H amino acids (histidines) in the peptide or salt thereof is preferably from 0 to 3, even more preferably from 0 to 2. Thus, the number of histidines in the peptide or salt thereof is preferably 0, 1, 2 or 3, more preferably 0, 1 or 2.

The number of reactive basic amino acid residues containing a reactive group in its side chain (preferably lysines and/or ornithines, preferably modified lysines and/or modified ornithines, also referred to as K* and/or O*, as described above) in the peptide or salt thereof is three.

The number of hydrophobic amino acids in the peptide or salt thereof is preferably from 0 to 8. Thus, the number of hydrophobic amino acids in the peptide or salt thereof is preferably 0, 1, 2, 3, 4, 5, 6, 7 or 8. In one embodiment, the hydrophobic amino acid is alanine (A). In another embodiment, the hydrophobic amino acid is valine (V). Preferably, the hydrophobic amino acid is leucine (L). In another embodiment, the hydrophobic amino acid is isoleucine (I). In another embodiment, the hydrophobic amino acids present in the peptide or salt thereof are any combination of two or more alanine (A), valine (V), leucine (L) and/or isoleucine (I).

The amino acids comprised in the peptide of the present invention or salt thereof may be L-amino acids or D-amino acids, or combinations thereof. Preferably, all of the amino acids comprised in the peptide of the present invention or salt thereof are L-amino acids or, alternatively, all of the amino acids comprised in the peptide of the present invention or salt thereof are D-amino acids.

In a more preferred embodiment, all of the amino acids comprised in the peptide of the present invention or salt thereof are L-amino acids.

In the peptide of the invention or salt thereof, there are not more than two consecutive amino acids of the same residue, except for the reactive basic amino acid residue containing a reactive group in its side chain (e.g., K* or O*) and the amino acid arginine, in which cases there can be a maximum of three consecutive arginines and/or a maximum of three consecutive reactive basic amino acid residues containing a reactive group in its side chain (e.g., there can be a maximum of three consecutive Arg, RRR, and/or a maximum of three consecutive reactive basic amino acid residues containing a reactive group in its side chain, e.g., K*K*K*, K*O*O*, O*O*O*, O*K*O*, K*O*K*, O*O*K*, O*K*K*, etc.).

In a preferred embodiment, in the peptide of the invention or salt thereof, there are not more than two consecutive hydrophobic amino acids, i.e., there is a maximum of two consecutive hydrophobic amino acids, e.g., AA, VV, AV, VA, LL, LA, VL, II, IL, LI, IA, VI, Al, etc.

Thus, the peptide of the invention or salt thereof may have no more than three adjacent R residues, no more than two adjacent H residues, no more than two adjacent hydrophobic residues and no more than three adjacent reactive basic amino acid residues containing a reactive group in its side chain (e.g., K* or O*).

In a particular embodiment, the peptide of the invention or salt thereof has a length of 7 amino acids, and comprises only basic amino acids selected from the group consisting of R and H and reactive basic amino acid residues containing a reactive group (that is, the peptide comprises no hydrophobic amino acids), wherein the number of R amino acids in the peptide is at least 2, wherein the number of H amino acids in the peptide is 1 or 2, preferably 2, and wherein the number of reactive basic amino acid residues containing a reactive group (preferably K* and/or O*) in the peptide is 3. In a preferred embodiment, the basic amino acid containing a reactive group is selected from K* and O*, preferably K*.

In a particular embodiment, the peptide sequence is selected from the group consisting of Ac-RK*HRK*K*H-NH₂ (SEQ ID NO: 1), Ac-RHK*K*K*HR-NH₂ (SEQ ID NO: 2), and Ac-RK*HHK*K*R-NH₂ (SEQ ID NO: 3), Ac-RO*HRO*O*H-NH₂ (SEQ ID NO: 52), Ac-RRK*HRK*K*-NH₂ (SEQ ID NO: 53); Ac-RO*HRK*K*H-NH₂ (SEQ ID NO: 58) and Ac-RK*HRK*O*H-NH₂ (SEQ ID NO: 59).

In another embodiment, the peptide of the invention or salt thereof has a length of at least 7 amino acids and comprises a core sequence RX₁X₂R (SEQ ID NO: 9), wherein any one of X₁ to X₂ is an amino acid independently selected from the group consisting of H, reactive basic amino acid residues containing a reactive group (preferably K* and/or O*) and a hydrophobic amino acid, wherein the hydrophobic amino acid is independently selected from the group consisting of A, V, L and I. In preferred embodiments, the hydrophobic amino acid is L. In the context of the present invention, a "core sequence" means a sequence wherein additional amino acids can be added on the C-terminus of the sequence and/or in the N-terminus of the sequence. For instance, in this embodiment, the peptide of the invention or salt thereof has a core sequence defined by RX₁X₂R (SEQ ID NO: 9) and has a length of at least 7 amino acids. This means that the peptide according to the present embodiment or salt thereof may have a sequence which is RX₁X₂R and at least three additional amino acids in the C-terminus and/or three amino acids in the N-terminus, or a combination of amino acids both in the C- and N-terminus of the peptide, as defined above.

In another embodiment, the peptide of the invention or salt thereof has a length of between 7 and 21 amino acids, preferably between 7 and 17 amino acids, more preferably between 7 and 13 amino acids and even more preferably between 7 and 9 amino acids, such as 7 amino acids, and comprises a core sequence RX₁X₂R (SEQ ID NO: 9), wherein any one of X₁ to X₂ is an amino acid independently selected from the group consisting of H, reactive basic amino acid residues containing a reactive group (preferably K* and/or O*) and a hydrophobic amino acid, wherein the hydrophobic amino acid is independently selected from the group consisting of A, V, L and I. In preferred embodiments, the hydrophobic amino acid is L.

In another embodiment, the peptide of the invention or salt thereof has a length of at least 7 amino acids, at least 8 amino acids, or at least 9 amino acids, and comprises a core sequence RX₁X₂RRX₃X₄ (SEQ ID NO: 4), wherein any one of X₁ to X₄ is an amino acid independently selected from the group consisting of H, reactive basic amino acid residues containing a reactive group (preferably K* and/or O*) and a hydrophobic amino acid, wherein the hydrophobic amino acid is independently selected from the group consisting of A, V, L and I. In preferred embodiments, the hydrophobic amino acid is L. In another embodiment, said peptide has a length of at least 9 amino acids, such as 9, 13 or 21 amino acids. As described above, if the peptide according to this embodiment or salt thereof has more than 7 amino acids, then the further amino acids are added on the C-terminus and/or on the N-terminus of the core sequence defined by SEQ ID NO: 4. Preferably, if the peptide according to this embodiment has more than 7 amino acids, then the further amino acids are added on the C-terminus of the core sequence defined by SEQ ID NO: 4.

In another embodiment, the peptide of the invention or salt thereof has a length of between 7 and 21 amino acids, such as between 9 and 21 amino acids, preferably between 9 and 17 amino acids, more preferably between 9 and 13 amino acids, such as 9 amino acids, and comprises a core sequence RX₁X₂RRX₃X₄ (SEQ ID NO: 4), wherein any one of X₁ to X₄ is an amino acid independently selected from the group consisting of H, reactive basic amino acid residues containing a reactive group (preferably K* and/or O*) and a hydrophobic amino acid, wherein the hydrophobic amino acid is independently selected from the group consisting of A, V, L and I. In preferred embodiments, the hydrophobic amino acid is L.

In a particular embodiment, the peptide of the invention or salt thereof is a peptide of sequence RX₁X₂RRX₃X₄RX₅ (SEQ ID NO: 5), wherein any one of X₁ to X₅ is an amino acid independently selected from the group consisting of H, reactive basic amino acid residues containing a reactive group (preferably K* and/or O*) and a hydrophobic amino acid, wherein the hydrophobic amino acid is independently selected from the group consisting of A, V, L and I. In preferred embodiments, the hydrophobic amino acid is L. In a particular embodiment, the peptide of the invention is a peptide of sequence RRX₁X₂RX₃X₄RRX₅X₆RX₇ (SEQ ID NO: 6), wherein any one of X₁ to X₇ is an amino acid independently selected from the group consisting of H, reactive basic amino acid residues containing a reactive group (preferably K* and/or O*) and a hydrophobic amino acid, wherein the hydrophobic amino acid is independently selected from the group consisting of A, V, L and I. In preferred embodiments, the hydrophobic amino acid is L. In a particular embodiment, the peptide of the invention is a peptide of sequence RX₁X₂X₃RX₄X₅RRX₆X₇RX₈ (SEQ ID NO: 10), wherein any one of X₁ to X₈ is an amino acid independently selected from the group consisting of H, reactive basic amino acid residues containing a reactive group (preferably K* and/or O*) and a hydrophobic amino acid, wherein the hydrophobic amino acid is independently selected from the group consisting of A, V, L and I. In preferred embodiments, the hydrophobic amino acid is L. In a particular embodiment, the peptide of the invention is a peptide of sequence RRX₁X₂RX₃X₄RRX₅X₆RX₇X₈RRX₉ (SEQ ID NO: 7), wherein any one of X₁ to X₉ is an amino acid independently selected from the group consisting of H, reactive basic amino acid residues containing a reactive group (preferably K* and/or O*) and a hydrophobic amino acid, wherein the hydrophobic amino acid is independently selected from the group consisting of A, V, L and I. In preferred embodiments, the hydrophobic amino acid is L. In a particular embodiment, the peptide of the invention is a peptide of sequence RRX₁X₂RX₃X₄RRX₅X₆RX₇X₈RRX₉RRLL (SEQ ID NO: 8), wherein any one of X₁ to X₉ is an amino acid independently selected from the group consisting of H, reactive basic amino acid residues containing a reactive group (preferably K* and/or O*) and a hydrophobic amino acid, wherein the hydrophobic amino acid is independently selected from the group consisting of A, V, L and I. In preferred embodiments, the hydrophobic amino acid is L.

In an embodiment, the peptide of the invention or salt thereof is a peptide of sequence RX₁X₂RRX₃X₄RX₅ (SEQ ID NO: 5), RRX₁X₂RX₃X₄RRX₅X₆RX₇ (SEQ ID NO: 6); RRX₁X₂RX₃X₄RRX₅X₆RX₇X₈RRX₉ (SEQ ID NO: 7) or RRX₁X₂RX₃X₄RRX₅X₆RX₇X₈RRX₉RRLL (SEQ ID NO: 8), wherein any one of X₁ to X₉ is an amino acid selected from the group consisting of H, reactive basic amino acid residues containing a reactive group (preferably K* and/or O*) and a hydrophobic amino acid, wherein the hydrophobic amino acid is selected from the group consisting of A, V, L and I. In preferred embodiments, the hydrophobic amino acid is L.

In a particular embodiment, the peptide or salt thereof is selected from the group consisting of Ac-RK*LRK*K*L-NH₂ (SEQ ID NO: 11), Ac-RK*RRK*K*R-NH₂ (SEQ ID NO: 12), Ac-RK*LRRK*LRK*-NH₂ (SEQ ID NO: 13), Ac-RRLK*RLK*RRLK*RL-NH₂ (SEQ ID NO: 14), Ac-RRLK*RK*LRRLK*RL-NH₂ (SEQ ID NO: 15), Ac-RRK*K*RK*LRRLLRL-NH₂ (SEQ ID NO: 16), Ac-RRHK*RLK*RRLK*RL-NH₂ (SEQ ID NO: 17), Ac-RHLK*RLK*RRLK*RL-NH₂ (SEQ ID NO: 18), Ac-RHLK*RHK*RRLK*RH-NH₂ (SEQ ID NO: 19), Ac-RRLK*RLLRRLK*RLK*RRL-NH₂ (SEQ ID NO: 21), and Ac-RRLK*RLLRRLK*RLK*RRLRRLL-NH₂ (SEQ ID NO: 23).

In a preferred embodiment, the peptide or salt thereof is selected from the group consisting of Ac-RRLK*RLK*RRLK*RL-NH₂ (SEQ ID NO: 14), Ac-RRLK*RK*LRRLK*RL-NH₂ (SEQ ID NO: 15), Ac-RRK*K*RK*LRRLLRL-NH₂ (SEQ ID NO: 16), Ac-RRHK*RLK*RRLK*RL-NH₂ (SEQ ID NO: 17), Ac-RHLK*RLK*RRLK*RL-NH₂ (SEQ ID NO: 18), Ac-RHLK*RHK*RRLK*RH-NH₂ (SEQ ID NO: 19), Ac-RRLK*RLLRRLK*RLK*RRL-NH₂ (SEQ ID NO: 21), Ac-RRLK*RLLRRLK*RLK*RRLRRLL-NH₂ (SEQ ID NO: 23).

In another preferred embodiment, the peptide or salt thereof is selected from the group consisting of Ac-RK*LRK*K*L-NH₂ (SEQ ID NO: 11), Ac-RK*RRK*K*R-NH₂ (SEQ ID NO: 12), Ac-RK*HK*HK*R-NH₂ (SEQ ID NO: 49), Ac-RK*LRRK*LRK*-NH₂ (SEQ ID NO: 13), Ac-RRK*HRK*K*H (SEQ ID NO: 54), RRK*LRK*K* (SEQ ID NO: 55), RRK*LRK*K*L (SEQ ID NO: 56) and RRRK*LRK*K*L (SEQ ID NO: 57).

Preferably, the peptide of the invention or salt thereof is *N*-acylated and/or C-amidated. Even more preferably, the peptide of the invention or salt thereof is *N*-acylated and C-amidated. The term "*N*-acylated peptide" according to the invention refers to a peptide in which the N-terminal residue of the amino acid is acylated, preferably acetylated with acetyl.

The term "C-amidated" refers to peptides in which the terminal carboxyl group is in the form of an amide group (-CO-NH₂) instead of a carboxyl group (-COOH).

As discussed above, the peptide of the invention or salt thereof may comprise L- and/ or D-amino acids. Preferably, the amino acids comprised in the peptide of the invention or salt thereof are all L-amino acids. However, the amino acids comprised in the peptide of the invention or salt thereof may all be D-amino acids.

All the terms and embodiments described elsewhere herein are equally applicable to these aspects of the invention.

### The amphiphilic molecule

In a second aspect, the invention relates to an amphiphilic molecule (also referred to "amphiphile" in the present invention) comprising or, alternatively, consisting of:
a) A peptide as defined in the first aspect of the invention or salt thereof, which is preferably a cationic peptide, as described above;
b) Three hydrophobic tails, wherein the three hydrophobic tails are connected to the peptide by a bond formed by a reaction between the reactive group in the side chain of the three basic amino acid residues (the "first reactive group") in the peptide and one or more precursors of the three hydrophobic tails, wherein each precursor comprises the hydrophobic tail and a "second reactive group", wherein the second reactive group can react with the reactive group in the side chain of the three reactive basic amino acid residues in the peptide (the "first reactive group"), as described above.

An "amphiphilic molecule" as used herein is a molecule composed of hydrophilic and hydrophobic groups.

The term "peptide" has been described in detail above and is equally applicable to the amphiphilic molecules according to the invention. All embodiments and preferred forms explained above apply equally to the amphiphilic molecule as defined herein.

As used herein, the term "hydrophobic moiety" or "hydrophobic tail" refers to a group or a molecule that is not attracted to water with significant non-polar surface area at physiological pH and/or salt conditions. This phase separation can be observed via a combination of dynamic light scattering and aqueous NMR measurements. Typically, the hydrophobic moiety is lipophilic, which means that is soluble in or miscible with fats, oils, lipids, and lipophilic non-polar solvents such as hexane or toluene. Suitable hydrophobic tails or hydrophobic moieties for use in the present invention include any hydrophobic group. It is preferably an alkyl group or an alkenyl group, most preferably an alkenyl group. The alkyl group may be substituted and functionalised for example to improve hydrophobicity. The alkyl group may be straight or branched, cyclic or acyclic. Carbon chains may contain one or more oxygen atoms included at one or more positions in the main chain.

In some embodiments, the hydrophobic moiety or hydrophobic tail is an aliphatic carbon chain. The term "aliphatic carbon chain" as used herein refers to hydrocarbons based on chains of carbon atoms. There are three types of aliphatic hydrocarbons. Alkanes are aliphatic hydrocarbons with only single covalent bonds. Alkenes are hydrocarbons that contain at least one C-C double bond, and alkynes are hydrocarbons that contain a C-C triple bond. In a preferred embodiment of the amphiphile of the invention, the hydrophobic moiety comprises alkanes. Suitable aliphatic carbon chains that can be incorporated in the amphiphilic moieties according to the invention are those having at least 2 carbon atoms (C2), at least 3 carbon atoms (C3), at least 4 carbon atoms (C4), at least 5 carbon atoms (C5), at least 6 carbon atoms (C6), at least 7 carbon atoms (C7), at least 8 carbon atoms (C8), at least 9 carbon atoms (C9), at least 10 carbon atoms (C10), at least 12 carbon atoms (C12), at least 14 carbon atoms (C14), at least 16 carbon atoms (C16), at least 18 carbon atoms (C18), at least 20 carbon atoms (C20), at least 22 carbon atoms (C22), at least 24 carbon atoms (C24) or more.

The term "hydrophobic tail precursor" refers to a molecule which contains a hydrophobic tail as defined previously and a reactive group (the "second reactive group" according to the present disclosure) which can react with the reactive group in the side chain of the three reactive basic amino acid residues (the "first reactive group" according to the present disclosure) in the peptide of the present invention, forming a covalent bond. In a particular embodiment, the second reactive group present in the hydrophobic tail precursor, as mentioned within the context of the first reactive group, refers to any entity capable of forming a covalent bond. Thus, the amphiphilic molecule is obtained by conjugating the peptide of the invention with three hydrophobic tail precursors, through the reactive group present in the side chain of the three reactive basic amino acid residues of the peptide ("first reactive group") and the reactive group of the three hydrophobic tail precursors ("second reactive group").

In a preferred embodiment, the three hydrophobic moieties or hydrophobic tails are selected from a C6 aliphatic chain, a C8 aliphatic chain, a C10 aliphatic chain, a C12 aliphatic chain, a myristoleyl group, a palmitoleyl group, a petroselinyl group, an oleyl group, a linoleoyl group, an eicosenoyl group, and a tetracosenoleyl group, or mixtures thereof.

More preferably, the three hydrophobic moieties or hydrophobic tails are selected from a C12 aliphatic chain, a myristoleyl group, a palmitoleyl group and a petroselinyl group, or mixtures thereof. Even more preferably, the three hydrophobic moieties or hydrophobic tails are selected from a myristoleyl group, a palmitoleyl group and a petroselinyl group, or mixtures thereof.

In particular embodiments, the second reactive group present in the hydrophobic tail precursor is selected from the group consisting of an aldehyde, a carboxylic acid and an alcohol.

In some embodiments, wherein the second reactive group is an aldehyde, the hydrophobic tail precursor is selected from the group consisting of hexanal, octanal, decanal, dodecanal, myristoleyl aldehyde, palmitoleyl aldehyde, petroselinyl aldehyde, oleyl aldehyde, linoleoyl aldehyde, eicosenoyl aldehyde, and tetracosenoleyl aldehyde, or mixtures thereof. These precursors will lead to amphiphilic peptides in which the hydrophobic tail is a C6 aliphatic chain, a C8 aliphatic chain, a C10 aliphatic chain, a C12 aliphatic chain, a myristoleyl group, a palmitoleyl group, a petroselinyl group, an oleyl group, a linoleoyl group, an eicosenoyl group, and a tetracosenoleyl group respectively, or mixtures thereof.

In some embodiments, when the second reactive group is a carboxylic acid, the hydrophobic tail precursor is selected from the group consisting of hexanoic acid, octanoic acid, decanoic acid, dodecanoic acid, myristoleic acid, palmitoleic acid, petroselinic acid, oleic acid, linoleic acid, eicosenoic acid, tetracosenoleic acid or mixtures thereof. These precursors will lead to amphiphilic peptides in which the three hydrophobic tails are a C6 aliphatic chain, a C8 aliphatic chain, a C10 aliphatic chain, a C12 aliphatic chain, a myristoleyl group, a palmitoleyl group, a petroselinyl group, an oleyl group, a linoleoyl group, an eicosenoyl group, a tetracosenoleyl group or mixtures thereof, respectively.

In some embodiments, when the second reactive group is an alcohol, the hydrophobic tail precursor is selected from the group consisting of hexanol, octanol, decanol, dodecanol, myristoleyl alcohol, palmitoleyl alcohol, petroselinyl alcohol, oleyl alcohol, linoleoyl alcohol, gondoyl alcohol, tetracosenoleyl alcohol, or mixtures thereof. These precursors will lead to amphiphilic peptides in which the hydrophobic tail is a C6 aliphatic chain, a C8 aliphatic chain, a C10 aliphatic chain, a C12 aliphatic chain, a myristoleyl group, a palmitoleyl group, a petroselinyl group, an oleyl group, a linoleoyl group, an eicosenoyl group, a tetracosenoleyl group, or mixtures thereof, respectively.

Preferably, the second reactive group is an aldehyde and the hydrophobic tail precursor is selected from the group consisting of hexanal, octanal, decanal, dodecanal, myristoleyl aldehyde, palmitoleyl aldehyde, petroselinyl aldehyde, oleyl aldehyde, linoleoyl aldehyde, eicosenoyl aldehyde, and tetracosenoleyl aldehyde, or mixtures thereof, more preferably from dodecanal, myristoleyl aldehyde, palmitoleyl aldehyde and petroselinyl aldehyde, or mixtures thereof, and even more preferably from myristoleyl aldehyde, palmitoleyl aldehyde and petroselinyl aldehyde, or mixtures thereof.

Hence, in preferred embodiments, the amphiphilic molecule of the present invention comprises
(i) a peptide according to the present invention or salt thereof, which is preferably a cationic peptide, which has a length of 7 amino acids and comprises only basic amino acids selected from the group consisting of R and H and reactive basic amino acid residues containing a reactive group (that is, the peptide comprises no hydrophobic amino acids), wherein the number of R amino acids in the peptide is at least 2, wherein the number of H amino acids in the peptide is 1 or 2, preferably 2, and wherein the number of reactive basic amino acid residues containing a reactive group in the peptide is 3, preferably wherein the basic amino acid containing a reactive group is selected from K* and O*, preferably K*; and
(ii) three hydrophobic moieties selected from a C12 aliphatic chain, a myristoleyl group, a palmitoleyl group and a petroselinyl group, more preferably selected from a myristoleyl group, a palmitoleyl group and a petroselinyl group.

In other embodiments, the amphiphilic molecule of the present invention comprises
(i) a peptide according to the present invention or salt thereof, which is preferably a cationic peptide, selected from the group consisting of Ac-RK*HRK*K*H-NH₂ (SEQ ID NO: 1), Ac-RHK*K*K*HR-NH₂ (SEQ ID NO: 2), Ac-RK*HHK*K*R-NH₂ (SEQ ID NO: 3), Ac-RO*HRO*O*H-NH₂ (SEQ ID NO: 52), Ac-RRK*HRK*K*-NH₂ (SEQ ID NO: 53), Ac-RO*HRK*K*H-NH₂ (SEQ ID NO: 58) and Ac-RK*HRK*O*H-NH₂ (SEQ ID NO: 59); and
(ii) three hydrophobic moieties selected from a C12 aliphatic chain, a myristoleyl group, a palmitoleyl group and a petroselinyl group, more preferably selected from a myristoleyl group, a palmitoleyl group and a petroselinyl group.

In another embodiment, the amphiphilic molecule of the present invention comprises
(i) a peptide according to the present invention or salt thereof, which is preferably a cationic peptide, which has a length of at least 7 amino acids and comprises a core sequence RX₁X₂R (SEQ ID NO: 9), wherein any one of X₁ to X₂ is an amino acid independently selected from the group consisting of H, reactive basic amino acid residues containing a reactive group (preferably K* and/or O*) and a hydrophobic amino acid, wherein the hydrophobic amino acid is independently selected from the group consisting of A, V, L and I, preferably wherein the hydrophobic amino acid is L; and
(ii) three hydrophobic moieties selected from a C12 aliphatic chain, a myristoleyl group, a palmitoleyl group and a petroselinyl group, more preferably selected from a myristoleyl group, a palmitoleyl group and a petroselinyl group.

In another embodiment, the amphiphilic molecule of the present invention comprises
(i) a peptide according to the present invention or salt thereof, which is preferably a cationic peptide, which has a length of between 7 and 21 amino acids, preferably between 7 and 17 amino acids, more preferably between 7 and 13 amino acids and even more preferably between 7 and 9 amino acids, such as 7 amino acids, and comprises a core sequence RX₁X₂R (SEQ ID NO: 9), wherein any one of X₁ to X₂ is an amino acid independently selected from the group consisting of H, reactive basic amino acid residues containing a reactive group (preferably K* and/or O*) and a hydrophobic amino acid, wherein the hydrophobic amino acid is independently selected from the group consisting of A, V, L and I, preferably wherein the hydrophobic amino acid is L; and
(ii) three hydrophobic moieties selected from a C12 aliphatic chain, a myristoleyl group, a palmitoleyl group and a petroselinyl group, more preferably selected from a myristoleyl group, a palmitoleyl group and a petroselinyl group.

In another embodiment, the amphiphilic molecule of the present invention comprises
(i) a peptide according to the present invention or salt thereof, which is preferably a cationic peptide, which has a length of at least 7 amino acids, at least 8 amino acids, or at least 9 amino acids, and comprises a core sequence RX₁X₂RRX₃X₄ (SEQ ID NO: 4), wherein any one of X₁ to X₄ is an amino acid independently selected from the group consisting of H, reactive basic amino acid residues containing a reactive group (preferably K* and/or O*) and a hydrophobic amino acid, wherein the hydrophobic amino acid is independently selected from the group consisting of A, V, L and I, preferably wherein the hydrophobic amino acid is L, and preferably wherein the peptide has a length of at least 9 amino acids, such as 9, 13 or 21 amino acids; and
(ii) three hydrophobic moieties selected from a C12 aliphatic chain, a myristoleyl group, a palmitoleyl group and a petroselinyl group, more preferably selected from a myristoleyl group, a palmitoleyl group and a petroselinyl group.

In another embodiment, the amphiphilic molecule of the present invention comprises
(i) a peptide according to the present invention or salt thereof, which is preferably a cationic peptide, which has a length of between 7 and 21 amino acids, such as between 9 and 21 amino acids, preferably between 9 and 17 amino acids, more preferably between 9 and 13 amino acids, such as 9 amino acids, and comprises a core sequence RX₁X₂RRX₃X₄ (SEQ ID NO: 4), wherein any one of X₁ to X₄ is an amino acid independently selected from the group consisting of H, reactive basic amino acid residues containing a reactive group (preferably K* and/or O*) and a hydrophobic amino acid, wherein the hydrophobic amino acid is independently selected from the group consisting of A, V, L and I, preferably wherein the hydrophobic amino acid is L; and
(ii) three hydrophobic moieties selected from a C12 aliphatic chain, a myristoleyl group, a palmitoleyl group and a petroselinyl group, more preferably selected from a myristoleyl group, a palmitoleyl group and a petroselinyl group.

In a further embodiment, the amphiphilic molecule of the present invention comprises
(i) a peptide according to the present invention or salt thereof, which is preferably a cationic peptide, of sequence RX₁X₂RRX₃X₄RX₅ (SEQ ID NO: 5), wherein any one of X₁ to X₅ is an amino acid independently selected from the group consisting of H, reactive basic amino acid residues containing a reactive group (preferably K* and/or O*) and a hydrophobic amino acid, wherein the hydrophobic amino acid is independently selected from the group consisting of A, V, L and I, preferably wherein the hydrophobic amino acid is L; and
(ii) three hydrophobic moieties selected from a C12 aliphatic chain, a myristoleyl group, a palmitoleyl group and a petroselinyl group, more preferably selected from a myristoleyl group, a palmitoleyl group and a petroselinyl group.

In a further embodiment, the amphiphilic molecule of the present invention comprises
(i) a peptide according to the present invention or salt thereof, which is preferably a cationic peptide, of sequence RRX₁X₂RX₃X₄RRX₅X₆RX₇ (SEQ ID NO: 6), wherein any one of X₁ to X₇ is an amino acid independently selected from the group consisting of H, reactive basic amino acid residues containing a reactive group (preferably K* and/or O*) and a hydrophobic amino acid, wherein the hydrophobic amino acid is independently selected from the group consisting of A, V, L and I, wherein preferably the hydrophobic amino acid is L; and
(ii) three hydrophobic moieties selected from a C12 aliphatic chain, a myristoleyl group, a palmitoleyl group and a petroselinyl group, more preferably selected from a myristoleyl group, a palmitoleyl group and a petroselinyl group.

In a further embodiment, the amphiphilic molecule of the present invention comprises
(i) a peptide according to the present invention or salt thereof, which is preferably a cationic peptide, of sequence RX₁X₂X₃RX₄X₅RRX₅X₇RX₈ (SEQ ID NO: 10), wherein any one of X₁ to X₈ is an amino acid independently selected from the group consisting of H, reactive basic amino acid residues containing a reactive group (preferably K* and/or O*) and a hydrophobic amino acid, wherein the hydrophobic amino acid is independently selected from the group consisting of A, V, L and I, wherein preferably the hydrophobic amino acid is L; and
(ii) three hydrophobic moieties selected from a C12 aliphatic chain, a myristoleyl group, a palmitoleyl group and a petroselinyl group, more preferably selected from a myristoleyl group, a palmitoleyl group and a petroselinyl group.

In a further embodiment, the amphiphilic molecule of the present invention comprises
(i) a peptide according to the present invention or salt thereof, which is preferably a cationic peptide, of sequence RRX₁X₂RX₃X₄RRX₅X₆RX₇X₈RRX₉ (SEQ ID NO: 7), wherein any one of X₁ to Xg is an amino acid independently selected from the group consisting of H, reactive basic amino acid residues containing a reactive group (preferably K* and/or O*) and a hydrophobic amino acid, wherein the hydrophobic amino acid is independently selected from the group consisting of A, V, L and I, wherein preferably the hydrophobic amino acid is L; and
(ii) three hydrophobic moieties selected from a C12 aliphatic chain, a myristoleyl group, a palmitoleyl group and a petroselinyl group, more preferably selected from a myristoleyl group, a palmitoleyl group and a petroselinyl group.

In a further embodiment, the amphiphilic molecule of the present invention comprises
(i) a peptide according to the present invention or salt thereof, which is preferably a cationic peptide, of sequence RRX₁X₂RX₃X₄RRX₅X₆RX₇X₈RRX₉RRLL (SEQ ID NO: 8), wherein any one of X₁ to Xg is an amino acid independently selected from the group consisting of H, reactive basic amino acid residues containing a reactive group (preferably K* and/or O*) and a hydrophobic amino acid, wherein the hydrophobic amino acid is independently selected from the group consisting of A, V, L and I, wherein preferably the hydrophobic amino acid is L; and
(ii) three hydrophobic moieties selected from a C12 aliphatic chain, a myristoleyl group, a palmitoleyl group and a petroselinyl group, more preferably selected from a myristoleyl group, a palmitoleyl group and a petroselinyl group.

In a further embodiment, the amphiphilic molecule of the present invention comprises
(i) a peptide according to the present invention or salt thereof, which is preferably a cationic peptide, of sequence RX₁X₂RRX₃X₄RX₅ (SEQ ID NO: 5), RRX₁X₂RX₃X₄RRX₅X₆RX₇ (SEQ ID NO: 6), RRX₁X₂RX₃X₄RRX₅X₆RX₇X₈RRX₉ (SEQ ID NO: 7) or RRX₁X₂RX₃X₄RRX₅X₆RX₇X₈RRX₉RRLL (SEQ ID NO: 8), wherein any one of X₁ to Xg is an amino acid selected from the group consisting of H, reactive basic amino acid residues containing a reactive group (preferably K* and/or O*) and a hydrophobic amino acid, wherein the hydrophobic amino acid is selected from the group consisting of A, V, L and I, wherein preferably the hydrophobic amino acid is L; and
(ii) three hydrophobic moieties selected from a C12 aliphatic chain, a myristoleyl group, a palmitoleyl group and a petroselinyl group, more preferably selected from a myristoleyl group, a palmitoleyl group and a petroselinyl group.

Two representative examples of reactions between the reactive group in the peptide or salt thereof are shown in table A below. A group within a modified lysine and/or ornithine, and the hydrophobic tail precursor, in these examples with an aldehyde as the second reactive group, are shown below.

**Table A. Tail binding to the lysine residue. The tail is represented with a solid ball attached to a reactive group. The reactive group is specified in each case.**

| Modified lysine residue (K*). |
|---|
| 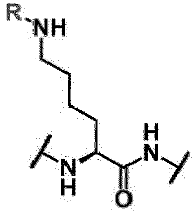 |

| 1. Hydrazide R group: aldehyde tail binding. Hydrazone formation on glutaric acid monohydrazide. |
|---|
| 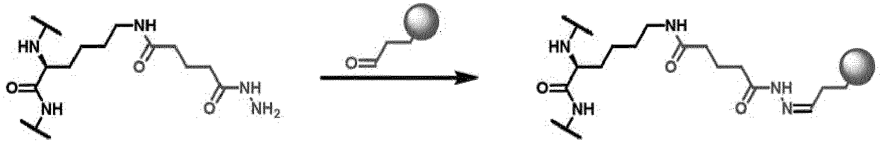 |

| 2. Aminooxy-carboxylic R group: aldehyde tail binding. Oxime formation on lysine modified with aminooxy-acetic acid. |
|---|
| 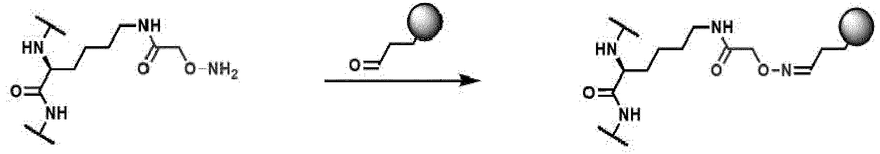 |

In an embodiment, the three hydrophobic tail precursors are hexanal, which leads to a hydrophobic tail which contains a C6 aliphatic chain. In an embodiment, the three hydrophobic tail precursors are octanal, which leads to a hydrophobic tail which contains a C8 aliphatic chain. In an embodiment the three hydrophobic tail precursors are decanal, which results in a hydrophobic tail containing a C10 aliphatic chain. In an embodiment the three hydrophobic tail precursors are dodecanal, which results in a hydrophobic tail containing a C12 aliphatic chain. In an embodiment the three hydrophobic tail precursors are myristoleyl aldehyde, which results in a hydrophobic tail consisting of a C14 aliphatic chain containing an unsaturation. In an embodiment the three hydrophobic tail precursors are palmitoleyl aldehyde, which results in a hydrophobic tail consisting of a C16 aliphatic chain containing an unsaturation. In an embodiment the three hydrophobic tail precursors are petroselinyl aldehyde, which results in a hydrophobic tail consisting of a C18 aliphatic chain containing an unsaturation. In an embodiment the three hydrophobic tail precursors are oleyl aldehyde, which results in a hydrophobic tail consisting of a C18 aliphatic chain containing a single unsaturation. In an embodiment the three hydrophobic tail precursors are linoleoyl aldehyde, which results in a hydrophobic tail consisting of a C18 aliphatic chain containing two unsaturations. In some embodiments the three hydrophobic tail precursors are an eicosenoyl aldehyde which results in a hydrophobic tail consisting of a C20 aliphatic chain containing a single unsaturation. In an embodiment the three hydrophobic tail precursors are a tetracosenoleyl aldehyde which results in a hydrophobic tail consisting of a C24 aliphatic chain containing a single unsaturation. In a particular embodiment, the tails are hydrophobic aldehyde precursors which are selected from the group consisting of myristoleyl aldehyde, palmitoleyl aldehyde, and petroselinyl aldehyde, or mixtures thereof.

In preferred embodiments, the amphiphilic molecule of the invention is any one of the following specific combinations:
- [7.3(Leu-His)] the cationic peptide is Ac-RK*HRK*K*H-NH₂ (SEQ ID NO: 1) or a salt thereof, the spacer including the reactive group is glutaric acid monohydrazide, and the tails result from the conjugation with a hydrophobic tail precursor selected from dodecanal, myristoleyl aldehyde, palmitoleyl aldehyde or petroselinyl aldehyde, preferably selected from myristoleyl aldehyde, palmitoleyl aldehyde or petroselinyl aldehyde, even more preferably myristoleyl aldehyde;
- [V1-7.3(Leu-His)] the cationic peptide is Ac-RHK*K*K*HR-NH₂ (SEQ ID NO: 2) or a salt thereof, the spacer including the reactive group is glutaric acid monohydrazide, and the tails result from the conjugation with a hydrophobic tail precursor selected from dodecanal, myristoleyl aldehyde, palmitoleyl aldehyde or petroselinyl aldehyde, preferably selected from myristoleyl aldehyde, palmitoleyl aldehyde or petroselinyl aldehyde, even more preferably myristoleyl aldehyde;
- [V2-7.3(Leu-His)] the cationic peptide is Ac-RK*HHK*K*R-NH₂ (SEQ ID NO: 3) or a salt thereof, the spacer including the reactive group is glutaric acid monohydrazide, and the tails result from the conjugation with a hydrophobic tail precursor selected from dodecanal, myristoleyl aldehyde, palmitoleyl aldehyde or petroselinyl aldehyde, preferably selected from myristoleyl aldehyde, palmitoleyl aldehyde or petroselinyl aldehyde, even more preferably myristoleyl aldehyde;
- [7.3] the cationic peptide is Ac-RK*LRK*K*L-NH₂ (SEQ ID NO: 11) or a salt thereof, the spacer including the reactive group is glutaric acid monohydrazide, and the tails result from the conjugation with a hydrophobic tail precursor selected from dodecanal, myristoleyl aldehyde, palmitoleyl aldehyde, petroselinyl aldehyde, or oleyl aldehyde, preferably selected from myristoleyl aldehyde, palmitoleyl aldehyde or petroselinyl aldehyde, even more preferably myristoleyl aldehyde;
- [7.3(Leu-Arg)] the cationic peptide is Ac-RK*RRK*K*R-NH₂ (SEQ ID NO: 12) or a salt thereof, the spacer including the reactive group is glutaric acid monohydrazide, and the tails result from the conjugation with a hydrophobic tail precursor selected from palmitoleyl aldehyde, or petroselinyl aldehyde, preferably selected from myristoleyl aldehyde, palmitoleyl aldehyde or petroselinyl aldehyde, even more preferably myristoleyl aldehyde;
- [7.3(L-H)(K-O)] the cationic peptide is Ac-RO*HRO*O*H-NH₂ (SEQ ID NO: 52) or a salt thereof, the spacer including the reactive group is glutaric acid monohydrazide, and the tails result from the conjugation with a hydrophobic tail precursor selected from myristoleyl aldehyde, palmitoleyl aldehyde or petroselinyl aldehyde, preferably myristoleyl aldehyde;
- [9.3] the cationic peptide is Ac-RK*LRRK*LRK*-NH₂ (SEQ ID NO: 13) or a salt thereof, the spacer including the reactive group is glutaric acid monohydrazide, and the tails result from the conjugation with a hydrophobic tail precursor selected from dodecanal, myristoleyl aldehyde, palmitoleyl aldehyde, petroselinyl aldehyde, or oleyl aldehyde, preferably selected from myristoleyl aldehyde, palmitoleyl aldehyde or petroselinyl aldehyde, or selected from dodecanal or palmitoleyl aldehyde;
- [13.3] the cationic peptide is Ac-RRLK*RLK*RRLK*RL-NH₂ (SEQ ID NO: 14) or a salt thereof, the spacer including the reactive group is glutaric acid monohydrazide, and the tails result from the conjugation with a hydrophobic tail precursor selected from dodecanal, myristoleyl aldehyde, palmitoleyl aldehyde, petroselinyl aldehyde or oleyl aldehyde;
- [13.3(2+1)] the cationic peptide is Ac-RRLK*RK*LRRLK*RL-NH₂ (SEQ ID NO: 15) or a salt thereof, the spacer including the reactive group is glutaric acid monohydrazide, and the tails result from the conjugation with a hydrophobic tail precursor selected from dodecanal, palmitoleyl aldehyde, or petroselinyl aldehyde;
- [13.3(3b)] the cationic peptide is Ac-RRK*K*RK*LRRLLRL-NH₂ (SEQ ID NO: 16) or a salt thereof, the spacer including the reactive group is glutaric acid monohydrazide, and the tails result from the conjugation with a hydrophobic tail precursor selected from dodecanal, myristoleyl aldehyde, or petroselinyl aldehyde;
- [13.3(Leu-His)] the cationic peptide is Ac-RRHK*RLK*RRLK*RL-NH₂ (SEQ ID NO: 17) or a salt thereof, the spacer including the reactive group is glutaric acid monohydrazide, and the tails result from the conjugation with a hydrophobic tail precursor selected from dodecanal, myristoleyl aldehyde, palmitoleyl aldehyde, petroselinyl aldehyde, oleyl aldehyde, tetracosenoleyl aldehyde;
- [13.3(Arg-His)] the cationic peptide is Ac-RHLK*RLK*RRLK*RL-NH₂ (SEQ ID NO: 18) or a salt thereof, the spacer including the reactive group is glutaric acid monohydrazide, and the tails result from the conjugation with a hydrophobic tail precursor selected from dodecanal, myristoleyl aldehyde, palmitoleyl aldehyde, petroselinyl aldehyde, oleyl aldehyde, eicosenoyl aldehyde, or tetracosenoleyl aldehyde;
- [13.3(3His)] the cationic peptide is Ac-RHLK*RHK*RRLK*RH-NH₂ (SEQ ID NO: 19) or a salt thereof, the spacer including the reactive group is glutaric acid monohydrazide, and the tails result from the conjugation with a hydrophobic tail precursor selected from dodecanal, myristoleyl aldehyde, palmitoleyl aldehyde, petroselinyl aldehyde, or oleyl aldehyde;
- [17.3] the cationic peptide is Ac-RRLK*RLLRRLK*RLK*RRL-NH₂ (SEQ ID NO: 21) or a salt thereof, the spacer including the reactive group is glutaric acid monohydrazide, and the tails result from the conjugation with a hydrophobic tail precursor selected from dodecanal, myristoleyl aldehyde, palmitoleyl aldehyde, or petroselinyl aldehyde;
- [21.3] the cationic peptide is Ac-RRLK*RLLRRLK*RLK*RRLRRLL-NH₂ (SEQ ID NO: 23) or a salt thereof, the spacer including the reactive group is glutaric acid monohydrazide, and the tails result from the conjugation with a hydrophobic tail precursor which is palmitoleyl aldehyde;
- [R + 7.3(L-H)-H] the cationic peptide is Ac-RRK*HRK*K*-NH₂ (SEQ ID NO: 53) or a salt thereof, the spacer including the reactive group is glutaric acid monohydrazide, and the tails result from the conjugation with a hydrophobic tail precursor which is myristoleyl aldehyde;
- [R + 7.3(L-H)] the cationic peptide is Ac-RRK*HRK*K*H-NH₂ (SEQ ID NO: 54) or a salt thereof, the spacer including the reactive group is glutaric acid monohydrazide, and the tails result from the conjugation with a hydrophobic tail precursor which is myristoleyl aldehyde;
- [R + 7.3 - L] the cationic peptide is Ac-RRK*LRK*K*-NH₂ (SEQ ID NO: 55) or a salt thereof, the spacer including the reactive group is glutaric acid monohydrazide, and the tails result from the conjugation with a hydrophobic tail precursor selected from myristoleyl aldehyde;
- [R + 7.3] the cationic peptide is Ac-RRK*LRK*K*L-NH₂ (SEQ ID NO: 56) or a salt thereof, the spacer including the reactive group is glutaric acid monohydrazide, and the tails result from the conjugation with a hydrophobic tail precursor selected from myristoleyl aldehyde;
- [RR + 7.3] the cationic peptide is Ac-RRRK*LRK*K*L-NH₂ (SEQ ID NO: 57) or a salt thereof, the spacer including the reactive group is glutaric acid monohydrazide, and the tails result from the conjugation with a hydrophobic tail precursor selected from myristoleyl aldehyde;
- [V3-7.3(L-H)] the cationic peptide is Ac-RO*HRK*K*H-NH₂ (SEQ ID NO: 58) or a salt thereof, the spacer including the reactive group is glutaric acid monohydrazide, and the tails result from the conjugation with a hydrophobic tail precursor selected from myristoleyl aldehyde; and
- [V4-7.3(L-H)] the cationic peptide is Ac-RK*HRK*O*H-NH₂ (SEQ ID NO: 59) or a salt thereof, the spacer including the reactive group is glutaric acid monohydrazide, and the tails result from the conjugation with a hydrophobic tail precursor selected from myristoleyl aldehyde.

In a particular embodiment, the amphiphilic molecule of the invention is obtained by reacting the cationic peptide or a salt thereof with three precursors of the hydrophobic tails, said three precursors containing a second reactive group which reacts with the reactive group in three reactive basic amino acid residues forming said covalent bond, as described above.

In another preferred embodiment, the bond formed by a reaction between the reactive group in the side chain of the three reactive basic amino acid residues and the three precursors of the hydrophobic tails which contains said hydrophobic tail and the second reactive group which can react with the reactive group in the side chain of the reactive basic amino acid residue in the cationic peptide is an amide bond, and ester bond, a hydrazone bond or an oxime bond.

In another embodiment the reactive basic amino acid residue containing a primary amine in its side chain is a reactive lysine (K*) or a reactive ornithine (O*) and the covalent bond is formed (i) between the hydrophobic tail precursor and the native lysine or native ornithine, or (ii) between the hydrophobic tail precursor and the modified lysine or modified ornithine, as described above.

In a preferred embodiment, the second reactive group is an aldehyde, and the aldehyde is selected from the group consisting of myristoleyl aldehyde, palmitoleyl aldehyde, and petroselinyl aldehyde.

In other embodiments:
(i) the peptide according to the present invention or a salt thereof, which is preferably a cationic peptide, of the amphiphilic molecule of the invention is: Ac-RK*HRK*K*H-NH₂ (SEQ ID NO: 1) or Ac-RK*LRK*K*L-NH₂ (SEQ ID NO: 11), or salts thereof, the spacers connecting each of the reactive groups in the side chain of the peptide and each of the ε-amino groups in the lysine residues are formed by reacting the peptide or salt thereof with glutaric acid monohydrazide (i.e., at least three molecules of glutaric acid monohydrazide per molecule of peptide), thereby obtaining a hydrazide-activated peptide (i.e., a polyhydrazide-activated peptide, because the peptide has three hydrazide reactive groups, as described above), and then contacting the polyhydrazide-activated peptide or salt thereof with palmitoleyl aldehyde or myristoleyl aldehyde as hydrophobic tail precursor,
(ii) the peptide according to the present invention or a salt thereof, which is preferably a cationic peptide, is Ac-RRLK*RLK*RRLK*RL-NH₂ (SEQ ID NO: 14) or a salt thereof, the spacers connecting each of the reactive groups in the side chain of the peptide and each of the ε-amino groups in the lysine residues are formed by reacting the peptide or salt thereof with glutaric acid monohydrazide (i.e., at least three molecules of glutaric acid monohydrazide per molecule of peptide), thereby obtaining a hydrazide activated peptide (i.e., a polyhydrazide-activated peptide, because the peptide has three hydrazide reactive groups, as described above) and then contacting the polyhydrazide-activated peptide or salt thereof with palmitoleyl aldehyde or petroselinyl aldehyde as hydrophobic tail precursor,
(iii) the peptide according to the present invention or a salt thereof, which is preferably a cationic peptide, is selected from the group consisting of Ac-RO*HRO*O*H-NH₂ (SEQ ID NO: 52), Ac-RRK*HRK*K*-NH₂ (SEQ ID NO: 53), Ac-RRK*HRK*K*H-NH₂ (SEQ ID NO: 54), Ac-RRK*LRK*K*-NH₂ (SEQ ID NO: 55), Ac-RRK*LRK*K*L-NH₂ (SEQ ID NO: 56), Ac-RRRK*LRK*K*L-NH₂ (SEQ ID NO: 57) Ac-RO*HRK*K*H-NH₂ (SEQ ID NO: 58) and Ac-RK*HRK*O*H-NH₂ (SEQ ID NO: 59), or salts thereof, the spacers connecting each of the reactive groups in the side chain of the peptide and each of the ε-amino groups in the lysine residues or δ-amino groups in the ornithine residues are formed by reacting the peptide or salt thereof with glutaric acid monohydrazide (i.e., at least three molecules of glutaric acid monohydrazide per molecule of peptide), thereby obtaining a hydrazide activated peptide (i.e., a polyhydrazide-activated peptide, because the peptide has three hydrazide reactive groups, as described above), and then contacting the polyhydrazide-activated peptide or salt thereof with myristoleyl aldehyde as hydrophobic tail precursor.

All the terms and embodiments described elsewhere herein are equally applicable to these aspects of the invention.

### Complexes containing amphiphilic molecules and molecules of biological interest

In a third aspect, the invention refers to a complex comprising
a) at least one amphiphilic molecule according to the second aspect of the invention, and
b) at least one molecule of biological interest.

In a preferred embodiment, the molecule of biological interest is any molecule that forms a complex with the amphiphilic molecule of the invention when it is put into contact with it at suitable conditions, e.g., at a physiological pH. In a particular embodiment, the pH is any of those specified below, preferably of 7.4.

In the context of the present invention, "physiological pH" refers to the pH of a body fluid, wherein the body fluid is preferably blood. In a particular embodiment, the physiological pH is of 6.5, 6.6, 6.7, 6.8, 6.9, 7.0, 7.1, 7.2, 7.3, 7.35, 7.4, 7.45, 7.5, 7.6, 7.7, 7.8, 7.9, 8.0, 8.1, 8.2, 8.3, 8.4, 8.5, preferably of 7.4. Methods to determine the pH of a body fluid are well known by an expert in the field. Non-limiting examples of said methods include extracting a sample of the body fluid of interest, preferably blood, and measuring its pH with a pH meter. In addition, methods to determine if a solution is at a specific pH, such as at a physiological pH, are well known by an expert in the field, and may simply consist of isolating a sample of said solution (with materials previously rinsed with milli-Q water and autoclaved) and using a pH meter to determine the pH of said sample which corresponds to that of the solution. Methods to adjust the pH of a solution, such as to a physiological pH, are also well known by an expert in the field. Said methods include progressively adding a hydroxide solution if the pH is too low, or hydrochloric acid if the pH is too high, until the pH of interest is reached. The expression "put into contact at a physiological pH", as used herein, refers to any process that allows the molecule of biological interest to coincide with the amphiphilic molecule of the invention in a specific space at the same time within a solution at a physiological pH. A non-limiting example of said process can consist of putting together a solution of the amphiphilic molecule of the invention and a solution of the molecule of biological interest into a single recipient, and in adjusting the pH to a physiological pH. Another non-limiting example of said process can consist on contacting the amphiphilic molecule of the invention and the molecule of biological interest into a buffered solution at physiological pH. Hence, preferably, the amphiphilic molecules of the present invention are able to form a complex with one or more molecules of biological interest, preferably when the contact between the molecule(s) of biological interest and the amphiphilic molecule(s) of the present invention is performed under conditions adequate for the binding of the amphiphilic molecule(s) and the molecule(s) of biological interest. In the context of the present invention, the formation of a complex between the amphiphilic molecule(s) and the molecule(s) of biological interest can be referred to as "binding or complexing of the amphiphilic molecule(s) and the molecule(s) of biological interest". The binding may also be referred to as supramolecular complexation, electrostatic interaction or packaging. The binding may occur through electrostatic and/or hydrophobic interactions. Methods to determine if a complex has been formed between the amphiphilic molecule and the molecule of biological interest at a certain pH are well known by an expert in the field. For instance, electrophoretic mobility shift assay (EMSA) or dynamic light scattering (DLS) can be used for this purpose.

In a preferred embodiment, the molecule of biological interest has negative net charge. Reference herein to "positive", "negative" or "neutral" charge means the overall charge status of the molecule of biological interest moieties under a particular condition. Also, under some conditions, e.g., under certain pH conditions, a moiety may be referred to as "partially deprotonated" or "partially protonated" or "partially charged", meaning that a certain percentage of the overall moieties in the composition are charged. It is contemplated that a zeta potential may be useful for determination of net charge measured using, for example, a laser Doppler Micro-electrophoresis technique to measure zeta potential (e.g., using a Zetasizer Nano ZS by Malvern Panalytical).

Preferably, the molecule of biological interest is selected from the group consisting of a protein, a nucleic acid, a nucleoprotein, and a small molecule. More preferably, the molecule of biological interest is selected from the group consisting of a protein, a nucleic acid and a nucleoprotein.

In a preferred embodiment, the molecule of biological interest is a **nucleic acid.** The term "nucleic acid" or "polynucleotide", as used herein, refers to a polymer having two or more deoxyribonucleotides, ribonucleotides, nucleotide analogues molecules and/or molecules that are structurally similar to a native nucleic acid, but differ from the native nucleic acid (e.g., through chemical modification) at one or more of the nucleic acid backbone (e.g., phosphate in native nucleic acids), nucleic acid sugar (e.g., deoxyribose for native DNA and ribose in native RNA), and/or nucleic acid base (e.g., adenine, cytosine, guanine, thymine or uracil in native nucleic acids), e.g., modified nucleotides.

The term "nucleic acid" or "polynucleotide", as used herein also refers to modified nucleic acids, i.e., synthetic genetic polymers, such as chemically modified nucleic acids or xeno nucleic acids (XNAs which exhibit modified backbones, sugars, or nucleobases, and even novel bases or base pairs, see e.g., Pardi, N., Hogan, M. J., Porter, F. W., & Weissman, D. (2018) mRNA vaccines-a new era in vaccinology, Nat. Rev. Drug Discov., 17(4), 261-279, Khvorova, A., & Watts, J. K. (2017) The chemical evolution of oligonucleotide therapies of clinical utility. Nat. Biotechnol., 35(3), 238-248, and Duffy, K., Arangundy-Franklin, S. & Holliger, P. (2020) Modified nucleic acids: replication, evolution, and next-generation therapeutics BMC Biol. 18, 112. Examples of chemically modified nucleic acids include those comprising methoxyuridine, N1-methylpseudouridine, phosphorothioate, PNA or LNA.

The nucleic acid of the invention can be a double stranded or single stranded oligonucleotide including, without limitation, plasmid DNA (pDNA), messenger RNA (mRNA), small interference RNAs (siRNA), small hairpin RNAs (shRNA), microRNAs (miRNA), antisense oligonucleotides (ASOs), aptamers, gene-editing guides, self-amplifying RNA (saRNA, see, e.g., Bloom, K., van den Berg, F. & Arbuthnot, P. (2021) Self-amplifying RNA vaccines for infectious diseases. Gene Ther 28, 117-129) or ribozymes. In a preferred embodiment, the nucleic acid is pDNA, siRNA, or mRNA. The polynucleotide may also be a minicircle DNA. Minicircles are small (~4kb) circular plasmid derivatives devoid of any bacterial plasmid DNA backbone.

As described above, the nucleic acid of the invention may comprise two or more deoxyribonucleotides, ribonucleotides, nucleotide analogues molecules and/or modified nucleotides. In a particular embodiment, transcription and translation of the above-mentioned nucleic acids are performed by the transcription and translation machinery inside of a cell where the complex of the invention has been introduced/delivered. In addition, transcription of the nucleic acid of the invention may lead to functional nucleic acids such as aptamers, ribozymes, and/or shRNA inside of a cell where the complex of the invention has been introduced/delivered.

The term "small interfering RNA" or "siRNA", in the context of the present invention, refers to a double-stranded RNA with a length of 20 to 25 nucleotides which is highly specific for the nucleotide sequence of its target messenger RNA, thereby interfering with the expression of the respective gene.

As used herein, "shRNA" or "short hairpin RNA" or "small hairpin RNA" is an artificial RNA molecule with a tight hairpin turn that can be used to silence target gene expression via RNA interference (RNAi). shRNAs usually comprise a double-stranded region and a loop region at one end forming a hairpin loop. The double-stranded region is typically about 19 nucleotides to about 29 nucleotides in length on each side of the stem, and the loop region is typically about three to about ten nucleotides in length.

The term "aptamer", in the context of the present invention, refers to single stranded nucleic acid chains adopting a specific tertiary structure that allows them to bind to molecular targets with high specificity and affinity, comparable to that of monoclonal antibodies, through interactions other than conventional Watson-Crick base pairing.

The term antisense refers to the use of a nucleic acid that is complementary to the coding (i.e., 'sense') base sequence of a target gene. When nucleic acids that are antisense in nature are introduced into cells, they can hybridise to the complementary 'sense' mRNA through normal Watson-Crick base pairing. Synthetic antisense DNA chains as short as 15-17 nucleotides in length can be used to block specific gene expression by either physically blocking translation of the target mRNA or causing its degradation.

In some embodiments the nucleic acid of the invention is an mRNA or a pDNA, that, when delivered into a cell (and transcribed, in the case of pDNA), translates into a protein of interest which can be directed to the cytoplasm or to the nucleus of the cell. It will be understood that the invention contemplates that the complexes of the invention may contain mRNAs encoding any of the proteins mentioned below, including any of the immunogenic proteins that are mentioned below. In some embodiments, one or more of the uridine residues in the mRNA are replaced by 5-methoxyuridine (5-moU) or by N1-methylpseudouridine.

In an embodiment, the nucleic acid forming part of the complexes of the invention comprises the mRNA sequence encoding a prefusion stabilized spike protein (protein S, with SEQ ID NO: 27) of the coronavirus SARS-CoV-2. In a particular embodiment, the nucleic acid of the invention is a polymer having ribonucleotides and/or nucleotide analogues, which comprises the mRNA sequence encoding a prefusion stabilized spike protein (protein S) of SARS-CoV-2. In a preferred embodiment, the mRNA sequence (Open Reading Frame, ORF) encoding the prefusion stabilized protein S of SARS-CoV-2 is the sequence with SEQ ID NO: 27.

In another embodiment, the nucleic acid forming part of the complexes of the invention comprises the mRNA sequence encoding ovalbumin (OVA, with SEQ ID NO: 63). In a particular embodiment, the nucleic acid of the invention is a polymer having ribonucleotides and/or nucleotide analogues, which comprises the mRNA sequence encoding ovalbumin. In a preferred embodiment, the mRNA sequence (ORF) encoding ovalbumin is the sequence with SEQ ID NO: 63.

In another embodiment, the nucleic acid forming part of the complexes of the invention comprises the mRNA sequence encoding a detectable marker. Useful detectable marker proteins which may be encoded by the mRNA and/or pDNA forming part of the complexes of the invention include luciferase, Green Fluorescence Protein (GFP), DsRed, lacZ, thymidine kinase and the like.

In a preferred embodiment, the detectable marker is the luciferase protein, preferably a firefly luciferase. In a preferred embodiment, the mRNA sequence (ORF) encoding the luciferase protein is the sequence with SEQ ID NO: 61. In a further embodiment, the mRNA sequence encoding GFP protein (ORF) is the sequence with SEQ ID NO: 62.

In a particular aspect, the nucleic acid is a ribozyme. Ribozymes (ribonucleic acid enzymes) are RNA molecules that have the ability to catalyse specific biochemical reactions, including RNA splicing in gene expression, similar to the action of protein enzymes. Ribozymes have been proposed and developed for the treatment of diseases. For instance, ribozymes have been proposed for the inhibition of RNA-based viruses, such as ribozymes against HIV infection, or against hepatitis C virus RNA, SARS coronavirus (SARS-CoV), Adenovirus and influenza A and B virus RNA.

Hence, in one embodiment, the complex of the present invention comprises
a) at least one amphiphilic molecule according to the second aspect of the invention, and
b) at least one molecule of biological interest, wherein the at least one molecule of biological interest is a nucleic acid molecule, preferably pDNA, mRNA or siRNA.

For instance, the complex of the present invention comprises
(i) a peptide according to the present invention, or a salt thereof, which is preferably a cationic peptide, which has a length of 7 amino acids and comprises only basic amino acids selected from the group consisting of R and H and reactive basic amino acid residues containing a reactive group (that is, the peptide or salt thereof comprises no hydrophobic amino acids), wherein the number of R amino acids in the peptide is at least 2, wherein the number of H amino acids in the peptide or salt thereof is 1 or 2, preferably 2, and wherein the number of reactive basic amino acid residues containing a reactive group in the peptide or salt thereof is 3, preferably wherein the basic amino acid containing a reactive group is selected from K* and O*, preferably K*;
(ii) three hydrophobic moieties selected from a C12 aliphatic chain, a myristoleyl group, a palmitoleyl group and a petroselinyl group, preferably selected from a myristoleyl group, a palmitoleyl group and a petroselinyl group; and
(iii) at least one molecule of biological interest, wherein the at least one molecule of biological interest is a nucleic acid molecule, preferably pDNA, mRNA or siRNA, more preferably pDNA or mRNA.
In another embodiment, the complex of the present invention comprises
(i) a peptide according to the present invention, or a salt thereof, which is preferably a cationic peptide, which has a length of between 7 and 21 amino acids, preferably between 7 and 17 amino acids, more preferably between 7 and 13 amino acids and even more preferably between 7 and 9 amino acids, such as 7 amino acids, and comprises a core sequence RX₁X₂R (SEQ ID NO: 9), wherein any one of X₁ to X₂ is an amino acid independently selected from the group consisting of H, reactive basic amino acid residues containing a reactive group (preferably K* and/or O*) and a hydrophobic amino acid, wherein the hydrophobic amino acid is independently selected from the group consisting of A, V, L and I, preferably wherein the hydrophobic amino acid is L;
(ii) three hydrophobic moieties selected from a C12 aliphatic chain, a myristoleyl group, a palmitoleyl group and a petroselinyl group, preferably selected from a myristoleyl group, a palmitoleyl group and a petroselinyl group, more preferably selected from a myristoleyl group and a petroselinyl group; and
(iii) at least one molecule of biological interest, wherein the at least one molecule of biological interest is a nucleic acid molecule, preferably pDNA, mRNA or siRNA, more preferably pDNA or mRNA.

In another embodiment, the complex of the present invention comprises
(i) a peptide according to the present invention, or a salt thereof, which is preferably a cationic peptide, which has a length of at least 7 amino acids, at least 8 amino acids, or at least 9 amino acids, and comprises a core sequence RX₁X₂RRX₃X₄ (SEQ ID NO: 4), wherein any one of X₁ to X₄ is an amino acid independently selected from the group consisting of H, reactive basic amino acid residues containing a reactive group (preferably K* and/or O*) and a hydrophobic amino acid, wherein the hydrophobic amino acid is independently selected from the group consisting of A, V, L and I, preferably wherein the hydrophobic amino acid is L, and preferably wherein the peptide or salt thereof has a length of at least 9 amino acids, such as 9, 13 or 21 amino acids;
(ii) three hydrophobic moieties selected from a C12 aliphatic chain, a myristoleyl group, a palmitoleyl group and a petroselinyl group, preferably selected from a myristoleyl group, a palmitoleyl group and a petroselinyl group; and
(iii) at least one molecule of biological interest, wherein the at least one molecule of biological interest is a nucleic acid molecule, preferably pDNA, mRNA or siRNA.

In another embodiment, the complex of the present invention comprises
(i) a peptide according to the present invention, or a salt thereof, which is preferably a cationic peptide, which has a length of between 7 and 21 amino acids, such as between 9 and 21 amino acids, preferably between 9 and 17 amino acids, more preferably between 9 and 13 amino acids, such as 9 amino acids, and comprises a core sequence RX₁X₂RRX₃X₄ (SEQ ID NO: 4), wherein any one of X₁ to X₄ is an amino acid independently selected from the group consisting of H, reactive basic amino acid residues containing a reactive group (preferably K* and/or O*) and a hydrophobic amino acid, wherein the hydrophobic amino acid is independently selected from the group consisting of A, V, L and I, preferably wherein the hydrophobic amino acid is L;
(ii) three hydrophobic moieties selected from a C12 aliphatic chain, a myristoleyl group, a palmitoleyl group and a petroselinyl group, preferably selected from a myristoleyl group, a palmitoleyl group and a petroselinyl group; and
(iii) at least one molecule of biological interest, wherein the at least one molecule of biological interest is a nucleic acid molecule, preferably pDNA, mRNA or siRNA.

In a further embodiment, the complex of the present invention comprises
(i) a peptide according to the present invention, or a salt thereof, which is preferably a cationic peptide, of sequence RX₁X₂RRX₃X₄RX₅ (SEQ ID NO: 5), wherein any one of X₁ to X₅ is an amino acid independently selected from the group consisting of H, reactive basic amino acid residues containing a reactive group (preferably K* and/or O*) and a hydrophobic amino acid, wherein the hydrophobic amino acid is independently selected from the group consisting of A, V, L and I, preferably wherein the hydrophobic amino acid is L;
(ii) three hydrophobic moieties selected from a C12 aliphatic chain, a myristoleyl group, a palmitoleyl group and a petroselinyl group, preferably selected from a myristoleyl group, a palmitoleyl group and a petroselinyl group; and
(iii) at least one molecule of biological interest, wherein the at least one molecule of biological interest is a nucleic acid molecule, preferably pDNA, mRNA or siRNA.

In a further embodiment, the complex of the present invention comprises
(i) a peptide according to the present invention, or a salt thereof, which is preferably a cationic peptide, of sequence RRX₁X₂RX₃X₄RRX₅X₆RX₇ (SEQ ID NO: 6), wherein any one of X₁ to X₇ is an amino acid independently selected from the group consisting of H, reactive basic amino acid residues containing a reactive group (preferably K* and/or O*) and a hydrophobic amino acid, wherein the hydrophobic amino acid is independently selected from the group consisting of A, V, L and I, wherein preferably the hydrophobic amino acid is L;
(ii) three hydrophobic moieties selected from a C12 aliphatic chain, a myristoleyl group, a palmitoleyl group and a petroselinyl group, preferably selected from a myristoleyl group, a palmitoleyl group and a petroselinyl group; and
(iii) at least one molecule of biological interest, wherein the at least one molecule of biological interest is a nucleic acid molecule, preferably pDNA, mRNA or siRNA, more preferably pDNA or siRNA.

In a further embodiment, the complex of the present invention comprises
(i) a peptide according to the present invention, or a salt thereof, which is preferably a cationic peptide, of sequence RX₁X₂X₃RX₄X₅RRX₆X₇RX₈ (SEQ ID NO: 10), wherein any one of X₁ to X₈ is an amino acid independently selected from the group consisting of H, reactive basic amino acid residues containing a reactive group (preferably K* and/or O*) and a hydrophobic amino acid, wherein the hydrophobic amino acid is independently selected from the group consisting of A, V, L and I, wherein preferably the hydrophobic amino acid is L;
(ii) three hydrophobic moieties selected from a C12 aliphatic chain, a myristoleyl group, a palmitoleyl group and a petroselinyl group, preferably selected from a myristoleyl group, a palmitoleyl group and a petroselinyl group; and
(iii) at least one molecule of biological interest, wherein the at least one molecule of biological interest is a nucleic acid molecule, preferably pDNA, mRNA or siRNA, more preferably pDNA or siRNA.

In a preferred embodiment, the complex of the invention comprises:
(i) The cationic peptide 7.3(L-H) - SEQ ID NO: 1, or a salt thereof;
(ii) Three hydrophobic moieties selected from a myristoleyl group, a palmitoleyl group and a petroselinyl group; and
(iii) At least one molecule of biological interest, wherein the at least one molecule of biological interest is pDNA, siRNA or mRNA, preferably pDNA or mRNA.

In another preferred embodiment, the complex of the invention comprises:
(i) The cationic peptide 13.3(R-H)- SEQ ID NO: 18, or a salt thereof;
(ii) Three hydrophobic moieties selected from a C12 aliphatic chain, a myristoleyl group, a palmitoleyl group, a petroselinyl group and an oleyl group; and
(iii) At least one molecule of biological interest, wherein the at least one molecule of biological interest is mRNA, siRNA or pDNA, preferably pDNA.

In another preferred embodiment, the complex of the invention comprises:
(i) The cationic peptide 9.3 - SEQ ID NO: 13, or a salt thereof;
(ii) Three hydrophobic moieties selected from a C12 aliphatic chain, a myristoleyl group, a palmitoleyl group and a petroselinyl group; and
(iii) At least one molecule of biological interest, wherein the at least one molecule of biological interest is siRNA, mRNA or DNA, preferably siRNA.

In another preferred embodiment, the complex of the invention comprises:
(i) The cationic peptide 13.3 - SEQ ID NO: 14, or a salt thereof;
(ii) Three hydrophobic moieties selected from a C12 aliphatic chain and a palmitoleyl group; and
(iii) At least one molecule of biological interest, wherein the at least one molecule of biological interest is siRNA, mRNA or DNA, preferably siRNA.

In another preferred embodiment, the complex of the invention comprises:
(i) The cationic peptide 13.3(Leu-His) - SEQ ID NO: 17, or a salt thereof;
(ii) Three hydrophobic moieties selected from a myristoleyl group, a palmitoleyl group and a petroselinyl group; and
(iii) At least one molecule of biological interest, wherein the at least one molecule of biological interest is siRNA.

In another embodiment, the molecule of biological interest is a polypeptide or a protein. In a particular embodiment, the molecule of biological interest is a protein, such as a protein negatively charged at a physiological pH, or a protein positively charged at a physiological pH, or an uncharged protein at a physiological pH.

The term "protein negatively charged at a physiological pH", as used herein, refers to a protein with an isoelectric point (pl) below a physiological pH. Similarly, the term "protein positively charged at a physiological pH", as used herein, refers to a protein with an isoelectric point (pl) above a physiological pH. The term "uncharged protein" at a physiological pH, as used herein, refers to a protein which does not present a net charge at physiological pH. Methods to determine the pl of a protein are well known by an expert in the field. Non-limiting examples of said methods include isoelectric focusing (IEF), free flow electrophoresis (FFE), capillary electrophoresis, or in-gel electrophoresis experiments using IPG strips, or the methods described in Audain E., et al. (2014) Curr. Top. Med. Chem., 14: 388-397 or in Ramos Y., et al. (2008) J. Proteome Res., 7: 2427-2434.

The term "protein" is equivalent to the term "peptide" already described previously in the application. Usually, proteins have a larger number of amino acids as compared with peptides.

Therapeutic peptides commonly act as hormones, growth factors, neurotransmitters, ion channel ligands, or anti-infective agents. Lei Wang *et al.* (Wang, L., Wang, N., Zhang, W. et al. (2022) Therapeutic peptides: current applications and future directions. Signal Transduct. Target Ther., 7: 48) provides a review of therapeutic peptides and their perspectives. Peptides such as the ones described in this review could benefit from the delivery with the amphiphilic molecule of the invention. Peptides such as protein-protein interaction inhibitors, antiapoptotic peptides such as the BH4 domain of the Bcl-xL protein, proapoptotic peptides such as KLAK, peptide probes such as the cyclic peptide phalloidin labelled with a fluorophore, or hormones such as insulin or insulin labelled with a fluorophore could also benefit from the delivery with the amphiphilic molecule of the invention.

Hence, in one embodiment, the complex of the present invention comprises
a) at least one amphiphilic molecule according to the second aspect of the invention, and
b) at least one molecule of biological interest, wherein the at least one molecule of biological interest is a peptide, preferably a therapeutic peptide.

Therapeutic proteins are extensively used in the treatment of diseases such as cancer, infectious diseases, autoimmune diseases, and many other diseases. Antibodies, IFNs, enzymes and cytokines are examples of therapeutic proteins. Preferred proteins for incorporation into the complex of the invention include, but are not limited to, hormones, antibodies, including monoclonal antibodies, antibody fragments and antibody mimetics and enzymes.

In another embodiment, the complex of the present invention comprises
a) at least one amphiphilic molecule according to the second aspect of the invention, and
b) at least one molecule of biological interest, wherein the at least one molecule of biological interest is a protein, preferably a therapeutic protein.

In some embodiments, the protein is an antibody and/or functional fragment (characteristic portion) thereof. In some embodiments, the antibody is a polyclonal, monoclonal, chimeric (i.e., "humanized"), single chain (recombinant), or bispecific antibody. In some embodiments, antibodies may have reduced effector functions and/or bispecific molecules. In some embodiments, antibodies may include Fab fragments and/or fragments produced by a Fab expression library (e.g., Fab, Fab', F(ab')2, scFv, Fv, dsFv diabody, and Fd fragments).

The term "antibody" means an immunoglobulin molecule that recognizes and specifically binds to a target, such as a protein, polypeptide, peptide, carbohydrate, polynucleotide, lipid, or combinations of the foregoing through at least one antigen recognition site within the variable region of the immunoglobulin molecule. As used herein, the term "antibody" encompasses intact polyclonal antibodies, intact monoclonal antibodies, antibody fragments (such as Fab, Fab', F(ab')2, and Fv fragments, dual affinity retargeting antibodies (DART)), single chain Fv (scFv) mutants, multispecific antibodies such as bispecific and trispecific antibodies generated from at least two intact antibodies, chimeric antibodies, humanized antibodies, human antibodies, fusion proteins comprising an antigen determination portion of an antibody, and any other modified immunoglobulin molecule comprising an antigen recognition site so long as the antibodies exhibit the desired biological activity. Since the first therapeutic antibody entered the market in 1986, various antibodies against different targets have been developed and more than 60 antibody therapeutics have been approved (Carter, P. J., and Lazar, G. A. (2018) Nat. Rev. Drug Discov. 17: 197-223).

In some embodiments, an antibody can be of any the five major classes of immunoglobulins: IgA, IgD, IgE, IgG, and IgM, or subclasses (isotypes) thereof (e.g., IgG1, IgG2, IgG3, IgG4, IgA1 and IgA2), based on the identity of their heavy-chain constant domains referred to as alpha, delta, epsilon, gamma, and mu, respectively. For instance, the antibody can be IgG or labelled IgG.

The term "antigen binding fragment" or "antibody fragment" refers to a portion of an intact antibody and comprises the antigenic determining variable regions of an intact antibody. Examples of antigen binding fragment include, but are not limited to Fab, Fab', F(ab')₂, and Fv fragments, linear antibodies, single chain antibodies, and multispecific antibodies formed from antibody fragments. In one embodiment, the antibody is an antibody fragment, such as Fab or scFv, for instance, labelled Fab or scFv.

A "monoclonal antibody" refers to a homogeneous antibody population involved in the highly specific recognition and binding of a single antigenic determinant, or epitope. This is in contrast to polyclonal antibodies that typically include different antibodies directed against different antigenic determinants. The term "monoclonal antibody" encompasses both intact and full-length monoclonal antibodies as well as antibody fragments (such as Fab, Fab', F(ab')₂, Fv), single chain (scFv) mutants, fusion proteins comprising an antibody portion, and any other modified immunoglobulin molecule comprising an antigen recognition site. Furthermore, "monoclonal antibody" refers to such antibodies made in any number of manners including but not limited to by hybridoma, phage selection, recombinant expression, and transgenic animals. For instance, the monoclonal antibody is anti-nuclear pore complex proteins monoclonal antibody (MAb414).

The term "humanized antibody" refers to forms of non-human (e.g., murine) antibodies that are specific immunoglobulin chains, chimeric immunoglobulins, or fragments thereof that contain minimal non-human (e.g., murine) sequences. Typically, humanized antibodies are human immunoglobulins in which residues from the complementary determining region (CDR) are replaced by residues from the CDR of a non-human species (e.g., mouse, rat, rabbit, hamster) that have the desired specificity, affinity, and capability (Jones et al., 1986, Nature, 321:522-525; Riechmann et al. (1988), Nature, 332:323-327; Verhoeyen et al. (1988), Science, 239:1534-1536).

In another embodiment, the antibody is an antibody mimetic. Antibody mimetics are organic compounds that, like antibodies, can specifically bind antigens, but that are not structurally related to antibodies. They are usually artificial peptides or proteins with a molar mass of about 3 to 20 kDa. They can also be nucleic acids and small molecules. Some examples of antibody mimetics are affibodies (based on the Z domain of protein A), affilins, ubiquitin, affimers, affitins, alphabodies, anticalins (artificial lipocalins), avimers, fynomers, gastrobodies, kunitz domain peptides, DARPins (design ankyrin repeats), nanobodies, monobodies, nanoCLAMPs, optimers, repebodies, Pronectin^{™}, centyrins or obodies.

In other embodiments, the protein is an enzyme. Enzymes include, without limitation, therapeutic enzymes and enzymes such as β-galactosidase, saporin, lysozyme, BSA, Cre-recombinase, ZFN, TALENs, etc.

Therapeutic enzymes may be broadly classified into four categories which are: enzymes involved in fibrinolysis, cancer treatment, enzyme replacement therapies, and treatment of other rare and common diseases (Siddhi Tandon *et a*/*.,* Therapeutic enzymes: Discoveries, production and applications, Journal of Drug Delivery Science and Technology, Volume 63, 2021).

In another embodiment, the molecule of biological interest is a nucleoprotein (NP). Nucleoproteins are any proteins that are structurally associated with nucleic acids, either DNA or RNA or any other modified nucleic acid. A deoxyribonucleoprotein (DNP) is a complex of DNA and protein. A ribonucleoprotein (RNP) is a complex of ribonucleic acid and RNA-binding protein. In a preferred embodiment, the nucleoprotein is a ribonucleoprotein. In a preferred embodiment, the nucleoprotein is a Cas9-RNP, wherein the protein is provided in association with a guide RNA (gRNA), that can be in the form of a single molecule (sgRNA) or a duplex consisting on crRNA:tracrRNA.

Cas9 is a dual RNA-guided DNA endonuclease enzyme associated with the Clustered Regularly Interspaced Short Palindromic Repeats (CRISPR) adaptive immune system in *Streptococcus pyogenes.* The Cas9 protein has been heavily utilized as a genome engineering tool in CRISPR to induce site-directed double-strand breaks in DNA. Cas9 can cleave nearly any sequence complementary to the guide RNA. Nidhi S. et al. (2021) Int. J. Mol. Sci., 22: 3327 provides a review on CRISPR-Cas systems, including their applications in therapy.

Hence, in a further embodiment, the complex of the present invention comprises
a) at least one amphiphilic molecule according to the second aspect of the invention and
b) at least one molecule of biological interest, wherein the at least one molecule of biological interest is a protein, preferably wherein the protein is a nucleoprotein, more preferably a Cas9-RNP.

For instance, the complex of the present invention comprises
(i) a peptide according to the present invention, or a salt thereof, which is preferably a cationic peptide, which has a length of 7 amino acids and comprises only basic amino acids selected from the group consisting of R and H and reactive basic amino acid residues containing a reactive group (that is, the peptide or salt thereof comprises no hydrophobic amino acids), wherein the number of R amino acids in the peptide or salt thereof is at least 2, wherein the number of H amino acids in the peptide or salt thereof is 1 or 2, preferably 2, and wherein the number of reactive basic amino acid residues containing a reactive group in the peptide or salt thereof is 3, preferably wherein the basic amino acid containing a reactive group is selected from K* and O*, preferably K*;
(ii) three hydrophobic moieties selected from a C12 aliphatic chain, a myristoleyl group, a palmitoleyl group and a petroselinyl group, more preferably selected from a myristoleyl group, a palmitoleyl group and a petroselinyl group; and
(iii) at least one molecule of biological interest, wherein the at least one molecule of biological interest is a protein, preferably wherein the protein is a nucleoprotein, more preferably a Cas9-RNP.

In another embodiment, the complex of the present invention comprises
(i) a peptide according to the present invention, or a salt thereof, which is preferably a cationic peptide, which has a length of between 7 and 21 amino acids, preferably between 7 and 17 amino acids, more preferably between 7 and 13 amino acids and even more preferably between 7 and 9 amino acids, such as 7 amino acids, and comprises a core sequence RX₁X₂R (SEQ ID NO: 9), wherein any one of X₁ to X₂ is an amino acid independently selected from the group consisting of H, reactive basic amino acid residues containing a reactive group (preferably K* and/or O*) and a hydrophobic amino acid, wherein the hydrophobic amino acid is independently selected from the group consisting of A, V, L and I, preferably wherein the hydrophobic amino acid is L;
(ii) three hydrophobic moieties selected from a C12 aliphatic chain, a myristoleyl group, a palmitoleyl group and a petroselinyl group, preferably selected from a myristoleyl group, a palmitoleyl group and a petroselinyl group; and
(iii) at least one molecule of biological interest, wherein the at least one molecule of biological interest is a protein, preferably wherein the protein is a nucleoprotein, more preferably a Cas9-RNP.

In another embodiment, the complex of the present invention comprises
(i) a peptide according to the present invention, or a salt thereof, which is preferably a cationic peptide, which has a length of at least 7 amino acids, at least 8 amino acids, or at least 9 amino acids, and comprises a core sequence RX₁X₂RRX₃X₄ (SEQ ID NO: 4), wherein any one of X₁ to X₄ is an amino acid independently selected from the group consisting of H, reactive basic amino acid residues containing a reactive group (preferably K* and/or O*) and a hydrophobic amino acid, wherein the hydrophobic amino acid is independently selected from the group consisting of A, V, L and I, preferably wherein the hydrophobic amino acid is L, and preferably wherein the cationic peptide has a length of at least 9 amino acids, such as 9, 13 or 21 amino acids;
(ii) three hydrophobic moieties selected from a C12 aliphatic chain, a myristoleyl group, a palmitoleyl group and a petroselinyl group, preferably selected from a myristoleyl group, a palmitoleyl group and a petroselinyl group or selected from a C12 aliphatic chain and a palmitoleyl group; and
(iii) at least one molecule of biological interest, wherein the at least one molecule of biological interest is a protein, preferably wherein the protein is a nucleoprotein, more preferably a Cas9-RNP.

In another embodiment, the complex of the present invention comprises
(i) a peptide according to the present invention, or a salt thereof, which is preferably a cationic peptide, which has a length of between 7 and 21 amino acids, such as between 9 and 21 amino acids, preferably between 9 and 17 amino acids, more preferably between 9 and 13 amino acids, such as 9 amino acids, and comprises a core sequence RX₁X₂RRX₃X₄ (SEQ ID NO: 4), wherein any one of X₁ to X₄ is an amino acid independently selected from the group consisting of H, reactive basic amino acid residues containing a reactive group (preferably K* and/or O*) and a hydrophobic amino acid, wherein the hydrophobic amino acid is independently selected from the group consisting of A, V, L and I, preferably wherein the hydrophobic amino acid is L;
(ii) three hydrophobic moieties selected from a C12 aliphatic chain, a myristoleyl group, a palmitoleyl group and a petroselinyl group, preferably selected from a myristoleyl group, a palmitoleyl group and a petroselinyl group or selected from a C12 aliphatic chain and a palmitoleyl group; and
(iii) at least one molecule of biological interest, wherein the at least one molecule of biological interest is a protein, preferably wherein the protein is a nucleoprotein, more preferably a Cas9-RNP.

In a further embodiment, the complex of the present invention comprises
(i) a peptide according to the present invention, or a salt thereof, which is preferably a cationic peptide, of sequence RX₁X₂RRX₃X₄RX₅ (SEQ ID NO: 5), wherein any one of X₁ to X₅ is an amino acid independently selected from the group consisting of H, reactive basic amino acid residues containing a reactive group (preferably K* and/or O*) and a hydrophobic amino acid, wherein the hydrophobic amino acid is independently selected from the group consisting of A, V, L and I, preferably wherein the hydrophobic amino acid is L;
(ii) three hydrophobic moieties selected from a C12 aliphatic chain, a myristoleyl group, a palmitoleyl group and a petroselinyl group, preferably selected from a myristoleyl group, a palmitoleyl group and a petroselinyl group or selected from a C12 aliphatic chain and a palmitoleyl group; and
(iii) at least one molecule of biological interest, wherein the at least one molecule of biological interest is a protein, preferably wherein the protein is a nucleoprotein, more preferably a Cas9-RNP.

In a further embodiment, the complex of the present invention comprises
(i) a peptide according to the present invention, or a salt thereof, which is preferably a cationic peptide, of sequence RRX₁X₂RX₃X₄RRX₅X₆RX₇ (SEQ ID NO: 6), wherein any one of X₁ to X₇ is an amino acid independently selected from the group consisting of H, reactive basic amino acid residues containing a reactive group (preferably K* and/or O*) and a hydrophobic amino acid, wherein the hydrophobic amino acid is independently selected from the group consisting of A, V, L and I, wherein preferably the hydrophobic amino acid is L;
(ii) three hydrophobic moieties selected from a C12 aliphatic chain, a myristoleyl group, a palmitoleyl group and a petroselinyl group, preferably selected from a myristoleyl group, a palmitoleyl group and a petroselinyl group, more preferably selected from a palmitoleyl group and a petroselinyl group; and
(iii) at least one molecule of biological interest, wherein the at least one molecule of biological interest is a protein, preferably wherein the protein is a nucleoprotein, more preferably a Cas9-RNP.

In a further embodiment, the complex of the present invention comprises
(i) a peptide according to the present invention, or a salt thereof, which is preferably a cationic peptide, of sequence RX₁X₂X₃RX₄X₅RRX₆X₇RX₈ (SEQ ID NO: 10), wherein any one of X₁ to X₈ is an amino acid independently selected from the group consisting of H, reactive basic amino acid residues containing a reactive group (preferably K* and/or O*) and a hydrophobic amino acid, wherein the hydrophobic amino acid is independently selected from the group consisting of A, V, L and I, wherein preferably the hydrophobic amino acid is L;
(ii) three hydrophobic moieties selected from a C12 aliphatic chain, a myristoleyl group, a palmitoleyl group and a petroselinyl group, preferably selected from a myristoleyl group, a palmitoleyl group and a petroselinyl group, more preferably selected from a palmitoleyl group and a petroselinyl group; and
(iii) at least one molecule of biological interest, wherein the at least one molecule of biological interest is a protein, preferably wherein the protein is a nucleoprotein, more preferably a Cas9-RNP.

In a preferred embodiment, the complex of the invention comprises:
(i) the cationic peptide 7.3 - SEQ ID NO: 11, or a salt thereof;
(ii) three hydrophobic moieties selected from a palmitoleyl group and a petroselinyl group; and
(iii) at least one molecule of biological interest, wherein the at least one molecule of biological interest is a Cas9-RNP.

In another preferred embodiment, the complex of the invention comprises:
(i) the cationic peptide 7.3(Leu-His) - SEQ ID NO: 1, or a salt thereof;
(ii) three palmitoleyl groups; and
(iii) at least one molecule of biological interest, wherein the at least one molecule of biological interest is a Cas9-RNP.

In another preferred embodiment, the complex of the invention comprises:
(i) the cationic peptide 13.3 - SEQ ID NO: 14; , or a salt thereof;
(ii) three myristoleyl groups; and
(iii) at least one molecule of biological interest, wherein the at least one molecule of biological interest is a Cas9-RNP.

In another embodiment, the molecule of biological interest is an antigen or immunogen.

The terms "antigen" or "immunogen" are used interchangeably to refer to a substance, typically a protein, which is capable of inducing an immune response in a subject. The term also refers to proteins that are immunologically active in the sense that once administered to a subject (either directly in the form of a peptide or a protein, or by administering to the subject a nucleotide sequence or vector that encodes a peptide or a protein) is able to evoke an immune response of the humoral and/or cellular type directed against that protein.

The immunogen may be derived from pathogenic or non-pathogenic organisms such as bacteria, virus, fungi, mold, protozoans, parasites, etc. In another embodiment, the immunogen may be a tumour-associated antigen, a tumour-specific antigen, a cell-related antigen, or an allergen.

The immunogen may be in the form of, for example, a lipid, a polysaccharide, a peptide, a protein, or a nucleotide sequence encoding a peptide or a protein. Preferably, the immunogen is in the form of a peptide, a protein or a nucleotide sequence encoding a peptide or a protein. More preferably, the immunogen is a nucleotide sequence encoding a peptide or a protein, even more preferably an mRNA encoding a peptide or a protein. Suitable nucleotide sequences encoding the peptide or protein of interest include, but are not limited, to plasmid DNA, minicircles, mRNA and saRNA. In some embodiments, the nucleotide sequence encoding the peptide or protein is chemically modified or is a xeno nucleic acid. In other embodiments, the nucleotide sequence is not chemically modified and comprises the standard deoxyribonucleotides (i.e., deoxyadenosine, deoxyguanosine, deoxycytidine, deoxythymidine) in the case of DNA or the standard ribonucleotides (i.e., adenosine, guanosine, cytidine, uridine) in the case of RNA. In one embodiment, the immunogen is a modified mRNA encoding the prefusion stabilized spike protein from SARS-CoV-2 (SEQ ID NO: 27). In another embodiment, the immunogen is a modified mRNA encoding chicken ovalbumin (OVA, SEQ ID NO: 63).

Also useful are immunogenic synthetic peptides that mimic antigenic peptide sequences. Such immunogens may be synthesized using a solid-phase technique as described, for example, in Merrifield R. B. (1963) J. Am. Chem. Soc. 85: 2149-2154. In one embodiment, the synthetic peptide that mimics an antigenic peptide sequence is the immunogenic fragment from OVA having the SIINFEKL sequence (SEQ ID NO: 64).

Other immunogens include purified, secreted antigen virulence factors, such as toxins or cytotoxins. Examples of toxins that may be used as an immunogen include bacterial endotoxins, exotoxins and enterotoxins.

### Methods for the preparation of the amphiphilic molecule

In another aspect, the invention refers to a method for the preparation of an amphiphilic molecule according to the invention, wherein the method comprises contacting a solution of the peptide, preferably the cationic peptide, of the invention, or a salt thereof, in an organic solvent with a solution of at least three precursors of the hydrophobic tail containing a reactive group which react with the reactive groups in the side chain of the reactive basic amino acids in the peptide under conditions adequate for the formation of a covalent bond between the hydrophobic tail precursor and the reactive groups within the peptide.

The terms "amphiphilic molecule", "peptide", "hydrophobic tail", "hydrophobic tail precursor", "reactive group in the side chain of the peptide" and "reactive group in the hydrophobic tail precursor" have been described above in detail in the previous aspects of the invention and are equally applicable to the present method.

Conditions adequate for the formation of a bond between the reactive group in the basic amino acid and the second reactive group within the hydrophobic tail precursor include:
- acidic media when the reactive group in the hydrophobic tail precursor is an aldehyde, or
- basic media and in the presence of a carboxylic acid activator reagent, when the reactive group in the hydrophobic tail precursor is an alcohol or a carboxylic acid.

Preferably, the adequate conditions for the formation of a bond between the reactive group in the basic amino acid and the second reactive group within the hydrophobic tail precursor include acidic media when the reactive group in the hydrophobic tail precursor is an aldehyde.

In a particular embodiment, the organic solvent is selected from dimethyl sulfoxide (DMSO), dimethyl sulfoxide/acetic acid mixtures (DMSO/AcOH), methanol, ethanol and dimethylformamide (DMF). In a preferred embodiment, the organic solvent is 5% AcOH in DMSO.

The expression "carboxylic acid activator reagent" refers to a chemical entity that is capable of reacting under mild reaction conditions with an equimolar amount (this means that for every mole of acid you need 1 mole of coupling reagent) of a carboxylic acid-bearing substrate to generate a so called "activated ester" intermediate. The generated active ester is more prone to reacting with nucleophiles because of the electron withdrawing effect incorporated from the activator reagent and, thus, this transient intermediate can then undergo the nucleophilic attack of an amino group or an alcohol group to provide amide or ester bonds. Typical carboxylic acid activator reagents for the generation of amide bonds are aminium/uronium salts (N-HBTU, N-HATU, N-TBTU) or carbodiimide reagents, such as EDC, DIC, DCC, which are also suitable for the generation of ester bonds. A more comprehensive list of suitable coupling reagents can be found in: El-Faham A. Albericio F. (2011) Chem. Rev., 111: 6557-6602, regarding examples with a variety of nucleophiles that could be potentially incorporated onto the carboxylic group moiety.

### Methods for the preparation of the complex

In another aspect, the invention refers to a method for the preparation of a complex according to the invention comprising contacting a solution of the at least one amphiphilic molecule according to the invention with a solution of the at least one molecule of biological interest in a suitable medium under conditions adequate for the formation of a complex between the at least one amphiphilic molecule and the at least one molecule of biological interest.

In a particular embodiment, the amphiphilic molecule is freshly prepared prior to the preparation of the complex for the intracellular delivery of the at least one molecule of biological interest. In another particular embodiment, the amphiphilic molecule is provided as a freeze-dried powder prior to dissolving, preferably in an organic solvent, and subsequent preparation of the complex.

In a particular embodiment, the organic solvent is dimethyl sulfoxide (DMSO).

In a particular embodiment, the expression "a solution of at least one molecule x" as used herein, refers to a solution comprising at least one molecule x.

In another particular embodiment, the solution of the at least one amphiphilic molecule comprises, in addition to said at least one amphiphilic molecule, at least one hydrophobic tail precursor as defined in the invention not linked to a peptide or salt thereof. In another particular embodiment, the solution of the at least one amphiphilic molecule comprises, in addition to said at least one amphiphilic molecule, at least one peptide of the invention or salt thereof not linked to a hydrophobic tail precursor, preferably a hydrophobic tail precursor as defined in the invention. In another particular embodiment, the solution of the at least one amphiphilic molecule comprises, in addition to said at least one amphiphilic molecule, at least one hydrophobic tail precursor as defined in the invention not linked to a peptide, preferably a peptide of the invention and at least one peptide of the invention or salt thereof not linked to a hydrophobic tail precursor, preferably a hydrophobic tail precursor as defined in the invention.

In an embodiment, the molecule of biological interest of the method for the preparation of the complex has a negative net charge.

### Methods for the in vitro delivery of a molecule of biological interest to a cell population

Further, the present invention provides methods for the *in vitro* delivery of a molecule of biological interest to a cell population, wherein the method comprises the following steps:
(i) contacting a first solution of the at least one amphiphilic molecule of the invention in a suitable solvent and a second solution containing the molecule of biological interest in suitable solvent under conditions adequate for the formation of a complex between the at least one amphiphilic molecule and the at least one molecule of biological interest and
(ii) adding the complex obtained in step (i) to the cell population under conditions adequate for the delivery of the molecule of biological interest to the cell population.

Preferably, the contacting step (i) is carried out in the same medium in which the cells are cultured and wherein the amphiphilic molecule is provided from a stock solution in an organic solvent. In another preferred embodiment, the molecule of biological interest has a negative net charge at the pH at which the contacting step (i) is carried out. In another embodiment, the molecule of biological interest of the method for the *in vitro* delivery of said molecule of biological interest to a cell population has neutral net charge or positive charge at physiological pH, as described above.

### Use as a transfection reagent and methods for in vitro delivery

In a further aspect, the invention refers to the use of a peptide, preferably a cationic peptide, according to the present invention, or a salt thereof, an amphiphilic molecule according to the present invention, or a complex according to the present invention, for the intracellular delivery (*in vitro*) of a molecule of biological interest.

In the context of the present invention, the term "transfection" refers to the process of deliberately introducing a molecule of biological interest into a cell. This may be also referred to as "delivery" or "intracellular delivery". The cell can be of any origin. In a particular embodiment, the cell is a eukaryotic cell. In a more particular embodiment, the cell is a mammalian cell. In an even more particular embodiment, the cell is a human cell. The molecule of biological interest has been described in detail above. In particular, the molecule of interest can be selected from the group consisting of a protein, a nucleic acid, a nucleoprotein, an immunogen, and a small molecule, as described in detail above. In a preferred embodiment, the nucleic acid is pDNA, siRNA, or mRNA, and the nucleoprotein is a ribonucleoprotein, as described in detail above. In a preferred embodiment, the nucleic acid is an mRNA. In a particular embodiment, the transfection is a transient transfection. In a particular embodiment, the transfection is a stable transfection. If a stable transfection is desired, that is, that the transfected gene actually remains in the genome of the cell and that it is passed onto its daughter cells, a marker gene may be co-transfected. The marker gene may be a fluorescently detectable gene, or it may provide resistance towards a specific toxin.

The use of the molecules of the invention as transfection reagents and/or delivery vectors allows the *in vitro* delivery of the molecules of interest as described above to a cell population of interest.

### Libraries and methods for obtaining libraries

The present invention further provides a method for obtaining a library of amphiphilic molecules as defined in the present invention, the method comprising contacting "n" peptide(s), preferably cationic peptides, according to the present invention, or salts thereof, in an organic solvent with at least "3n" hydrophobic tail precursors, said precursor comprising a hydrophobic tail and a second reactive group which reacts with the reactive group in the side chain of the reactive basic amino acid in the peptide or salt thereof, wherein the contacting is carried out under conditions adequate for the formation of a covalent bond between the hydrophobic tail precursor and the reactive group within the peptide or salt thereof. "n" corresponds to a natural number, such as 1, 2, 3, 4, 5, 6, 7, 8, 9, 10, etc. For instance, in the method for obtaining a library comprising at least two different amphiphilic molecules according to the present invention, at least 2 peptides or salts thereof as defined in the present invention are contacted in an organic solvent with at least 6 hydrophobic tail precursors, as described above. Preferably, the peptide is selected from any one of SEQ ID NOs 1-19, 21, 23, 49, 52-59.

In a further preferred embodiment, the hydrophobic tail comprised in the at least "3n" hydrophobic tail precursors are selected from an aldehyde, a carboxylic acid or an alcohol, preferably an aldehyde, wherein the aldehyde is selected from the group consisting of hexanal, octanal decanal, dodecanal, myristoleyl aldehyde, palmitoleyl aldehyde, petroselinyl aldehyde, oleyl aldehyde, linoleoyl aldehyde, eicosenoyl aldehyde and tetracosenoleyl aldehyde; wherein the carboxylic acid is selected from the group consisting of hexanoic acid, octanoic acid, decanoic acid, dodecanoic acid, myristoleic acid, palmitoleic acid, petroselinic acid, oleic acid, linoleic acid, eicosenoic acid and tetracosenoleic acid; and wherein the alcohol is selected from the group consisting of hexanol, octanol, decanol, dodecanol, myristoleyl alcohol, palmitoleyl alcohol, petroselinyl alcohol, oleyl alcohol, linoleoyl alcohol, gondoyl alcohol and tetracosenoleyl alcohol. More preferably, the hydrophobic tail comprised in the at least "3n" hydrophobic tail precursors is selected from dodecanal, myristoleyl, palmitoleyl, petroselinyl, oleyl, eicosenoyl and tetracosenoleyl aldehydes.

In a further preferred embodiment, the reactive group in the peptide of the invention or salt thereof is a hydrazide group or a hydroxylamine group, and the reactive group in the at least three hydrophobic tail precursors is an aldehyde group.

Preferably, the contacting step of the method for obtaining a library of amphiphilic molecules is performed contacting one type of peptide or salt thereof with one type of hydrophobic tail precursor, separately, as described herein.

The present invention further provides a library comprising at least two different amphiphilic molecules, each amphiphilic molecule comprising
(i) a peptide according to the present invention, or a salt thereof, which is preferably a cationic peptide, according to the present invention, and
(ii) three hydrophobic tails,
wherein the hydrophobic tails are connected to the peptide or salt thereof by a bond formed by a reaction between the reactive group in the side chain of the reactive basic amino acid residues of the peptide or salt thereof and a precursor of the hydrophobic tails, wherein each precursor of the hydrophobic tail contains said hydrophobic tail and a second reactive group which can react with the reactive group in the side chain of the reactive basic amino acid residues in the peptide or salt thereof, and
wherein one amphiphilic molecule differs from another amphiphilic molecule at least in the peptide or salt thereof and/or in the hydrophobic tails.

Preferably, the bond formed by a reaction between the reactive group in the side chain of the reactive basic amino acid residues of the peptide or salt thereof and the precursors of the hydrophobic tails which contain said hydrophobic tail and a second reactive group which can react with the reactive group in the side chain of the reactive basic amino acid residue in the peptide or salt thereof is an amide bond, an ester bond, a hydrazone bond or an oxime bond.

In a further preferred embodiment, each amphiphilic molecule of the library is obtained by reacting the "n" peptides or salts thereof with at least "3n" precursors of the hydrophobic tails, said precursor comprising a second reactive group which reacts with the reactive group in the reactive basic amino acid residue, thereby forming a covalent bond. Preferably, one type of peptide or salt thereof is contacted and reacts with one type of hydrophobic tail precursor, i.e., each peptide in the amphiphilic molecule comprises a single type of hydrophobic tail.

### Method for the identification of amphiphilic molecules

The present invention further provides a method for the identification of an amphiphilic molecule suitable for the delivery of a molecule of biological interest into a cell, the method comprising or, alternatively, consisting of:
(i) contacting the molecule of biological interest with a library according to the present invention, under conditions adequate for the binding/complexing of the amphiphilic molecule and the molecule of biological interest;
(ii) optionally selecting the amphiphilic molecules that are capable of binding to/complexing the molecule of biological interest from those amphiphilic molecules which do not bind to/complex the molecule of biological interest, and/or
(iii) screening for an amphiphilic molecule which is suitable for the delivery of the molecule of biological interest into a cell.

Preferably, the molecule of biological interest is selected from the group consisting of a protein, a nucleic acid, a nucleoprotein and a small molecule, as described in detail above in this description.

All the terms and embodiments described elsewhere in respect of the previous aspects of the invention are equally applicable to the present aspect of the invention.

### Use in medicine

In still a further aspect, the invention refers to a peptide according to the present invention, or a salt thereof, an amphiphilic molecule according to the present invention, or a complex according to the present invention for use in medicine/as a medicament. Thus, the invention also refers to the use of a peptide according to the present invention, or a salt thereof, an amphiphilic molecule according to the present invention, or a complex according to the present invention in medicine and/or for the manufacture of a medicament.

In yet a further aspect, the invention refers to a peptide according to the present invention, or a salt thereof, an amphiphilic molecule according to the present invention, or a complex according to the present invention, for use as a vaccine or as an immunogenic composition. Thus, the invention also refers to the use of a peptide, preferably a cationic peptide, according to the present invention, or a salt thereof, an amphiphilic molecule according to the present invention, or a complex according to the present invention for the manufacture of a vaccine. Hence, the present invention provides a vaccine comprising the peptide according to the present invention, or a salt thereof, an amphiphilic molecule according to the present invention, or a complex according to the present invention.

The term "vaccine", as used herein, refers to a substance or composition that establishes or improves immunity to a particular disease by inducing an adaptive immune response including an immunological memory. Non-limiting examples of said particular disease includes infectious diseases and cancer. Non-limiting examples of infectious diseases include those caused by the HIV, HIV-1, SARS-CoV-1, SARS-CoV-2, rabies virus, cytomegalovirus, zika virus, hepatitis C virus, human papillomavirus, respiratory syncytial virus, *Streptococcus* spp., Moloney murine leukaemia virus, Ebola virus, *Toxoplasma gondii,* or influenza virus. Non-limiting examples of cancers to be treated with the products of the invention include acute myeloid leukaemia, chronic myeloid leukaemia, multiple solid tumours, mesothelioma, glioblastoma, renal cell carcinoma, pancreatic cancer, breast cancer, non-small-cell lung cancer, prostate cancer, metastatic prostate cancer, malignant glioma, brain cancer, brain metastases, ovarian cancer, colorectal cancer, myelodysplastic syndromes, metastatic lung cancer, renal cell carcinoma, or melanoma. In a particular embodiment, the infectious diseases or cancers are any of those referred in Pardi N. et al. (2018), Nature Reviews, 17: 261-279. A vaccine typically contains an agent that resembles a disease-causing microorganism or a part thereof (e.g., a polypeptide or a nucleic acid encoding for a polypeptide). Vaccines can be prophylactic or therapeutic. In another aspect, the invention relates to a vaccine comprising the peptide of the invention, or a salt thereof, an amphiphilic molecule according to the present invention, or a complex according to the present invention. The term "adjuvant", as used herein, refers to a substance which, when added to an immunogenic agent, non-specifically enhances or potentiates an immune response to the agent in a recipient host upon exposure to the mixture. The term "immunogenic agent" or "immunogen", as used herein, refers to an antigen capable of provoking an adaptative immune response if injected by itself. All immunogens are also antigens but not all antigens are immunogens.

The vaccine of the invention may be a tolerogenic vaccine. Tolerogenic vaccines are used to reestablish immunological tolerance, restore immune homeostasis, and thereby reverse autoimmune disease and/or allergies. Tolerogenic vaccines induce long-term, antigen-specific, inhibitory memory that blocks pathogenic T cell responses via loss of effector T cells and gain of regulatory T cell function (see, e.g., Mannie, M. D., Curtis II, A. D., (2013). "Tolerogenic vaccines for Multiple sclerosis." Human vaccines & immunotherapeutics 9, 1032-1038.

The peptide, preferably the cationic peptide, according to the present invention, or salt thereof, the amphiphilic molecule according to the present invention, or the complex according to the present invention is also envisioned as suitable for use as a nucleic acid vaccine (NAV), particularly an mRNA vaccine. Nucleic acid vaccines (NAVs) are described in detail in, e.g., patent US 9,872,900 B2.

The term "therapy" or "treatment", as used herein, refers to any type of therapy, which is aimed at terminating, preventing, ameliorating, or reducing the susceptibility to a clinical condition as described herein. In a preferred embodiment, the term treatment relates to prophylactic treatment (i.e., a therapy to reduce the susceptibility to a clinical condition), of a disorder or a condition as defined herein. Thus, "treatment," "treating," and their equivalent terms refer to obtaining a desired pharmacologic or physiologic effect, covering any treatment of a pathological condition or disorder in a mammal, including a human. The effect may be prophylactic in terms of completely or partially preventing a disorder or symptom thereof and/or may be therapeutic in terms of a partial or complete cure for a disorder and/or adverse effect attributable to the disorder. That is, "treatment" includes (1) preventing the disorder from occurring or recurring in a subject, (2) inhibiting the disorder, such as arresting its development, (3) stopping or terminating the disorder or, at least, symptoms associated therewith, so that the host no longer suffers from the disorder or its symptoms, such as causing regression of the disorder or its symptoms, for example, by restoring or repairing a lost, missing or defective function, or stimulating an inefficient process, or (4) relieving, alleviating, or ameliorating the disorder, or symptoms associated therewith, where ameliorating is used in a broad sense to refer to at least a reduction in the magnitude of a parameter, such as inflammation, pain, or immune deficiency.

The peptide, preferably the cationic peptide, according to the present invention, or salt thereof, the amphiphilic molecule according to the present invention, or the complex according to the present invention may be provided as a pharmaceutical composition comprising the peptide, the amphiphilic molecule, or the complex and a pharmaceutically acceptable carrier.

The term "pharmaceutical composition", as used herein, refers to a composition comprising a therapeutically effective amount of the peptide or salt thereof, the amphiphilic molecule, or the complex and at least one pharmaceutically acceptable excipient. Pharmaceutical compositions according to the invention can be prepared, for instance, as injectables such as liquid solutions, suspensions, and emulsions. The terms "pharmaceutically acceptable excipient", or "pharmaceutically acceptable carrier", "pharmaceutically acceptable diluent", or "pharmaceutically acceptable vehicle", used interchangeably herein, refer to a non-toxic solid, semisolid or liquid filler, diluent, encapsulating material or formulation auxiliary of any conventional type. A pharmaceutically acceptable carrier is essentially non-toxic to recipients at the dosages and concentrations employed and is compatible with other ingredients of the formulation. Suitable carriers include, but are not limited to water, dextrose, glycerol, saline, ethanol, and combinations thereof. The carrier can contain additional agents such as wetting or emulsifying agents, pH buffering agents, or adjuvants, which enhance the effectiveness of the formulation. Water or saline aqueous solutions and aqueous dextrose and glycerol solutions, particularly for injectable solutions, are preferably used as vehicles. Suitable pharmaceutical vehicles are described in "Remington: the science and practice of pharmacy", (21st Ed., Lippincott Williams & Wilkins, 2006).

The term "therapeutically effective amount", as used herein in relation to the peptide, the amphiphilic molecule, or the complex comprised by the pharmaceutical composition of the invention, relates to the sufficient amount of the peptide or salt thereof, the amphiphilic molecule, or the complex to provide the desired effect, i.e. to achieve an appreciable prevention, cure, delay, reduction of severity or amelioration of one or more symptoms derived from a disease, and will generally be determined by, among other causes, the characteristics of the agent itself and the therapeutic effect to be achieved. It will also depend on the subject to be treated, the severity of the disease suffered by said subject, the chosen dosage form, etc. For this reason, the doses mentioned in this invention must be considered only as guides for the person skilled in the art, who must adjust the doses depending on the aforementioned variables. In an embodiment, the effective amount produces the amelioration of one or more symptoms of the disease that is being treated. Those skilled in the art are familiar with the principles and procedures discussed in widely known and available sources as Remington's Pharmaceutical Science (17th Ed., Mack Publishing Co., Easton, Pa., 1985) and Goodman and Gilman's The Pharmaceutical Basis of Therapeutics (8th Ed., Pergamon Press, Elmsford, N.Y., 1990).

The compound or pharmaceutical composition for use according to the invention can be administered to a subject by any suitable route of administration, for example, parenteral (e.g., intramuscular, intravenous, subcutaneous, nasal, etc.), enteral (i.e., oral, rectal, etc.), topical, etc. In a particular embodiment, the compositions for the uses of the invention are administered via an intraperitoneal, intratecal, intravesical, intrapleural, endovenous, intramuscular, subcutaneous, nasal, or topical route. In the case of solid tumours, the compound or pharmaceutical composition for use according to the invention can be administered directly into the tumour. In another particular embodiment, the compound or pharmaceutical composition for use according to the invention is administered parenterally, preferably by intravenous or intramuscular route. In a preferred embodiment, the compound or pharmaceutical composition for use according to the invention is administered by intravenous route. The compounds or pharmaceutical composition for use according to the invention may be administered directly to a subject by conventional methods. Alternatively, said pharmaceutical composition for use according to the invention may be used to transfect cells, for example, mammal cells, including human, *ex vivo,* which subsequently will be implanted into a human body or an animal to obtain the desired therapeutic effect. For administration to a human body or an animal, said cells will be formulated in a suitable medium that will have no adverse influence on cell viability. The terms "subject", "patient" or "individual" are used herein interchangeably to refer to any member of the animal kingdom and can be a vertebrate, such as, a fish, a bird, a reptile, an amphibian or a mammal, including a human, non-human primate, horse, pig, rabbit, dog, sheep, goat, cow, bird, cat, guinea pig or rodent. Preferably, the subject is a mammal, more preferably a human.

All the terms and embodiments described elsewhere herein are equally applicable to these aspects of the invention.

### Pharmacological composition of the invention

In a further aspect, the invention refers to a pharmaceutical composition comprising a therapeutically effective amount of the peptide, preferably cationic peptide, of the invention, or salt thereof, the amphiphilic molecule of the invention or the complex of the invention, together with at least one pharmaceutically acceptable excipient. Hence, the present invention further provides a pharmaceutical composition comprising the peptide according to the present invention, the amphiphilic molecule according to the present invention, or the complex according to the present invention and a pharmaceutically acceptable carrier and/or pharmaceutically acceptable excipients. The present invention further provides the pharmaceutical composition according to the present invention for use in therapy/as a medicament.

All the terms and embodiments described elsewhere herein are equally applicable to these aspects of the invention.

### Methods for in vivo delivery

In an additional aspect, the invention relates to methods for the delivery of a molecule of biological interest to a subject comprising the administration of the complex according to the invention to said subject.

All the terms and embodiments described elsewhere in respect of the previous aspects of the invention are equally applicable to the present aspect of the invention.

The term "comprising" or "comprises", as used herein, discloses also "consisting of" according to generally accepted patent practice.

### EXAMPLES

The following invention is hereby described by way of the following examples, which are to be construed as merely illustrative and not limitative of the scope of the invention.

### Example 1: Materials and methods

### Peptide synthesis

Figure 1 shows a schematic overview of the synthesis procedure. Briefly, all peptides were synthesized by manual Fmoc solid-phase peptide synthesis using Rink Amide (LL) ProTide resin (loading 0.2 mmol/g). The resin (0.1 mmol) was swelled in DMF (peptide synthesis grade, 2 mL) for 10 min in a peptide synthesis vessel prior synthesis. Coupling cycle consisted of the removal of Fmoc protecting group with a solution of piperidine in DMF (20%, 2 mL) for 10 min and then the mixture was filtered and the resin was washed with DMF (3 × 2 mL, 1 min). The amino acid coupling was carried out by treatment with a solution of α-amino acids (3 equiv) and *N*-HBTU (2.95 equiv) in DMF (2 mL), which was mixed with DIEA (0.195 M solution in DMF, 1.2 equiv) 1 min before the addition and the resulting mixture was shaken by bubbling N₂ for 15 min. Finally, the resin was washed with DMF (3 × 2 mL, 1 min). The protected amino acid used (in their L- or D- forms, depending on the peptide) were the following:
Fmoc-Arg(Pbf)-OH;
Fmoc-Leu-OH;
Fmoc-His(Trt)-OH;
Fmoc-Lys(Mtt)-OH; and
Fmoc-Orn(Mtt)-OH.

Once the linear peptide was finished, acetylation of the *N*-terminal group was performed by standard Fmoc removal conditions (20% piperidine in DMF) followed by treatment with a solution of acetic anhydride and 2,6-lutidine (1:1, 1 mL) for 20 min. The resin was washed with DCM (2 × 2 mL, 5 min), and the Mtt protecting groups were selectively removed by mechanical shaking of the resin with a mixture of DCM/HFIP/TFE/TIS (6.5:2:1:0.5, 3 × 2 mL, 2 h). Finally, the mixture was filtered, and the resin was washed with DCM (2 × 2 mL, 2 min) and DMF (2 mL, 10 min). A solution of Boc protected glutaric acid monohydrazide (2.5 equiv per free amine) and *N*-HATU (2.5 equiv per free amine) in DMF (1 mL) was added to the resin followed by the dropwise addition of a solution of DIEA (10 equiv). The resin was shaken by bubbling N₂ for 30 min and finally washed with DMF (3 × 2 mL, 2 min) and DCM (3 × 2 mL, 2 min).

| **Peptide reference** | **Peptide sequence** | **SEQ ID NO:** |
|---|---|---|
| 7.2 | Ac-RK*LRRK*L-NH₂ | SEQ ID NO: 26 |
| 7.3 | Ac-RKLRK*K*L-NH₂ | SEQ ID NO: 11 |
| 7.3(Leu-Arg) | Ac-RK*RRK*K*R-NH₂ | SEQ ID NO: 12 |
| 7.3(Leu-His) or 7.3(L-H) | Ac-RK*HRK*K*H-NH₂ | SEQ ID NO: 1 |
| 7.3(L-H)(K-O) | Ac-RO*HRO*O*H-NH₂ | SEQ ID NO: 52 |
| R + 7.3(L-H) - H | Ac-RRK*HRK*K*NH₂ | SEQ ID NO: 53 |
| R + 7.3(L-H) | Ac-RRK*HRK*K*H-NH₂ | SEQ ID NO: 54 |
| R + 7.3 - L | Ac-RRK*LRK*K*NH₂ | SEQ ID NO: 55 |
| R + 7.3 | Ac-RRK*LRK*K*L-NH₂ | SEQ ID NO: 56 |
| RR + 7.3 | Ac-RRRK*LRK*K*L-NH₂ | SEQ ID NO: 57 |
| V3-7.3(L-H) | Ac-RO*HRK*K*H-NH₂ | SEQ ID NO: 58 |
| V4-7.3(L-H) | Ac-RK*HRK*O*H-NH₂ | SEQ ID NO: 59 |
| 9.2 | Ac-RK*LRRLLRK*-NH₂ | SEQ ID NO: 31 |
| 9.3 | Ac-RK*LRRK*LRK*-NH₂ | SEQ ID NO: 13 |
| 13.2 | Ac-RRLK*RLLRRLK*RL-NH₂ | SEQ ID NO: 34 |
| 13.3 | Ac-RRLK*RLK*RRLK*RL-NH₂ | SEQ ID NO: 14 |
| 13.3(2+1) | Ac-RRLK*RK*LRRLK*RL-NH₂ | SEQ ID NO: 15 |
| 13.3(3b) | Ac-RRK*K*RK*LRRLLRL-NH₂ | SEQ ID NO: 16 |
| 13.3(Leu-His) | Ac-RRHK*RLK*RRLK*RL-NH₂ | SEQ ID NO: 17 |
| 13.3(R-H) | Ac-RHLK*RLK*RRLK*RL-NH₂ | SEQ ID NO: 18 |
| 13.3(3His) | Ac-RHLK*RHK*RRLK*RH-NH₂ | SEQ ID NO: 19 |
| 17.3 | Ac-RRLK*RLLRRLK*RLK*RRL-NH₂ | SEQ ID NO: 21 |
| 21.3 | Ac-RRLK*RLLRRLK*RLK*RRLRRLL-NH₂ | SEQ ID NO: 23 |

Finally, peptides were deprotected and cleaved from the resin by standard TFA cleavage procedure at rt by using TFA/DCM/H₂O/TIS (90:5:2.5:2.5, 1 mL per 70 mg of resin) for 2 h. Then, the mixture was filtered, washed with TFA (1 mL) and the peptide was precipitated with ice-cold Et₂O (25 mL). The precipitate was centrifuged and dissolved in H₂O (4 mL). The purification was carried by a C18 RP-HPLC [Phenomenex Luna C18(2) 100A column, H₂O (0.1% TFA)/ CH₃CN (0.1% TFA) 95:5→5:95 (0→5 min), 95:5→5:95 (5→35 min)] with a binary gradient of *Solvent A* and *Solvent B,* the collected fractions were lyophilized and stored at -20 °C. Purity and identity were confirmed by analytical HPLC-MS and ¹H-NMR.

The synthesised peptides with L-amino acids are as follows:
In addition, peptide 7.3(Leu-His) (Ac-RK*HRK*K*H-NH₂, SEQ ID NO: 1) was synthesized with D-amino acids.

Of note, all synthesized peptides are *N*-acetylated and C-amidated.

### Preparation of amphiphilic molecules

A solution of pure peptide in DMSO (10 mM) was mixed with a solution of hydrophobic aldehyde tails in 15% AcOH/DMSO (5 equiv per reactive basic amino acid residue containing a reactive group in its side chain). The mixture was stirred at 60 °C for 2 h and the resulting library of amphiphiles were tested and combined with the corresponding cargo, without requiring further purification steps, for robotic screening, minimising in this way the synthetic effort and the time required to identify novel candidates for polynucleotide delivery.

The hydrophobic aldehyde tails are as follows (chemical structures are shown below):

| | | |
|---|---|---|
| Hex | Hexanal | CAS: 66-25-1 |
| Dec | Decanal | CAS: 112-31-2 |
| Dod (DO) | Dodecanal | CAS: 112-54-9 |
| Myr (M) | Myristoleyl aldehyde | From myristoleyl alcohol |
| Pal (PA) | Palmitoleyl aldehyde | From palmitoleyl alcohol |
| Pet (PE) | Petroselinyl aldehyde | From petroselinyl alcohol |
| Ole (O) | Oleyl aldehyde | From oleyl alcohol |
| Lin | Linoleyl aldehyde | From linoleyl alcohol |
| Eic | Eicosenoyl aldehyde | From cis-11-eicosenoic acid |
| Tet | Tetracosenoleyl aldehyde | From methyl cis-15-tetracosenoate |

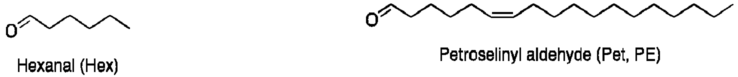

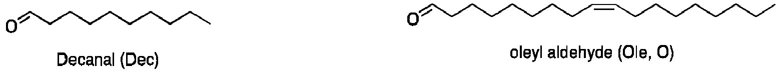

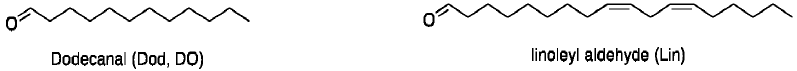

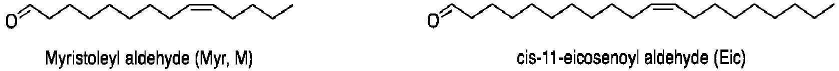

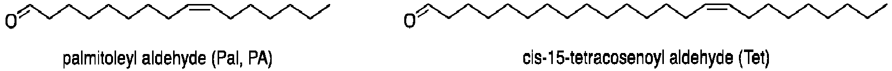

### DLS and ζ potential measurements

DLS and *ζ* potential measurements were performed in a Malvern Zetasizer NanoZS using standard disposable cuvettes. All experiments were done in triplicate at 25 °C. Freshly prepared amphiphiles solutions in AcOH/DMSO were diluted with MQ water, complexed with pDNA or mRNA and, after the complexation step, the pH was adjusted with diluted NaOH (initial pH value = 4).

### NMR characterization

Amphiphile 7.3(L-H) M was prepared according to the protocol described in Example 1 (Preparation of amphiphilic molecules) and then it was washed with Et₂O to remove the excess of aldehyde tail employed in the reaction. Then, the resulting gel was dissolved in DMSO-*d6* at a final concentration of 6.6 mM and NMR spectra were recorded on a Varian Inova 500 MHz spectrometer. Figure 22 shows the ¹H NMR spectrum. The presence of a set of singlet signals above 10 ppm and the corresponding integral signal ratio confirm the successful attachment of three hydrophobic tails onto the peptide scaffold.

### Example 2: In vitro transfection protocols

### EGFP expressing cells

To generate an EGFP expressing HeLa cell line, HeLa cells were transfected with EGFP pDNA (pEGFP-C1, Clontech) and stable transfectants were selected with 400 µg/mL of G-418 (Millipore). Green clones were selected by epifluorescence microscopy and tested for EGFP expression stability through several passages. A clone with a highly stable EGFP expression (-90 % EGFP positive cells) was chosen for further experiments, see I. Lostalé-Seijo, *et al.* (Peptide/Cas9 nanostructures for ribonucleoprotein cell membrane transport and gene edition, Chem. Sci., 2017) for further details. All other EGFP-expressing cell lines were obtained in a similar way from parent non-EGFP-expressing cell lines.

The following cell lines were used in the examples:
HeLa, corresponding to ATCC number CCL-2 (https://www.atcc.org/products/ccl-2);
A549, corresponding to ATCC number CCL-185 (https://www.atcc.org/products/ccl-185);
HepG2, corresponding to ATCC number HB-8065 (https://www.atcc.org/products/hb-8065);
HCT116, corresponding to ATCC number CCL-247 (https://www.atcc.org/products/ccl-247);
ARPE19, corresponding to ATCC number CRL-2302 (https://www.atcc.org/products/crl-2302); and
Hek293, corresponding to ATCC number CRL-1573 (https://www.atcc.org/products/crl-1573).

### siRNA

Cells expressing EGFP were seeded in a 96 well plate the day before the experiment at a concentration of 50,000 cell/mL (100 µL/w) in DMEM + 10% FBS + 1% Gln-Abt. For robotic library screening, the delivery of an EGFP-targeting siRNA (EGFP: enhanced green fluorescent protein, Ambion^{®} Silencer^{®} GFP Positive Control siRNA (siGFP) (AM4626)) in six different cell lines expressing EGFP was performed. Amphiphilic molecules stock solutions in DMSO were diluted with DMEM and freshly mixed with siRNA stock solutions in DMEM prior to transfection experiments, to afford several complexes at different amphiphile concentration (2-10 µM) while maintaining a constant final siRNA concentration of 30 nM. The mixture was shaken for 30 min at room temperature (rt) before the addition to the cells. The cell medium was aspirated, and cells were washed twice with PBS. Finally, 50 µL of the solution of siRNA/amphiphile complexes were added to the cells and incubated for 4 h. After transfection, the medium was replaced by 100 µL of fresh DMEM supplemented with 10% FBS. The fluorescence knockdown was quantified after 72 h in a plate reader, by comparison with cells transfected with a non-targeting siRNA control (siMOCK, Silencer^{™} Negative Control No. 1 siRNA (siMOCK) (AM4611)). EGFP knockdown was compared with Lipofectamine^{®} RNAiMAX silencing efficiency (30-60% depending on cell line, defined with a numeric value in the below tables). All experiments were done in triplicate.

As shown in Tables 1-5 and 7, the tested amphiphiles were able to deliver siRNA into the cells, outperforming the gold-standard Lipofectamine^{®}. In addition, as shown in Tables 20-25, amphiphiles incorporating three hydrophobic tails in their structure presented an improved delivery performance (EGFP fluorescence decrease as percentage with respect to siMOCK transfection) as compared to amphiphiles containing less than three hydrophobic tails. Amphiphilic molecules comprising a peptide with 9 or 13 amino acids performed particularly well for siRNA delivery, especially when combined with DO, M, PA or PE hydrophobic tails, in particular with PE hydrophobic tails. For instance, peptide 13.3 combined with DO or PA hydrophobic tails or 13.3(L-H) combined with PE, PA, or M hydrophobic tails performed particularly well for the delivery of siRNA to the tested cell lines.

### pDNA

Cells were seeded in a 96 well plate the day before the experiment at a concentration of 60,000 cell/mL (100 µL/w) in DMEM + 10% FBS + 1% Gln-Abt. For robotic library screening, the delivery of pDNA encoding for the expression of *Renilla* luciferase gene (Rluc, pRL-CMV (Promega, #E2261)) in six different cell lines was performed. Amphihilic molecule stock solutions in DMSO were diluted with DMEM and freshly mixed with pDNA stock solutions in DMEM prior to transfection experiments, to afford several complexes at different amphiphile concentration (2-10 µM) while maintaining a constant final pDNA concentration of 2 ng/µL. The mixture was shaken for 30 min at room temperature before the addition to the cells. The cell medium was aspirated, and cells were washed twice with PBS. Finally, 50 µL of the complex's solution were added to the cells and incubated for 4 h. After the incubation, the medium was replaced by 100 µL of fresh DMEM supplemented with 10% FBS. 72 hours after transfection the DMEM was removed, cells were washed once with HBSS and 90 µl of HBSS were added to each well. Cells were incubated at 37°C for 30 minutes. Then basal luminescence levels were read in a plate reader. Immediately, 10 µl of coelenterazine h in HBSS were added to each well (5 µM final concentration) and cells were incubated at 37°C for 10 minutes. Finally, the total luminescence measurement of the plates was performed. Amphiphile transfection efficiency was compared and normalized to Lipofectamine^{®} 2000 efficiency (value of 1 in Tables 1-5 and 7; bold numbers). All experiments were done in triplicate.

As shown in Tables 1-5 and 7, the tested amphiphiles were able to deliver pDNA into the cells, outperforming the gold-standard Lipofectamine^{®} 2000. In addition, amphiphiles incorporating three hydrophobic tails in their structure presented an improved delivery performance as compared to amphiphiles containing two hydrophobic tails. Examples of this are illustrated in Tables 8-13, particularly for cell lines A549, ARPE19, HCT116, HEK293, HeLa and HepG2, where a variety of three-tailed amphiphilic molecules outperform their two-tailed counterparts with a variety of hydrophobic tails. The best candidates were amphiphiles comprising a cationic peptide with a number of amino acids ranging from 7 to 17. Most prevalent hydrophobic tails are M and PE, although not exclusively. Particularly good candidates are peptide 7.3(L-H) (Ac-RK*HRK*K*H-NH₂, SEQ ID NO: 1) combined with M, PA or PE tails, which present the best transfection results for pDNA delivery throughout all cell lines tested, or peptide 13.3(R-H) (Ac-RHLK*RLK*RRLK*RL-NH₂, SEQ ID NO: 18), with a variety of aldehyde tails (DO, M, PA, PE, O), which is highly effective in many cell lines. In addition, peptide 9.3 (Ac-RK*LRRK*LRK*-NH₂, SEQ ID NO: 13) provides scattered efficacy for the delivery of pDNA.

### mRNA

Cells were seeded in a 96 well plate the day before the experiment at a concentration of 50,000 cell/mL (100 µL/w) in DMEM + 10% FBS + 1% Gln-Abt. For robotic library screening, the delivery of EGFP-mRNA (from TriLink, fully substituted with 5-methoxyuridine, capped and polyadenylated; ORF in SEQ ID NO: 62) in six different cell lines was performed. Amphiphilic molecule stock solutions were prepared in DMSO as described above and diluted in DMEM to afford a range of concentrations. The solutions of EGFP-mRNA/amphiphilic molecule complexes were freshly prepared prior to the transfection experiments. 35 µL of the corresponding amphiphile were added to 10 µL of a solution of EGFP-mRNA at 10 ng/µL in DMEM. The mixture was shaken for 30 min at room temperature before the addition to the cells. The cell medium was aspirated, and cells were washed with PBS twice. Cells were covered with 39 µL of DMEM and 11 µL of the complex's solution were added to the cells and incubated for 4 h (final concentration of peptide = 2.5, 5 and 10 µM; final concentration of EGFP-mRNA = 25 ng/well). After the incubation, the medium was replaced by 100 µL of fresh DMEM supplemented with 10% FBS. The total EGFP fluorescence was quantified after 72 h in a well plate reader. Amphiphile transfection efficiency was compared and normalized to Lipofectamine^{®} MessengerMax efficiency (value of 1 in Tables 1-5 and 7; double underlined numbers). All experiments were done in triplicate.

As shown in Tables 1-5 and 7, the tested amphiphiles were able to deliver mRNA into the cells, outperforming the reagent Lipofectamine^{®} MessengerMax. In addition, amphiphiles incorporating three hydrophobic tails in their structure outperformed amphiphiles incorporating two hydrophobic tails in their structure, see Tables 14-19. Amphiphiles comprising a peptide of 7 amino acids and three hydrophobic tails performed the best in mRNA delivery. For instance, peptide 7.3 presents good mRNA delivery results with a variety of hydrophobic tails. In particular, peptide 7.3(L-H) combined with M, PA or PE tails presents the best transfection results for mRNA delivery throughout all cell lines tested. Amphiphiles comprising a peptide of 13 amino acids and three hydrophobic tails had also a very good performance in mRNA delivery.

### Cas9-RNP (Ribonucleoprotein)

Cells expressing EGFP were seeded in a 96 well plate the day before the experiment at a concentration of 50,000 cell/mL (100 µL/w) in DMEM + 10% FBS + 1% Gln-Abt. For robotic library screening, the delivery of Cas9-RNP against EGFP (RNP designed to knock-out the gene encoding for the enhanced green fluorescent protein (EGFP)) was performed. For RNP assembly, 9.2 µL of crRNA and 9.2 µL of tracrRNA were mixed in 211.6 µL of Nuclease-Free Duplex Buffer (IDT), incubated for 5 min at 95 °C and slowly cooled at rt (see I. Lostalé-Seijo, *et al.* Peptide/Cas9 nanostructures for ribonucleoprotein cell membrane transport and gene edition, Chem. Sci., 2017). Cas9 protein was dissolved in DMEM at a final concentration of 4 µM and equal volumes of the RNA complex and Cas9 were mixed and incubated for 5 min at rt. 75 µL of the corresponding amphiphile concentrations were added to 5 µL of RNP at 2 µM. The mixture was shaken for 30 min at room temperature before the addition to the cells. The cell medium was aspirated, and cells were washed twice with PBS. Cells were covered with 30 µL of DMEM and 20 µL of the RNP/amphiphile complexes were added to the cells (final peptide concentrations = 2.5, 5 and 10 µM; final Cas9-RNP concentration = 50 nM) and incubated for 4 h. After the incubation, the medium was replaced by 100 µL of fresh DMEM supplemented with 10% FBS. The total EGFP fluorescence was quantified after 72 h in a plate reader (Figure 2, Table 1 (right) and Table 6). All experiments were done in triplicate.

As shown in Tables 1 and 6, the tested amphiphiles were able to deliver Cas9-RNP into the cells, outperforming Lipofectamine^{®} CRISPRMAX. Tables 26-31 show that amphiphiles incorporating three hydrophobic tails in their structure outperformed amphiphiles incorporating two hydrophobic tails in their structure.

The following tables show transfection results as compared with the gold-standard Lipofectamine^{®} series for the above-discussed cargos in several cell lines.

**Tables 1-7:** RLuc (pDNA) or EGFP (mRNA) expression levels were normalized to Lipofectamine reagents (value = 1) and expressed as fold-times signal increase. In the case of siRNA, the siRNA treated cells fluorescence was compared with siMOCK control fluorescence, while Cas9-RNP mediated knockout was measured against the fluorescence of untreated cells. In both siRNA and Cas9-RNP, values are expressed as % fluorescence reduction. Amphiphile concentration was 5 µM in all cases. All experiments were performed in triplicate.

**Table 1. Delivery of pDNA, mRNA, siRNA (left part of the table) and Cas9-RNP (right part of the table) in A549 cells. For siRNA delivery, Lipofectamine^{®} values represent 50% of fluorescence knockdown and for Cas9-RNP Lipofectamine CRISPRMax % of reduction was 50%.**

| pDNA/mRNA/siRNA | | | | | | | Cas9 | | |
|---|---|---|---|---|---|---|---|---|---|
| | Dod | Myr | Pal | | Pet | Ole | Myr | Pal | Pet |
| 7.3(Leu-His) | | **46** | **30** | 2 | **30** | | 60 | 67 | 58 |
| 9.3 | | **8** | 75 | | 75 | | | | |
| 13.3 | 58 | 57 | 60 | | 58 | | | | |
| 13.3(2+1) | **6** | | **6** | | **5** | | | | |
| 13.3(Leu-His) | | | | | 76 | **4** | | | |
| 13.3(3His) | | **9** | **5** | | 71 | **6** | | | |

For the three nucleic acid cargos tested (pDNA, mRNA and siRNA), peptides with three tails were able to deliver nucleic acids efficiently. See also Tables 8-31.

For pDNA delivery, from all hydrophobic tails tested, amphiphiles comprising dodecanal, myristoleyl, palmitoleyl, petroselinyl and oleyl aldehydes in their structure are able to transfect pDNA with good efficiencies. Amphiphiles comprising peptide 7.3(Leu-His) with palmitoleyl or petroselinyl aldehydes are the best candidates for the delivery of pDNA into this cell line, performing 30 times better than Lipofectamine 2000.

For mRNA delivery, the amphiphile comprising peptide 7.3(Leu-His) with palmitoleyl aldehyde outperformed the activity of Lipofectamine^{®} MessengerMAX in this cell line, being two times better.

For siRNA delivery, amphiphiles comprising peptides with 9 and 13 amino acids provide GFP silencing levels of between 60 and 75%, while Lipofectamine RNAiMAX silencing level is about 50%.

For Cas9-RNP delivery, the efficiency on GFP silencing was around 60-70% when complexing the protein with an amphiphile comprising 7.3(Leu-His) and myristoleyl, palmitoleyl or petroselinyl aldehydes. Cells transfected with the above-indicated complexes present a cell viability higher than 80%. Viability assays were performed employing the CellTiter 96^{®} Aqueous Non-Radioactive Cell Proliferation Assay (MTS) and following the manufacturer's instructions. Briefly, 20 µL of the combined MTS/PMS solution were added into each well of the 96-well assay plate containing cells in culture medium (100 µl). The plate was incubated 4 hours at 37°C and 5% CO₂ in a humidified atmosphere. Then, absorbance (490 nm) was recorded in an INFINITE M1000 PRO (TECAN).

**Table 2. Delivery of pDNA, mRNA, siRNA in ARPE19 cells. For siRNA delivery, Lipofectamine^{®} values represent 40% of fluorescence knockdown.**

| | | | | | | | | | | |
|---|---|---|---|---|---|---|---|---|---|---|
| | Dod | | Myr | | Pal | | | Pet | | Ole |
| 7.3 | 2 | | 6 | | 5 | | | **3** | 3 | 3 |
| 7.3(Leu-His) | | | **3** | 3 | **10** | 5 | 65 | **7** | 5 | |
| 9.3 | 57 | | | | 51 | | | **5** | 51 | 55 |
| 13.3 | 51 | | | | | | | **3** | | |
| 13.3(Arg-His) | **6** | 60 | | | **6** | | 51 | **4** | | |
| 13.3(3His) | | | **3** | | **3** | | | **3** | 56 | |

For the three cargos tested, peptides with three tails were able to deliver nucleic acids efficiently. See also tables 8-31.

For pDNA delivery, from all hydrophobic tails tested, amphiphilic molecules comprising dodecanal, myristoleyl, palmitoleyl or petroselinyl aldehydes deliver pDNA in this cell line with efficiencies higher than the gold standard Lipofectamine^{®} 2000. In particular, complexes comprising amphiphiles formed by cationic peptide 7.3(Leu-His) with palmitoleyl or petroselinyl aldehydes are the best candidates for the delivery of pDNA into this cell line, being 10 and 7 times better than Lipofectamine^{®} 2000, respectively.

For mRNA delivery, complexes comprising amphiphiles formed by peptides with 7 amino acids are able to deliver mRNA better than Lipofectamine^{®} MessengerMAX, being amphiphiles comprising myristoleyl, palmitoleyl and petroselinyl aldehydes up to six times better than Lipofectamine^{®} MessengerMAX.

For siRNA delivery, amphiphiles comprising peptides with 7, 9 and 13 amino acids achieve GFP silencing levels of between 50 and 65%, while Lipofectamine^{®} RNAiMAX silencing efficiency is about 40%.

**Table 3. Delivery of pDNA, mRNA, siRNA in HCT116 cells. For siRNA delivery, Lipofectamine^{®} values represent 40% of fluorescence knockdown.**

| | Dod | | Myr | | Pal | | | | Pet | | Ole | Eic |
|---|---|---|---|---|---|---|---|---|---|---|---|---|
| 7.3 | | | 3 | | | | | | | | | |
| 7.3(Leu-His) | | | **7** | 6 | **10** | 3 | | 55 | **5** | | | |
| 9.3 | | | | | **5** | | | | **4** | | 53 | |
| 13.3 | | | | | **5** | | | | **4** | | 55 | |
| 13.3(2+1) | **5** | | | | | | | | | | | |
| 13.3(Leu-His) | | | | | **5** | | | | **4** | | | |
| 13.3(Arg-His) | **9** | 60 | 62 | | **9** | | 63 | | **4** | 56 | | **8** |
| 13.3(3His) | 61 | | **4** | | **4** | | 56 | | 60 | | | |
| 17.3 | 54 | | 50 | | | | | | | | | |

For the three cargos tested, amphiphiles comprising peptides with three hydrophobic tails are able to deliver nucleic acids efficiently. See also tables 8-31.

For pDNA delivery into HCT116 cells, from all hydrophobic tails tested, amphiphiles comprising dodecanal, myristoleyl, palmitoleyl petroselinyl, oleyl and eicosenoyl aldehydes deliver pDNA with efficiencies higher than the gold standard Lipofectamine^{®} 2000. In particular, amphiphiles comprising 7.3(Leu-His) peptide with palmitoleyl aldehyde and amphiphiles comprising 13 amino acids peptides with histidines (13.3(Leu-His), 13.3(Arg-His) or 13.3(3His)) and palmitoleyl aldehyde are the best candidates for pDNA delivery in these cells, being up to 10 and 9 times better than Lipofectamine^{®} 2000, respectively.

For mRNA delivery, amphiphiles comprising peptides with 7 amino acids are able to deliver mRNA better than Lipofectamine^{®} MessengerMAX, being amphiphiles comprising peptides with myristoleyl or palmitoleyl aldehydes between three and six times better than Lipofectamine^{®} MessengerMAX.

For siRNA delivery, amphiphiles comprising peptides with 7, 9 and 13 amino acids achieve GFP silencing levels of between 51 and 63%, while Lipofectamine^{®} RNAiMAX silencing activity is about 40%. Amphiphiles comprising peptides with 13 amino acids are the most efficient when delivering siRNA into HCT116 cells.

**Table 4. Delivery of pDNA, mRNA, siRNA in Hek293 cells. For siRNA delivery, Lipofectamine^{®} values represent 30% of fluorescence knockdown.**

| | Dod | | Myr | | Pal | Pet | | Ole | Eic | Tet |
|---|---|---|---|---|---|---|---|---|---|---|
| 7.3 | | | **4** | | | | | | | |
| 7.3(Leu-His) | | | **7** | 2 | **6** | **6** | 3 | | | |
| 9.3 | 40 | | 43 | | | | | | | |
| 13.3 | **6** | 52 | 7 | | 42 | | | | | |
| 13.3(2+1) | **5** | | | | | | | | | |
| 13.3(3b) | **5** | | **5** | | | | | | | |
| 13.3(Leu-His) | 57 | | **4** | 40 | **5** | **4** | 43 | 62 | | |
| 13.3(Arg-His) | **6** | 44 | **6** | 48 | **6** | **5** | | 47 | **4** | |
| 13.3(3His) | | | | | | **7** | | **6** | | |
| 17.3 | | | **3** | 48 | | 52 | | 41 | | **4** |

For this cell line, a wide variety of peptides combined with a wide variety of hydrophobic tails work efficiently for the delivery of the three cargos tested. See also tables 8-31.

For pDNA delivery, from all hydrophobic tails tested, myristoleyl aldehyde is the best-performing hydrophobic tail for the delivery of pDNA with a wide variety of peptide lengths, achieving delivery efficiencies up to seven times better than the gold standard Lipofectamine^{®} 2000.

For mRNA delivery, amphiphiles comprising peptides with 7 amino acids are able to deliver mRNA better than Lipofectamine^{®} MessengerMAX, being amphiphiles comprising peptides with myristoleyl or petroselinyl aldehydes between two and three times better than Lipofectamine^{®} MessengerMAX.

For siRNA delivery, amphiphiles comprising peptides with 9, 13 and 17 amino acids achieve GFP silencing levels of between 40 and 62%, while Lipofectamine^{®} RNAiMAX silencing activity is about 30%.

**Table 5. Delivery of pDNA, mRNA, siRNA in HeLa cells For siRNA delivery, Lipofectamine^{®} values represent 60% of fluorescence knockdown.**

| | Dod | | Myr | | Pal | | Pet | | Ole | Lin | Tet |
|---|---|---|---|---|---|---|---|---|---|---|---|
| 7.3(Leu-Arg) | | | | | **6** | 64 | 79 | | | | |
| 7.3(Leu-His) | | | **11** | 2 | **20** | 3 | **25** | 2 | | | |
| 9.3 | **27** | | | | | | | | | | |
| 13.3 | **16** | 73 | 64 | | **13** | 79 | **8** | 62 | 66 | | |
| 13.3(2+1) | | | | | | | 67 | | | | |
| 13.3(3b) | | | | | | | 65 | | | | |
| 13.3(Leu-His) | | | 62 | | 61 | | 82 | | | | 83 |
| 13.3(Arg-His) | | | | | | | 70 | | | 68 | 79 |
| 13.3(3His) | | | | | 72 | | **11** | 79 | **20** | | |
| 17.3 | | | | | **22** | | **22** | | | | |
| 21.3 | | | | | **6** | | | | | | |

A wide variety of peptides combined with a wide variety of hydrophobic tails work efficiently for the delivery of the three cargos tested. See also tables 8-31.

For pDNA delivery, from all hydrophobic tails tested, palmitoleyl and petroselinyl aldehydes are the hydrophobic tails that, when present in the amphiphilic molecule together with peptides of different lengths, perform best for pDNA delivery into HeLa cells. With these hydrophobic tails, amphiphiles achieve efficiencies up to 25 times better than the gold standard Lipofectamine^{®} 2000. Amphiphiles comprising peptides of 9 and 13 amino acids and dodecanal as the hydrophobic tail are also able to efficiently deliver pDNA into HeLa cells.

For mRNA delivery, amphiphiles comprising 7.3(Leu-His) peptide are able to deliver mRNA better than Lipofectamine^{®} MessengerMAX, being amphiphiles with myristoleyl, palmitoleyl or petroselinyl aldehydes between two and three times better than Lipofectamine^{®} MessengerMAX.

For siRNA delivery, amphiphiles comprising peptides with 13 amino acids achieve the best levels of GFP silencing, reaching in some cases more that 80% of silencing activity, while Lipofectamine^{®} RNAiMAX's silencing activity is about 60%.

For Cas9 delivery (see table 6 below), the efficiency on GFP silencing was around 80% with amphiphiles comprising a nine amino acid peptide with three hydrophobic tails and with dodecanal or palmitoleyl aldehyde. Amphiphiles comprising 13 amino acid peptides with three hydrophobic tails are also very efficient for the intracellular delivery of Cas9 in HeLa cells. All tested amphiphiles present cell viabilities higher than 80% when used in a concentration of 5 µM, at which the transfection experiments were carried out.

**Table 6. Cas9-RNP delivery in HeLa cells. Table shows % GFP silencing due to gene editing. Lipofectamine CRISPRMax silenced up to 70% in this case.**

| | Dod | Myr | Pal | Pet | Ole |
|---|---|---|---|---|---|
| 9.3 | 80 | | 77 | | |
| 13.3 | | | 68 | 63 | 76 |
| 13.3(Leu-His) | | 73 | | | |
| 13.3(Arg-His) | | | 73 | | 73 |

**Table 7. Delivery of pDNA, mRNA, siRNA in HepG2 cells. For siRNA delivery, Lipofectamine^{®} values represent 30% of fluorescence knockdown.**

| | Dod | Myr | | Pal | Pet | |
|---|---|---|---|---|---|---|
| 7.3 | | **3** | | | | |
| 7.3(Leu-His) | | **6** | 1.3 | **6** | **4** | 1.5 |
| 9.3 | | **3** | | | | |
| 13.3 | | | | 46 | 41 | |
| 13.3(2+1) | **3** | **4** | | **3** | | |
| 13.3(Arg-His) | | **3** | | **2** | | |

For the three cargos tested, amphiphilic molecules comprising peptides with three tails were able to deliver nucleic acids efficiently. See also tables 8-31.

For pDNA delivery, from all hydrophobic tails tested in the amphiphilic molecules described in the table, myristoleyl aldehyde is the hydrophobic tail that, in combination with peptides of 7 amino acids, performs best when delivering pDNA in HepG2 cells, being the transfection efficiency 6 times better than Lipofectamine^{®} 2000.

For mRNA delivery, amphiphiles comprising 7.3(Leu-His) peptide with myristoleyl or petroselinyl aldehydes were able to outperform the activity of Lipofectamine^{®} MessengerMAX.

For siRNA delivery, amphiphiles comprising peptides of 13 amino acids and palmitoleyl or petroselinyl aldehydes achieve GFP silencing levels of between 41 and 46%, while Lipofectamine^{®} RNAiMAX silencing activity is about 30%.

### Example 3: In vitro transfection results

Tables 8-31 show the results of pDNA, mRNA, siRNA and Cas9 delivery using the indicated amphiphiles, at the indicated concentrations, in the different cell lines. All experimental details are the same as explained in Example 2.

### pDNA delivery

Tables 8-13 show the mean (triplicates) luminescent intensity (RLU) measured after the transfection of RLuc pDNA by different amphiphiles at different concentrations in a variety of cell lines. Background luminescence values, accounted by untransfected control cells, were subtracted for processing.

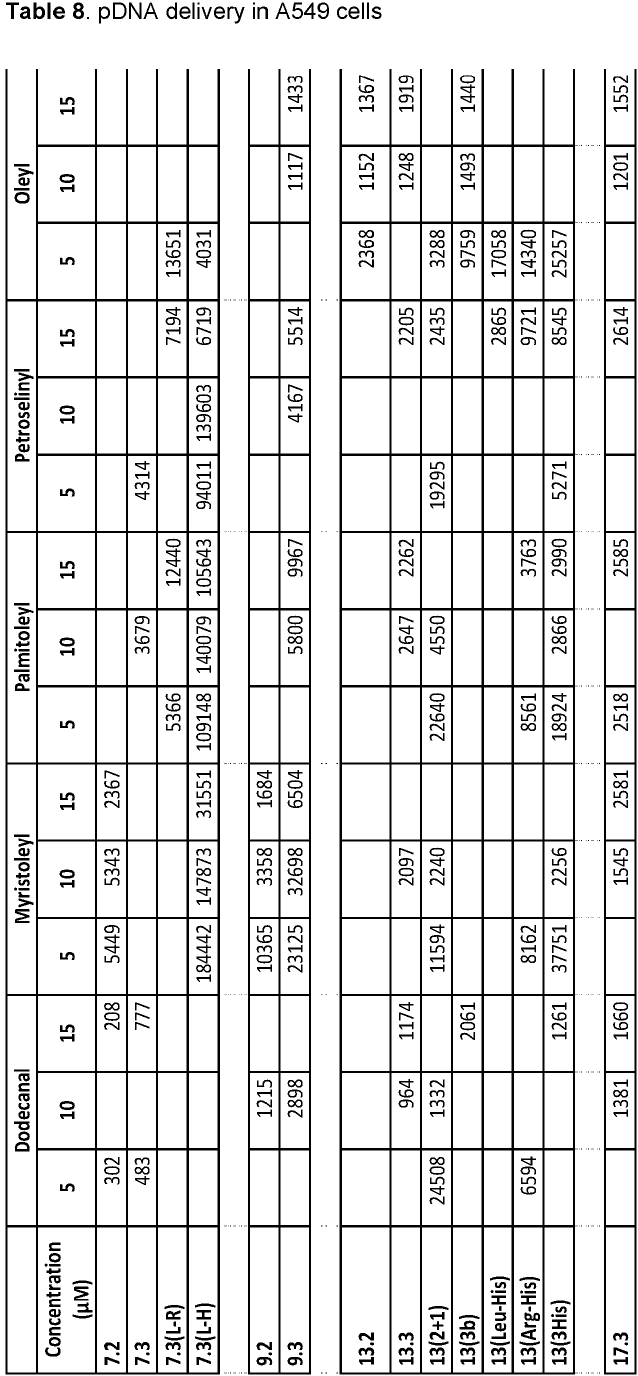

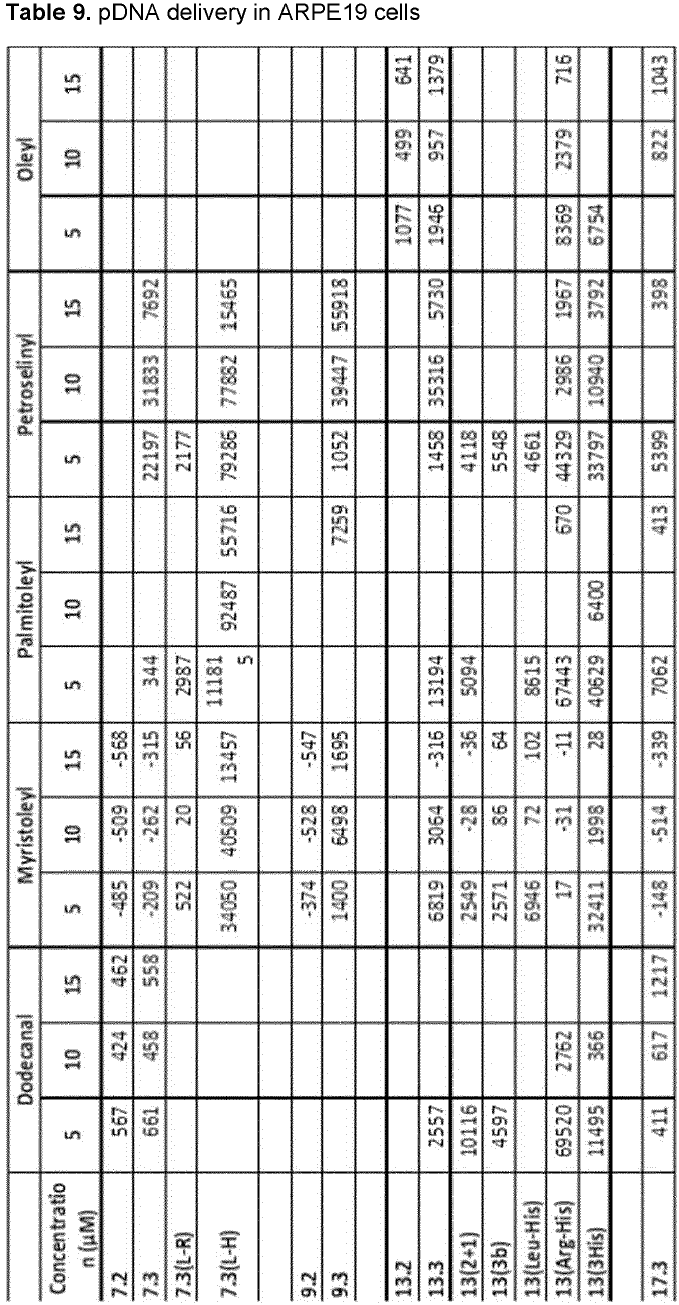

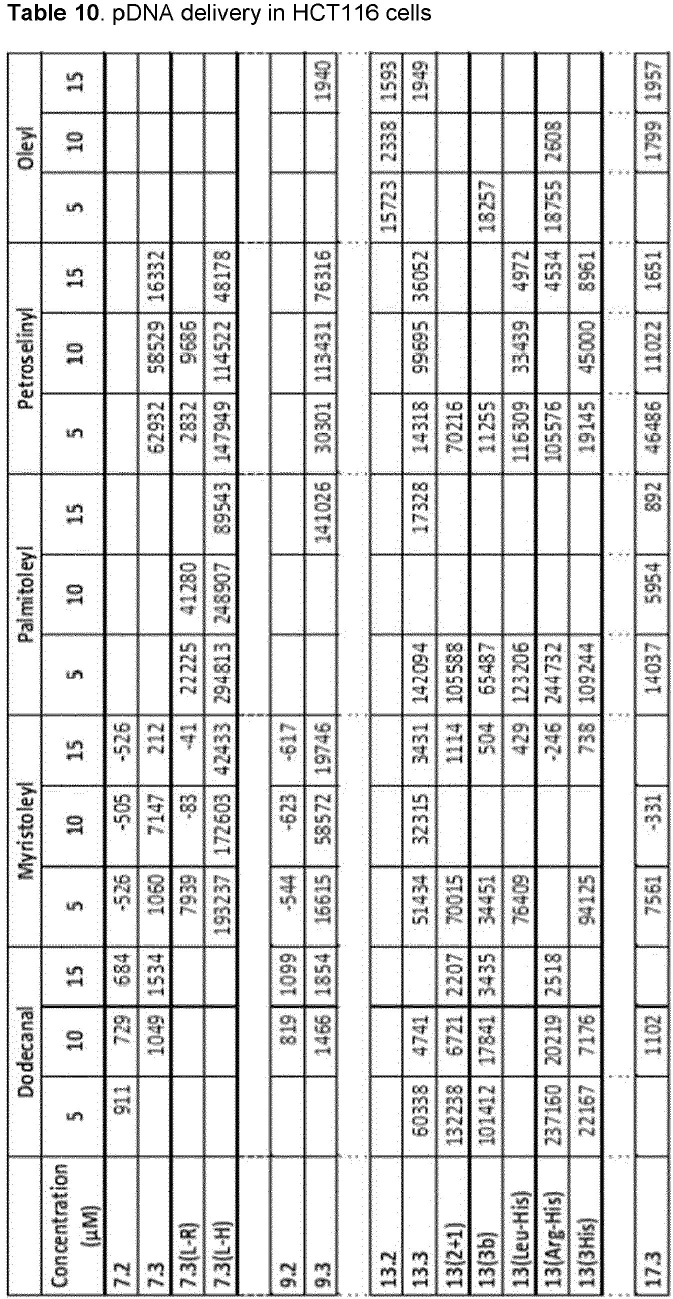

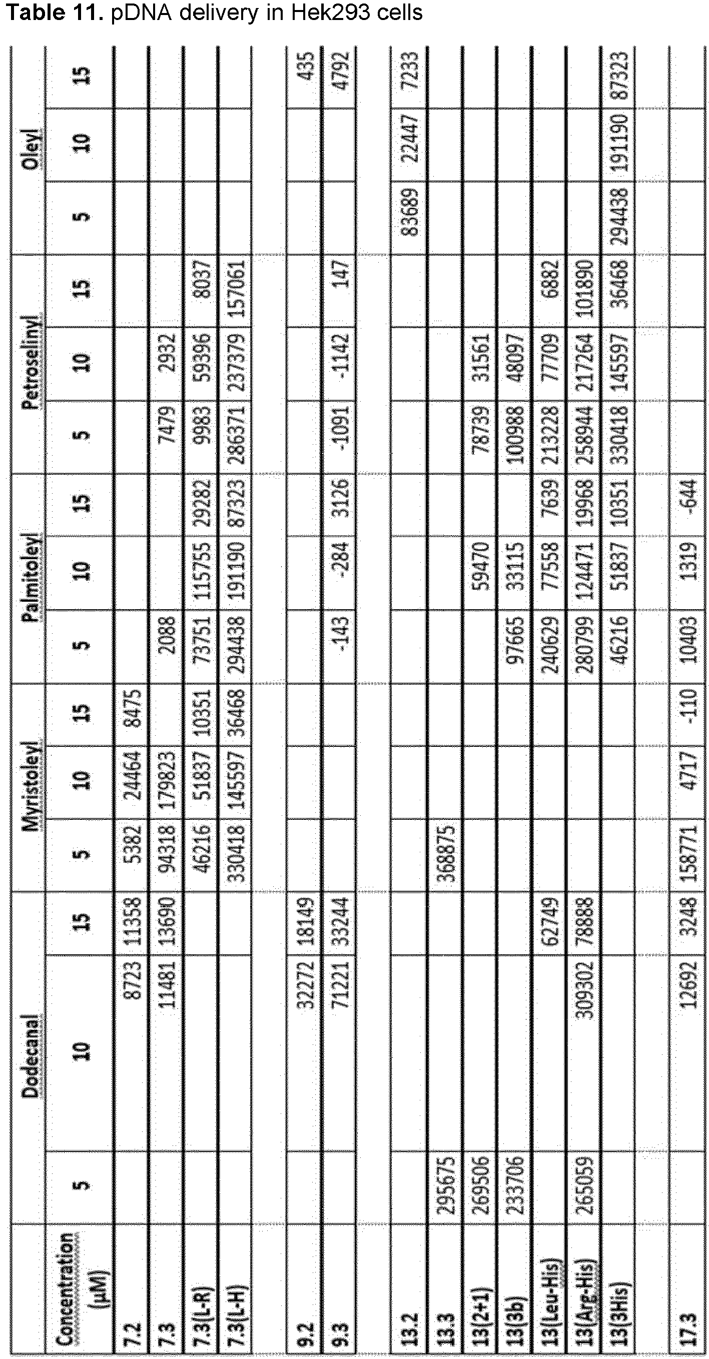

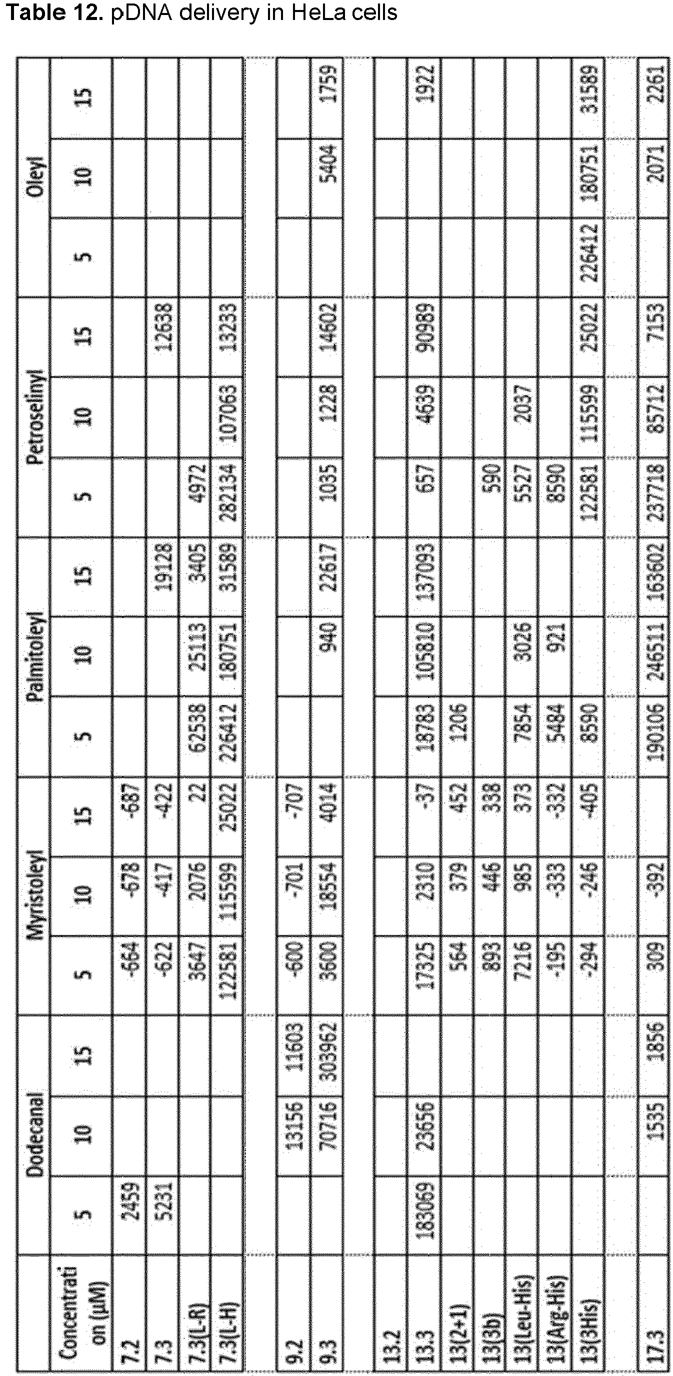

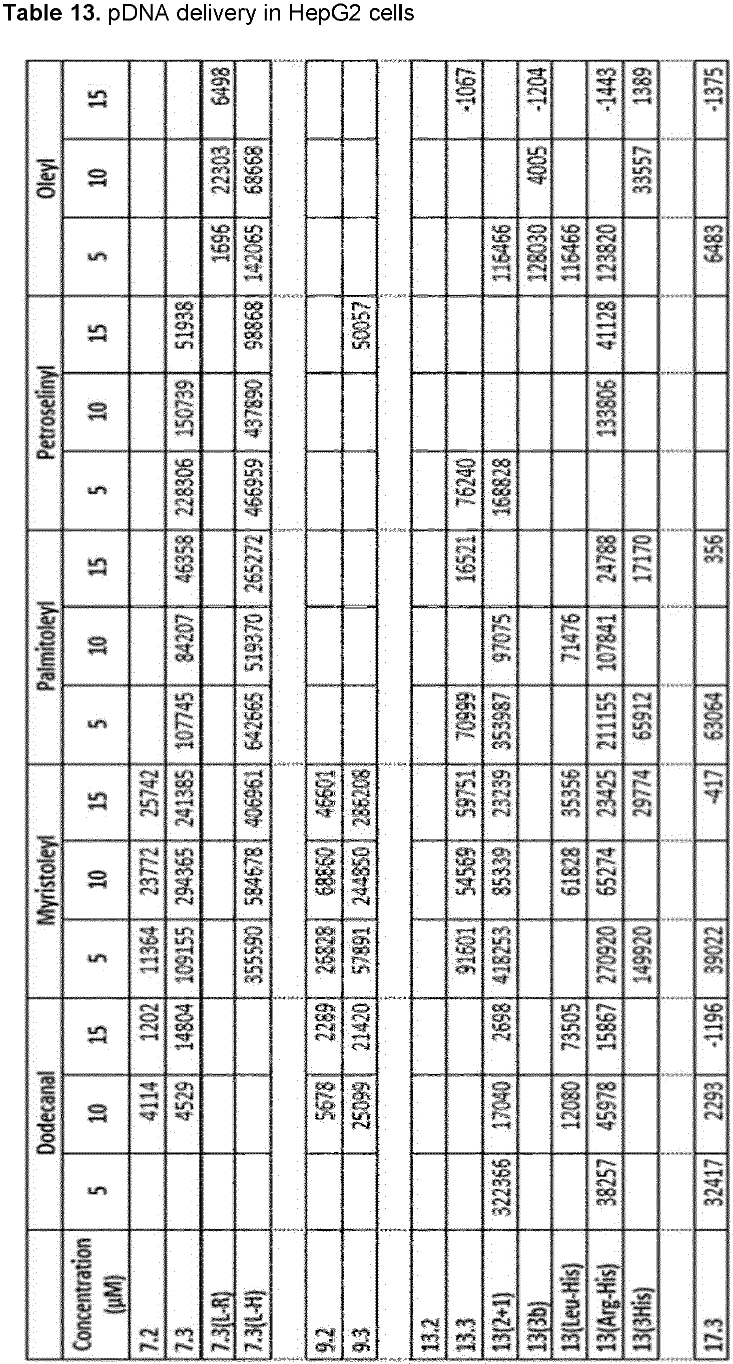

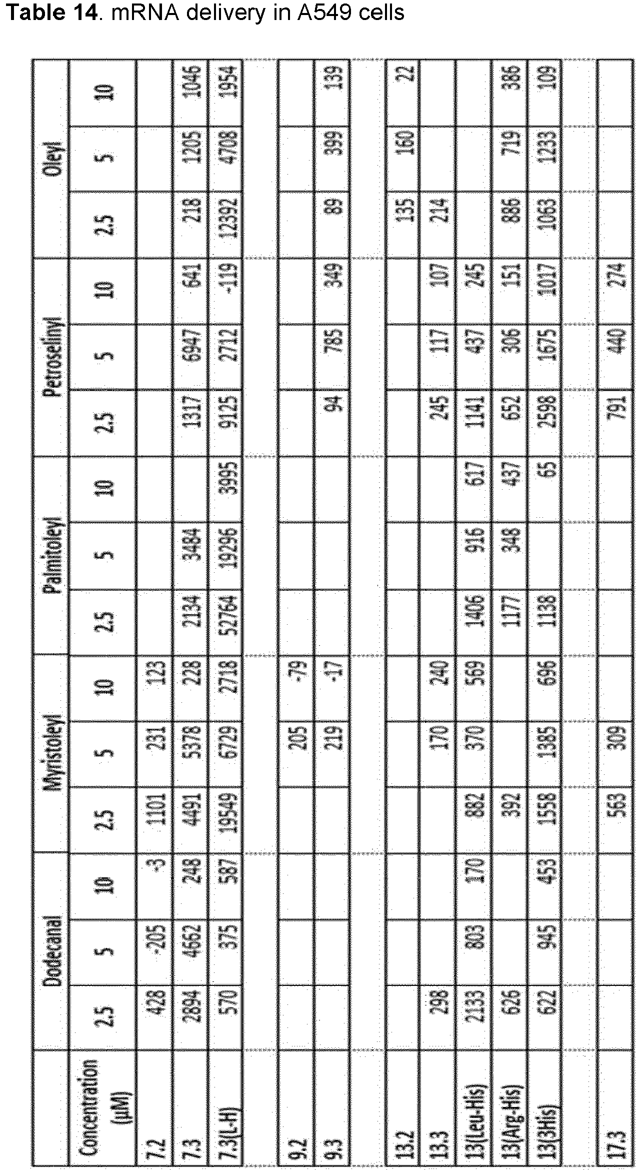

### mRNA delivery

Tables 14-19 show the mean (triplicates) fluorescence intensity measured after the transfection of EGFP mRNA by different amphiphiles at different concentrations in a variety of cell lines. Background fluorescence values, accounted by untreated control cells, were subtracted for processing.

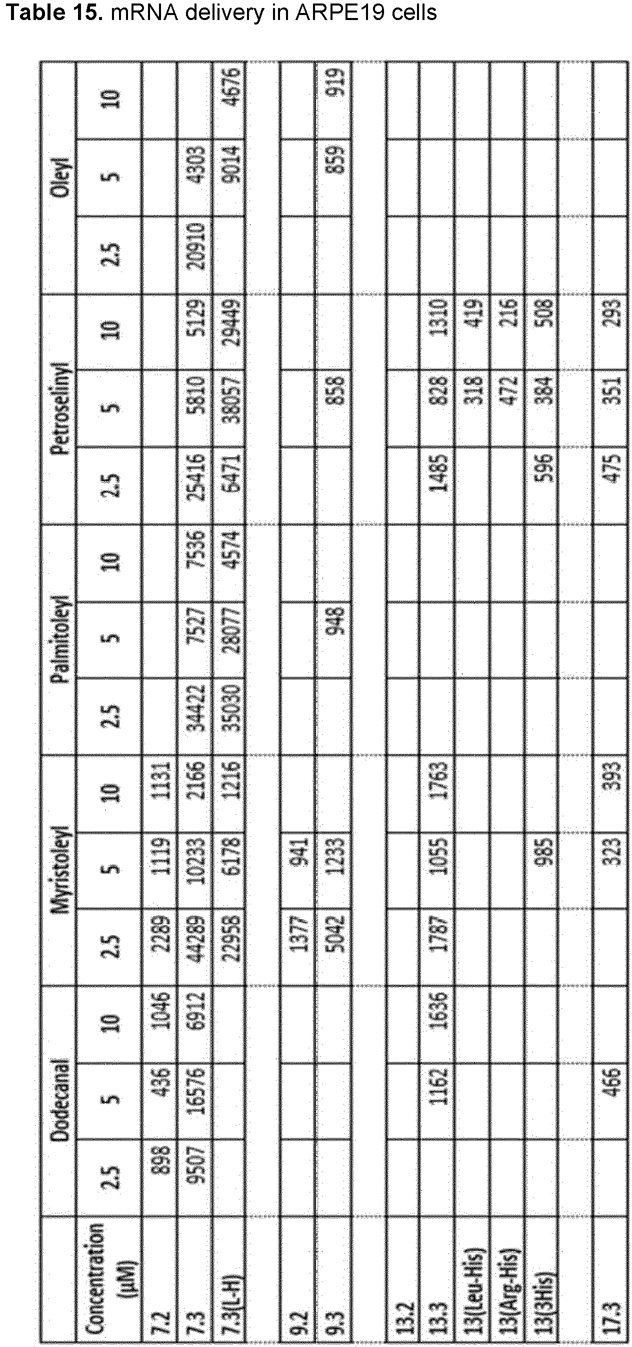

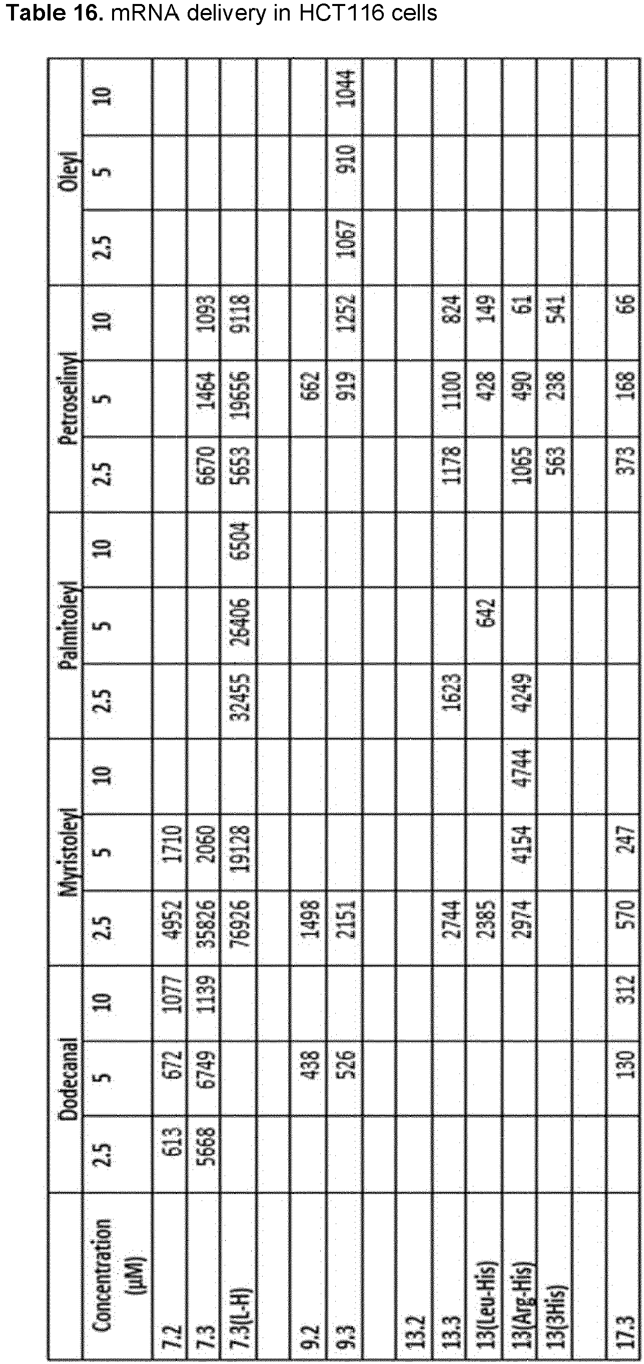

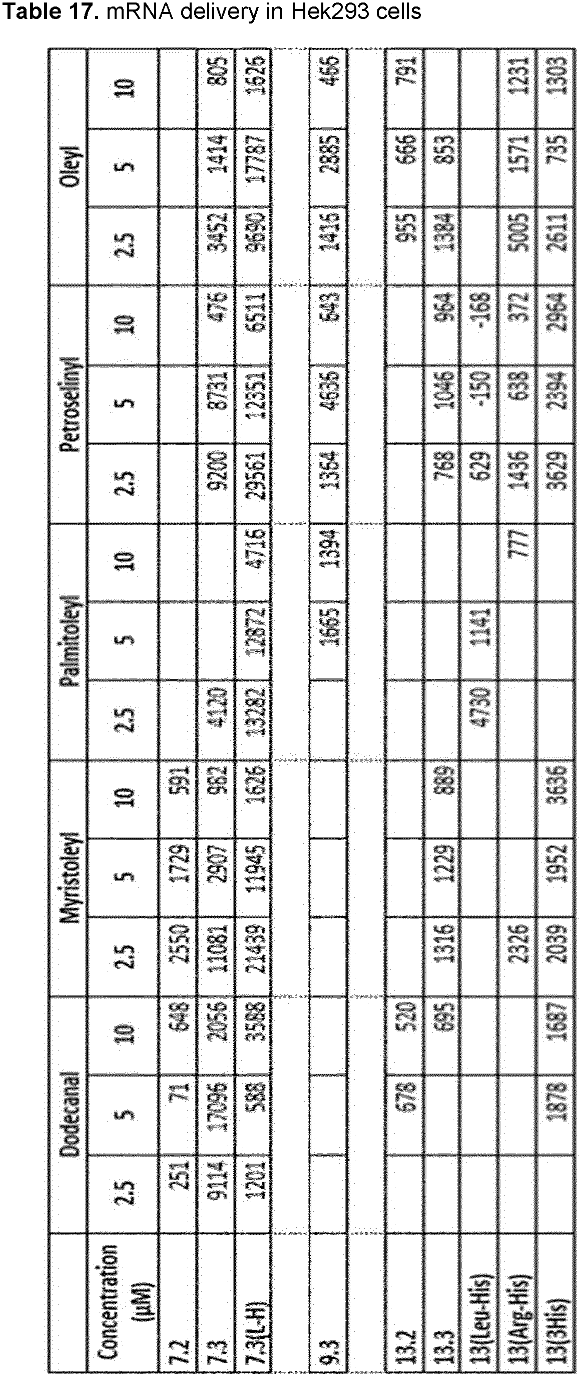

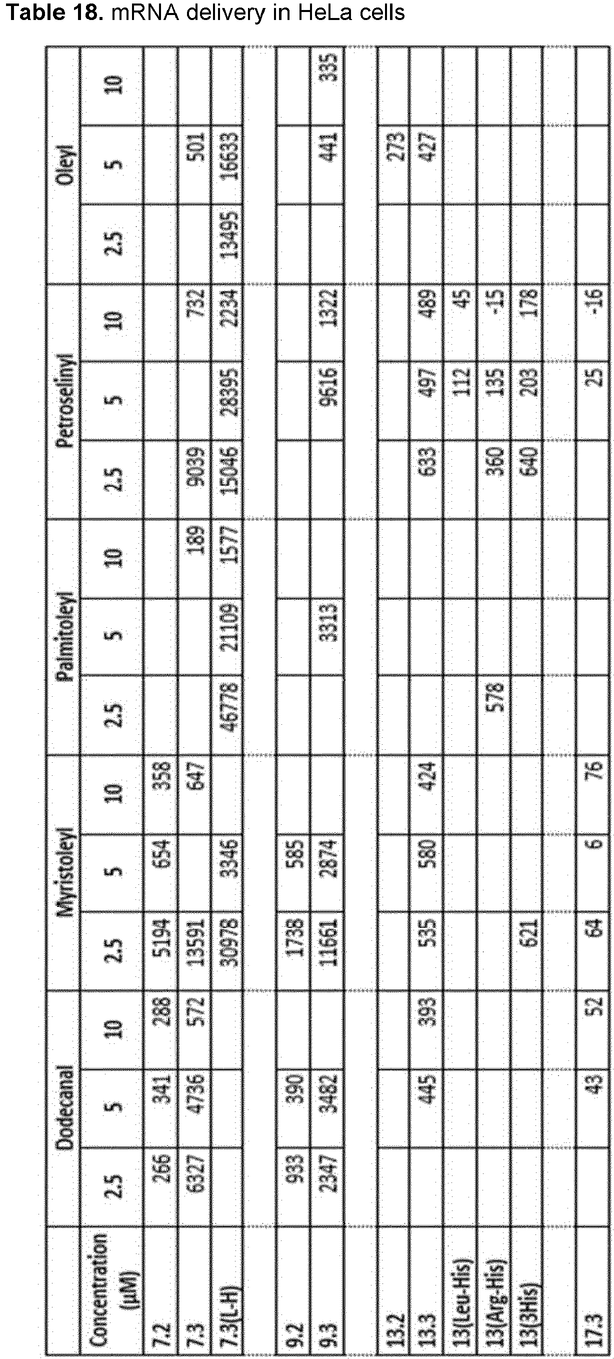

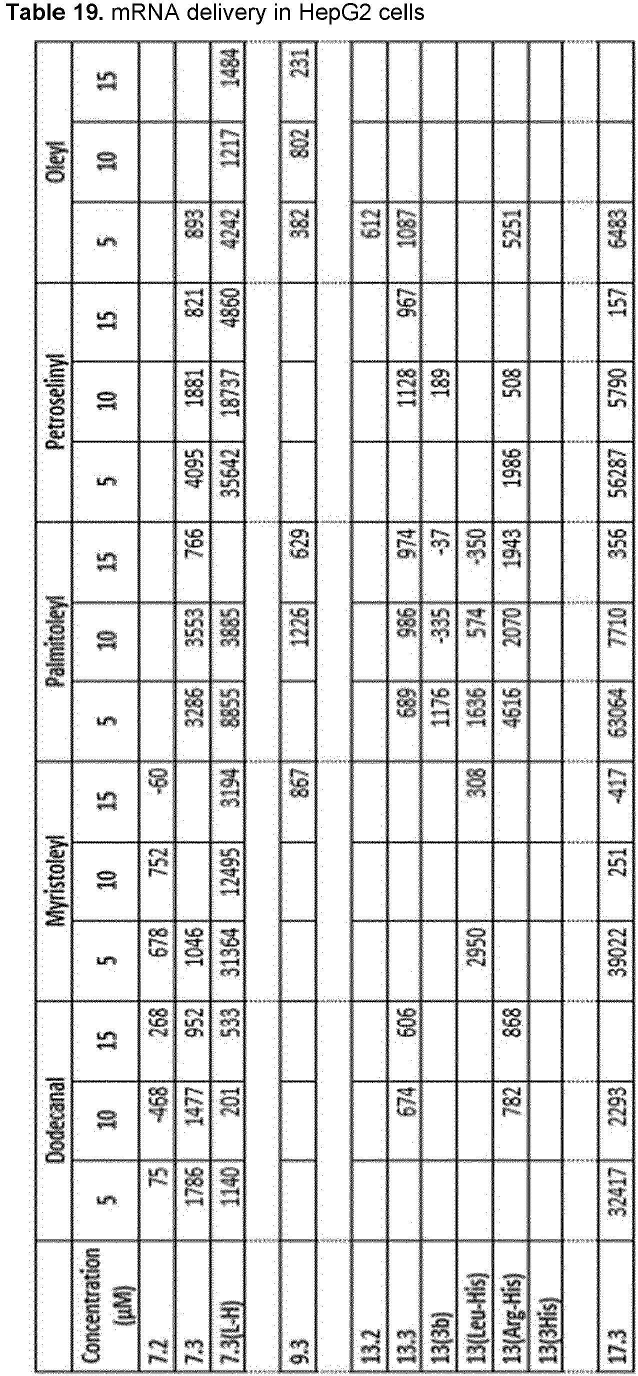

### siRNA delivery

Tables 20-25 show the mean (triplicates) percentage of GFP-silencing, accounted by fluorescence intensity decrease relative to a non-targeting siRNA (siMOCK) control, measured after the transfection of siRNA by different amphiphiles at different concentrations in a variety of cell lines. Background values, accounted by untreated control cells, were subtracted for processing.

**Table 20. siRNA delivery in A549 cells**

| | **Dodecanal** | | | **Myristoleyl** | | | **Palmitoleyl** | | | **Petroselinyl** | | | **Oleyl** | | |
|---|---|---|---|---|---|---|---|---|---|---|---|---|---|---|---|
| **Concentration (µM)** | **5** | **10** | **15** | **5** | **10** | **15** | **5** | **10** | **15** | **5** | **10** | **15** | **5** | **10** | **15** |
| **7.2** | 5 | 11 | 8 | 8 | 14 | 16 | | | | | | | | | |
| **7.3** | 7 | 22 | 19 | | | | | | | | 13 | | 3 | | 7 |
| **7.3(L-H)** | | | | 18 | 22 | 31 | | | 40 | | 20 | 52 | 2 | 14 | 21 |
| | | | | | | | | | | | | | | | |
| **9.2** | 32 | 29 | 16 | -1 | -6 | -14 | | | | | | | | | |
| **9.3** | 35 | 35 | 55 | 34 | 58 | 23 | 42 | 75 | 64 | 6 | 58 | 75 | | 32 | 28 |
| | | | | | | | | | | | | | | | |
| **13.2** | | | | | | | | | | | | | 19 | -2 | -21 |
| **13.3** | 59 | 54 | 46 | 57 | 25 | 28 | 60 | 37 | 29 | 31 | 53 | 59 | | 14 | 4 |
| **13(Leu-His)** | | 10 | 55 | | 10 | 14 | | 54 | 50 | | 38 | 76 | 29 | 20 | 17 |
| **13(Arg-His)** | | 28 | 18 | | 17 | 5 | | | 14 | | 20 | 11 | 23 | 22 | 15 |
| **13(3His)** | | 10 | 37 | | 28 | 25 | | 36 | 28 | | 42 | 71 | 22 | 26 | 29 |
| | | | | | | | | | | | | | | | |
| **17.3** | 25 | 4 | | 24 | 8 | 6 | | | | | | | | | |

**Table 21. siRNA delivery in ARPE19 cells**

| | **Dodecanal** | | | **Myristoleyl** | | | **Palmitoleyl** | | | **Petroselinyl** | | | **Oleyl** | | |
|---|---|---|---|---|---|---|---|---|---|---|---|---|---|---|---|
| **Concentration (µM)** | **5** | **10** | **15** | **5** | **10** | **15** | **5** | **10** | **15** | **5** | **10** | **15** | **5** | **10** | **15** |
| **7.2** | 4 | 10 | 20 | 14 | 28 | | | | | | | | | | |
| **7.3** | 15 | 35 | 45 | | | | | | -5 | | | | | | |
| **7.3(L-H)** | 10 | | | 45 | 45 | | 49 | 65 | 40 | 5 | | | 15 | 47 | 46 |
| | | | | | | | | | | | | | | | |
| **9.2** | 13 | 34 | 30 | 24 | | | | | | | | | | | |
| **9.3** | 57 | 57 | 34 | 47 | | | 51 | 30 | 11 | 18 | 49 | 52 | 20 | 55 | 51 |
| | | | | | | | | | | | | | | | |
| **13.2** | | | | | | | | | | | | | | 22 | 1 |
| **13.3** | 29 | 51 | 37 | | | | | 11 | | 11 | 13 | -42 | | | |
| **13(Leu-His)** | 27 | 31 | 41 | | 25 | 0 | 46 | 26 | 24 | | 34 | 13 | | 26 | 15 |
| **13(Arg-His)** | 60 | 54 | 34 | | 0 | 14 | 51 | -6 | 7 | 42 | 24 | 26 | | 29 | 31 |
| **13(3His)** | 16 | | 22 | | 42 | 30 | | 49 | 25 | 13 | 56 | 30 | | 43 | 41 |
| | | | | | | | | | | | | | | | |
| **17.3** | 29 | 14 | 22 | 43 | | -5 | 36 | 24 | -4 | 36 | 20 | | | 22 | |

**Table 22. siRNA delivery in HCT116 cells**

| | **Dodecanal** | | | **Myristoleyl** | | | **Palmitoleyl** | | | **Petroselinyl** | | | **Oleyl** | | |
|---|---|---|---|---|---|---|---|---|---|---|---|---|---|---|---|
| **Concentration (µM)** | **5** | **10** | **15** | **5** | **10** | **15** | **5** | **10** | **15** | **5** | **10** | **15** | **5** | **10** | **15** |
| **7.2** | -20 | -5 | | | 19 | -22 | | | | | | | | | |
| **7.3** | 8 | 14 | | | | 27 | | 23 | | 15 | | 11 | | | |
| **7.3(L-H)** | -10 | 5 | | | 49 | 3 | 21 | 56 | 38 | 4 | 24 | 43 | 24 | | 46 |
| | | | | | | | | | | | | | | | |
| **9.2** | -7 | 3 | 11 | | -9 | -38 | | | | | | | | | |
| **9.3** | 33 | 33 | 48 | | 40 | 13 | | 25 | 45 | | | 33 | 35 | | 54 |
| | | | | | | | | | | | | | | | |
| **13.2** | 25 | 41 | 18 | | | | | | | | | | 45 | 43 | 10 |
| **13.3** | 45 | | 31 | | | -5 | | 31 | 28 | 1 | | | 55 | 46 | 14 |
| **13(Leu-His)** | 43 | 43 | 40 | | | | 43 | 49 | | 47 | 29 | | | | |
| **13(Arg-His)** | 65 | | | 62 | 24 | | 64 | | | 56 | | | | | |
| **13(3His)** | | 62 | 56 | | 45 | 29 | | 56 | | 13 | 60 | 27 | | | 31 |
| | | | | | | | | | | | | | | | |
| **17.3** | 280 | 130 | 312 | 570 | 247 | 78 | | | | 373 | 168 | 66 | | 44 | 64 |

**Table 23. siRNA delivery in Hek293 cells**

| | **Dodecanal** | | | **Myristoleyl** | | | **Palmitoleyl** | | | **Petroselinyl** | | | **Oleyl** | | |
|---|---|---|---|---|---|---|---|---|---|---|---|---|---|---|---|
| **Concentration (µM)** | **5** | **10** | **15** | **5** | **10** | **15** | **5** | **10** | **15** | **5** | **10** | **15** | **5** | **10** | **15** |
| **7.2** | | | 7 | -8 | | | | | | | | | | | |
| **7.3** | | | | 29 | | | | | 19 | | | 3 | 26 | | |
| **7.3(L-H)** | | | 14 | -3 | | | | 6 | | | | | | | |
| | | | | | | | | | | | | | | | |
| **9.2** | 14 | | 3 | | 10 | -1 | | | | | | | | | |
| **9.3** | 19 | | 40 | | 10 | 43 | | 17 | 36 | | 3 | -12 | | | 28 |
| | | | | | | | | | | | | | | | |
| **13.2** | | | | | | | | | | | | | | -7 | 17 |
| **13.3** | 52 | 44 | 48 | | | 25 | 42 | | | 18 | 0 | 20 | | 2 | |
| **13(Leu-His)** | 43 | 18 | 57 | 41 | 37 | 9 | 26 | | | 43 | 43 | 33 | | 62 | 21 |
| **13(Arg-His)** | 24 | 39 | 44 | | 48 | 25 | | | | 30 | 5 | 18 | | 39 | 47 |
| **13(3His )** | | -11 | | | | 11 | | | | | | | | 2 | |
| | | | | | | | | | | | | | | | |
| 17.3 | | | | | 48 | | | 37 | | 9 | | 52 | | | 25 |

**Table 24. siRNA delivery in HeLa cells**

| | **Dodecanal** | | | **Myristoleyl** | | | **Palmitoleyl** | | | **Petroselinyl** | | | **Oleyl** | | |
|---|---|---|---|---|---|---|---|---|---|---|---|---|---|---|---|
| **Concentration (µM)** | **5** | **10** | **15** | **5** | **10** | **15** | **5** | **10** | **15** | **5** | **10** | **15** | **5** | **10** | **15** |
| **7.2** | -19 | 11 | 10 | 2 | | | | | | | 34 | 25 | | | |
| **7.3** | -7 | 19 | 49 | | | | | | | | | 28 | | | |
| **7.3(L-H)** | 9 | | | 30 | | | | | | | 37 | 48 | | | |
| | | | | | | | | | | | | | | | |
| **9.2** | -6 | -19 | 3 | -15 | -18 | -18 | | | | | | | | | |
| **9.3** | 14 | 21 | 10 | 20 | 13 | 0 | 38 | 46 | 23 | 1 | 56 | 55 | 38 | 53 | 37 |
| | | | | | | | | | | | | | | | |
| **13.2** | | | | | | | | | | | | | | 16 | 4 |
| **13.3** | | 73 | 65 | | 64 | 60 | | 79 | 49 | | 62 | 45 | | 66 | 55 |
| **13(Leu-His)** | | | | 47 | 37 | 62 | | 61 | | | 82 | 61 | | 36 | 16 |
| **13(Arg-His)** | | | 37 | 33 | | 45 | | 42 | 42 | 70 | 42 | 35 | | | |
| **13(3His)** | | | 25 | 46 | | 13 | 72 | 24 | 62 | | 67 | 79 | | 26 | |
| | | | | | | | | | | | | | | | |
| **17.3** | 60 | | -1 | 46 | | | 60 | | -1 | | 45 | 20 | | | 27 |

**Table 25. siRNA delivery in HepG2 cells**

| | **Dodecanal** | | | **Myristoleyl** | | | **Palmitoleyl** | | | **Petroselinyl** | | | **Oleyl** | | |
|---|---|---|---|---|---|---|---|---|---|---|---|---|---|---|---|
| **Concentration (µM)** | **5** | **10** | **15** | **5** | **10** | **15** | **5** | **10** | **15** | **5** | **10** | **15** | **5** | **10** | **15** |
| **7.2** | | 14 | 7 | -12 | -1 | -32 | | | | | | | | | |
| **7.3** | | 17 | 10 | 6 | 5 | 3 | 9 | | | 18 | 19 | | 7 | 9 | |
| **7.3(L-H)** | | | | | | -13 | | | 10 | | | 1 | | -9 | |
| | | | | | | | | | | | | | | | |
| **9.2** | | | | -22 | | -22 | | | | | | | | | |
| **9.3** | | | | -11 | | 6 | -2 | | -2 | | | -3 | | -9 | |
| | | | | | | | | | | | | | | | |
| **13.2** | | 13 | -5 | | | | 16 | 4 | 7 | -11 | 2 | 0 | | | |
| **13.3** | | 31 | 33 | 13 | 20 | | 19 | 46 | 10 | 9 | 41 | 8 | | | |
| **13(Leu-His)** | | | 27 | | -3 | | | 12 | 25 | | 25 | 6 | | | |
| **13(Arg-His)** | | | | | | | | 30 | | 21 | | | | | |
| **13(3His)** | | | | | 8 | | | 6 | | -1 | 19 | | | | |
| | | | | | | | | | | | | | | | |
| **17.3** | 13 | 18 | -4 | | | -18 | 4 | | -2 | -11 | 5 | | | 8 | |

### Cas9 delivery

Tables 26-31 show the mean (triplicates) percentage of gene editing, accounted by GFP fluorescence intensity decrease (% of reduction), measured after the transfection of Cas9-RNP by different amphiphiles at different concentrations in a variety of cell lines. Background values, accounted by untreated control cells, were subtracted for processing.

**Table 26. Cas9 delivery in A549 cells**

| | **Dodecanal** | | | **Myristoleyl** | | | **Palmitoleyl** | | | **Petroselinyl** | | | **Oleyl** | | |
|---|---|---|---|---|---|---|---|---|---|---|---|---|---|---|---|
| **Concentration (µM)** | **5** | **10** | **15** | **5** | **10** | **15** | **5** | **10** | **15** | **5** | **10** | **15** | **5** | **10** | **15** |
| **7.2** | | -3 | -13 | 3 | 7 | 7 | | | | | | | | | |
| **7.3** | | -2 | 1 | 7 | 10 | 12 | 27 | 33 | 13 | 23 | 22 | 7 | -9 | | -1 |
| **7.3(L-H)** | | | -9 | 60 | 29 | 11 | 67 | 35 | -6 | 59 | 29 | 26 | 22 | 31 | 9 |
| | | | | | | | | | | | | | | | |
| **9.2** | | -22 | 4 | 9 | 7 | 14 | | | | | | | | | |
| **9.3** | | 0 | 25 | 13 | 17 | 50 | | | 39 | | 13 | 47 | | | 18 |
| | | | | | | | | | | | | | | | |
| **13.2** | | | | | | | | | | | | | 10 | 12 | 4 |
| **13.3** | | 20 | | 21 | 33 | 62 | | 33 | 48 | 12 | 32 | 56 | | 14 | 48 |
| **13(Leu-His)** | | | | 13 | 20 | 45 | | | 21 | 13 | 16 | 42 | | | |
| **13(Arg-His)** | 27 | 18 | | 5 | 9 | | 15 | 10 | 22 | 29 | | | 20 | | 15 |
| **13(3His)** | | | | 5 | 11 | 43 | | | 18 | | | | | | 4 |
| | | | | | | | | | | | | | | | |
| **17.3** | | 75 | 34 | 17 | | | 17 | 60 | 72 | 23 | | 66 | | 72 | 34 |

**Table 27. Cas9 delivery in ARPE19 cells**

| | **Dodecanal** | | | **Myristoleyl** | | | **Palmitoleyl** | | | **Petroselinyl** | | | **Oleyl** | | |
|---|---|---|---|---|---|---|---|---|---|---|---|---|---|---|---|
| **Concentration (µM)** | **5** | **10** | **15** | **5** | **10** | **15** | **5** | **10** | **15** | **5** | **10** | **15** | **5** | **10** | **15** |
| **7.2** | 0 | 0 | | 3 | | -8 | | | | | | | | | |
| **7.3** | 9 | 14 | | | | | 7 | 9 | 12 | 25 | 13 | | 22 | 35 | 42 |
| **7.3(L-H)** | | | | 19 | | 12 | 37 | 31 | | 18 | | | 1 | 6 | 15 |
| | | | | | | | | | | | | | | | |
| **9.2** | -2 | | -17 | | | | | | | | | | | | |
| **9.3** | 5 | | -14 | | | -25 | 3 | | 8 | | | -2 | | 23 | 22 |
| | | | | | | | | | | | | | | | |
| **13.2** | | | | | | | | | | | | | 13 | -7 | |
| **13.3** | | 13 | 18 | | 32 | 38 | | | | | | -11 | 20 | | |
| **13(Leu-His)** | | | | | | | | -13 | 6 | | | 22 | | -6 | |
| **13(Arg-His)** | 25 | | 21 | | 15 | 9 | 25 | -12 | 20 | | | 15 | 14 | -6 | |
| **13(3His)** | | | | | | | | | | | | 34 | | | |
| | | | | | | | | | | | | | | | |
| **17.3** | | | | | 44 | | 12 | 60 | 31 | | 26 | 27 | | 45 | -9 |

**Table 28. Cas9 delivery in HCT116 cells**

| | **Dodecanal** | | | **Myristoleyl** | | | **Palmitoleyl** | | | **Petroselinyl** | | | **Oleyl** | | |
|---|---|---|---|---|---|---|---|---|---|---|---|---|---|---|---|
| **Concentration (µM)** | **5** | **10** | **15** | **5** | **10** | **15** | **5** | **10** | **15** | **5** | **10** | **15** | **5** | **10** | **15** |
| **7.2** | | | | -18 | | | | | | | | | | | |
| **7.3** | | | | -11 | | | 4 | -12 | 17 | 35 | 39 | 34 | | -7 | 4 |
| **7.3(L-H)** | | | | 3 | | | 26 | 28 | 22 | | | | | -5 | 5 |
| | | | | | | | | | | | | | | | |
| **9.2** | -34 | -15 | | -22 | | | | | | | | | | | |
| **9.3** | 7 | 1 | | -8 | | | -11 | -21 | | -38 | | | 4 | 24 | 14 |
| | | | | | | | | | | | | | | | |
| **13.2** | | | | | | | | | | | | | | -6 | |
| **13.3** | 25 | 10 | | 20 | | | 10 | -18 | | 18 | | | | -1 | |
| **13(Leu-His)** | -28 | -5 | | | 22 | 105 | | 4 | | 0 | 18 | | | 7 | |
| **13(Arg-His)** | 4 | 5 | | 16 | -7 | | 29 | 30 | 22 | 9 | | 6 | | 5 | |
| **13(3His)** | -33 | | | | | | 0 | -9 | | 14 | 26 | | | | |
| | | | | | | | | | | | | | | | |
| **17.3** | -2 | | | 22 | | | 26 | | | | -4 | | | | |

**Table 29. Cas9 delivery in Hek293 cells**

| | **Dodecanal** | | | **Myristoleyl** | | | **Palmitoleyl** | | | **Petroselinyl** | | | **Oleyl** | | |
|---|---|---|---|---|---|---|---|---|---|---|---|---|---|---|---|
| **Concentration (µM)** | **5** | **10** | **15** | **5** | **10** | **15** | **5** | **10** | **15** | **5** | **10** | **15** | **5** | **10** | **15** |
| **7.2** | -21 | | -29 | -23 | | | | | | | | | | | |
| **7.3** | -11 | | -15 | -20 | | | 23 | -1 | 3 | 29 | 34 | 23 | | -34 | |
| **7.3(L-H)** | | | | -6 | | | 23 | -5 | -2 | 1 | | | -5 | 6 | 0 |
| | | | | | | | | | | | | | | | |
| **9.2** | -38 | -55 | | | | | | | | | | | | | |
| **9.3** | -11 | -53 | | | | | | | | -12 | | | 12 | -5 | |
| | | | | | | | | | | | | | | | |
| **13.2** | -23 | -26 | | | | | | | | | | | | -10 | -13 |
| **13.3** | 18 | -18 | | -12 | | -48 | -7 | | | | | | | | |
| **13(Leu-His)** | | | | | -16 | | -8 | -33 | -39 | 16 | 7 | -32 | | -4 | |
| **13(Arg-His)** | 21 | | | | | -75 | 1 | 5 | -26 | 8 | -19 | -44 | | -8 | -8 |
| **13(3His)** | -4 | | | -13 | | | -16 | | -47 | 21 | 4 | -13 | | | |
| | | | | | | | | | | | | | | | |
| **17.3** | | | | | | | -19 | -32 | -34 | 2 | -37 | | | | |

**Table 30. Cas9 delivery in HeLa cells**

| | **Dodecanal** | | | **Myristoleyl** | | | **Palmitoleyl** | | | **Petroselinyl** | | | **Oleyl** | | |
|---|---|---|---|---|---|---|---|---|---|---|---|---|---|---|---|
| **Concentration (µM)** | **5** | **10** | **15** | **5** | **10** | **15** | **5** | **10** | **15** | **5** | **10** | **15** | **5** | **10** | **15** |
| **7.2** | 6 | -22 | -26 | -7 | -18 | -6 | | | | | | | | | |
| **7.3** | 10 | 2 | -3 | 24 | 27 | 25 | 20 | 13 | | 6 | 11 | 33 | | -9 | |
| **7.3(L-H)** | | -8 | -21 | 39 | 22 | 18 | 55 | 28 | 17 | 46 | 14 | | 16 | 13 | 22 |
| | | | | | | | | | | | | | | | |
| **9.2** | 6 | 14 | 47 | -12 | | 52 | | | | | | | | | |
| **9.3** | 25 | 61 | 80 | 14 | | 81 | | 44 | 77 | | 49 | 77 | 11 | 26 | |
| | | | | | | | | | | | | | | | |
| **13.2** | | | | | | | | | | | | | 21 | 62 | |
| **13.3** | | | | 29 | | 86 | 18 | 68 | 80 | | | 85 | | | |
| **13(Leu-His)** | | | | 16 | 73 | | | | | 39 | 74 | 81 | | | |
| **13(Arg-His)** | 37 | 78 | | 48 | 85 | | 48 | 73 | 78 | 23 | 69 | 80 | 46 | 73 | |
| **13(3His)** | | | | 18 | | | | | | | | | | | |
| **17.3** | 81 | 87 | 78 | 57 | | | 35 | 90 | 88 | 48 | | 87 | | 90 | 82 |

**Table 31. Cas9 delivery in HepG2 cells**

| | **Dodecanal** | | | **Myristoleyl** | | | **Palmitoleyl** | | | **Petroselinyl** | | | **Oleyl** | | |
|---|---|---|---|---|---|---|---|---|---|---|---|---|---|---|---|
| **Concentration (µM)** | **5** | **10** | **15** | **5** | **10** | **15** | **5** | **10** | **15** | **5** | **10** | **15** | **5** | **10** | **15** |
| **7.2** | -23 | -56 | -47 | -42 | -40 | -21 | | | | | | | | | |
| **7.3** | -21 | -24 | -29 | -19 | -20 | -14 | -14 | -14 | -4 | | -1 | | -7 | -3 | -1 |
| **7.3(L-H)** | | -28 | -28 | -16 | -25 | -19 | -23 | -12 | -25 | | -20 | | -17 | -13 | -12 |
| | | | | | | | | | | | | | | | |
| **9.2** | | -35 | -35 | | -17 | | | | | | | | | | |
| **9.3** | | -29 | -26 | | -5 | | | | | -24 | -19 | | -10 | 3 | |
| | | | | | | | | | | | | | | | |
| **13.2** | -36 | -41 | 9 | | | | | | | | | | | -21 | -33 |
| **13.3** | -27 | -11 | | -22 | 3 | | -30 | -19 | | -20 | | | | | |
| **13(Leu-His)** | | -37 | | | 11 | | -36 | 8 | 13 | -7 | 9 | 14 | | | -22 |
| **13(Arg-His)** | -31 | -10 | 14 | 10 | 33 | | -8 | 26 | 7 | -27 | | | | -4 | 15 |
| **13(3His)** | -33 | -34 | | | -1 | | -28 | -24 | -15 | -16 | | | | | |
| **17.3** | | | | 6 | 38 | | -2 | 31 | 31 | -2 | 39 | 35 | | | |

### Example 4: Intracellular delivery of Cas9 ribonucleoprotein

The inventors evaluated the intracellular delivery of Cas9 ribonucleoprotein with a gRNA targeting EGFP into HeLa-EGFP cells, using an amphiphilic molecule comprising peptide 7.3(Leu-His) combined with myristoleyl, palmitoleyl or petroselinyl aldehydes. After 3 days, EGFP negative cells were quantified by flow cytometry.

Figure 2 shows that the amphiphilic molecules comprising peptide 7.3(Leu-His) and myristoleyl or palmitoleyl aldehydes reached 70% of editing as compared with the 30% editing achieved by the control with Lipofectamine^{®} CRISPRMAX. Moreover, the concentration of cells obtained with both amphiphiles is, on average, three times higher than the concentration of cells obtained with Lipofectamine^{®}, showing that both amphiphiles achieve an improved level of cell viability as compared with Lipofectamine^{®}.

### Example 5: Transfection experiments with D-peptide

7.3(Leu-His) peptide with D-amino acids was synthesized. This peptide would be more resistant to the degradation by proteases. The inventors studied the delivery of EGFP pDNA using amphiphiles comprising either enantiomer (either 7.3(Leu-His) peptide with D-amino acids or with L-amino acids) and myristoleyl aldehyde to compare their transfecting activity in HeLa cells. Number of EGFP expressing cells was measured by flow cytometry. Figure 3 shows that both peptides perform equally efficiently.

### Example 6: Transfection experiments in confluent cells

Experiments in cultured cells at different confluence levels were performed to check the effect of this parameter on the activity of amphiphile 7.3(Leu-His) M. As described above, transfection experiments are usually performed on cells seeded the day before at a cell concentration of 60,000 cell/mL (100 µL/well). In the current example, transfection experiments with EGFP pDNA are performed in HeLa cells two and four times more concentrated (120,000 and 240,000 cells/mL, respectively). Figure 4 shows that the above-mentioned amphiphile performs equally well at all cell concentrations tested. However, for this experiment more volume of complex amphiphile/pDNA was needed as the cell concentration was increased (50, 100 and 200 µL of complexes were used for 60,000, 120,000 and 240,000 cells/mL, respectively) .

### Example 7: Transfection experiments in chicken embryo fibroblasts

The amphiphile obtained by combining the peptide 7.3(Leu-His) with myristoleyl aldehyde (7.3(Leu-His) M) was used to deliver EGFP pDNA in primary chicken embryo fibroblasts (CEF, obtained from 9-10 day-old chicken embryos), which are non-immortal cells difficult to transfect. The transfection efficiency of amphiphile 7.3(Leu-His) M was compared to that of Lipofectamine^{®} 2000 (LF) and *Trans*IT-LT1 Transfection Reagent (Mirus). Figure 5 shows that the transfection efficiency of amphiphile 7.3(Leu-His) M was better than that of both controls (LF and Mirus), being the amphiphile of the invention two times better than Lipofectamine^{®} 2000 (LF) and up to five times better than *Trans*IT-LT1 Transfection Reagent (Mirus).

### Example 8: Cell viability for amphiphile 7.3(Leu-His) M

MTT assays were performed in order to determine the IC50 of the amphiphile comprising 7.3(Leu-His) peptide with myristoleyl aldehyde (7.3(Leu-His) M) in six cell lines. As shown in Figure 18, the IC50 is between 13-24 µM for all the cell lines except for HepG2 where the IC50 is around 60 µM.

### Example 9: Short peptides

Several structural modifications were performed on the peptide 7.3(Leu-His). The delivery of EGFP pDNA in HeLa cells using amphiphiles comprising these peptides and myristoleyl aldehyde was tested. The modifications consisted of the modification in the order of the amino acids, the replacement of 1 to 3 K* residues for O* residues and the addition of 1 or 2 R residues on the N-terminus. In SEQ ID NO: 53 and SEQ ID NO: 55, the final residue, either H or L, respectively, was eliminated. All peptides have the same number of hydrazide-modified reactive basic amino acids (K* or O*). Some of the structures can be seen in the Table 32 below. The synthesised short peptides with L-amino acids are as follows:

| | | |
|---|---|---|
| 7.3(Leu-His) | Ac-RK*HRK*K*H-NH₂ | SEQ ID NO: 1 |
| V1-7.3(Leu-His) | Ac-RHK*K*K*HR-NH₂ | SEQ ID NO: 2 |
| V2-7.3(Leu-His) | Ac-RK*HHK*K*R-NH₂ | SEQ ID NO: 3 |
| 7.3(L-H)(K-O) | Ac-RO*HRO*O*H-NH₂ | SEQ ID NO: 52 |
| R + 7.3(L-H) - H | Ac-RRK*HRK*K*NH₂ | SEQ ID NO: 53 |
| R + 7.3(L-H) | Ac-RRK*HRK*K*H-NH₂ | SEQ ID NO: 54 |
| R + 7.3 - L | Ac-RRK*LRK*K*NH₂ | SEQ ID NO: 55 |
| R + 7.3 | Ac-RRK*LRK*K*L-NH₂ | SEQ ID NO: 56 |
| RR + 7.3 | Ac-RRRK*LRK*K*L-NH₂ | SEQ ID NO: 57 |
| V3-7.3(L-H) | Ac-RO*HRK*K*H-NH₂ | SEQ ID NO: 58 |
| V4-7.3(L-H) | Ac-RK*HRK*O*H-NH₂ | SEQ ID NO: 59 |

**Table 32. Peptide sequence modifications derived from 7.3(Leu-His). (K* = hydrazide-modified lysine, see general synthetic scheme; O* = hydrazide-modified ornithine).**

| | | |
|---|---|---|
| 7.3(Leu-His) | 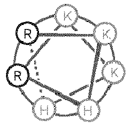 | Ac-RK^{*}HRK^{*}K^{*}H-NH₂ |
| V1-7.3(Leu-His) | 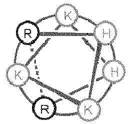 | Ac-RHK*K*K*HR-NH₂ |
| V2-7.3(Leu-His) | 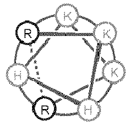 | Ac-RK*HHK*K*R-NH₂ |

Amphiphile comprising peptide V1-7.3(Leu-His) and myristoleyl aldehyde generally show the same transfection efficiency as compared with amphiphile 7.3(Leu-His) M, with the exception of A549 cell line in which efficiency is about half (see Figure 6). Amphiphiles comprising peptide V2-7.3(Leu-His) and myristoleyl aldehyde show about 50% of the transfection efficiency of amphiphiles V1-7.3(Leu-His) M or 7.3(Leu-His) M, except for A549 cell line in which V2-7.3(Leu-His) M shows an enhanced efficiency matching V1-7.3(Leu-His) M transfection efficiency. In all these cases, as described above, myristoleyl aldehyde as hydrophobic tail was used.

### Example 9: In vivo transfection protocol

*In vivo* bioluminescence imaging (BLI) enables localization and quantification of expression following mRNA delivery in living animals.

### Predilution formulation

The inventors evaluated the mRNA delivery efficiency of amphiphile by i.m. injections of an amphiphile-complexed FLuc mRNA in anesthetized mice using BLI. Amphiphilic molecule stock solutions were prepared in DMSO as described above and diluted in a suitable buffer, such as PBS, or phosphate buffer enriched with inorganic salts. The solutions of mRNA/amphiphile complexes were freshly prepared prior to *in vivo* experiments. Amphiphile solutions in a suitable buffer, such as PBS, or phosphate buffer enriched with inorganic salts, were mixed with mRNA solutions in a suitable buffer, such as PBS, or phosphate buffer enriched with inorganic salts (1:1 volume ratio, final amount of mRNA per mice = 5 µg/mouse). The mixture was shaken for 30 min at room temperature before injection. For each mouse, 5 µg mRNA was complexed with the best candidates obtained from the *in vitro* experiments, 7.3(Leu-His) with myristoleyl aldehyde (7.3(Leu-His) M) (Figure 7) or with palmitoleyl aldehyde (7.3(Leu-His) PA) (Figure 8), and administered by i.m. injection in the right flank in 50 µL of a suitable buffer, such as PBS, or phosphate buffer enriched with inorganic salts. As control, 5 µg of naked mRNA was injected in three mice. Luciferase expression was evaluated over 48 h, starting at 1 h after the administration of mRNA complexes. High levels of luciferase activity were observed at the site of injection for both amphiphilic compounds. This expression peaked at 7 h with all the concentrations tested in the case of 7.3(Leu-His) M (Figure 7), whereas 7.3(Leu-His) PA exhibited a fluctuation in the best performing amphiphile concentration over time, being the formulation with 2.5 µM more efficient at shorter times after injection and 10 µM more efficient at longer observation times (Figure 8). In contrast, i.m. injections of naked mRNA afforded only low levels of luciferase expression, as measured by photon flux, in all mice. The experiment was performed in, at least, triplicates (3 mice per condition).

After obtaining the conditions providing the highest expression, a second round of i.m. delivery of FLuc mRNA was performed to further optimize the formulation of the complexes. Under totally analogous experimental conditions, the best observed concentration of amphiphile 7.3(Leu-His) M (2.5 µM) was fixed and complexes with increasing amounts of FLuc mRNA (1.25, 3 or 5 µg/mouse) were prepared and administered i.m. as described above (Figure 9). This experiment revealed that the highest bioluminescence, and thus, the highest levels of FLuc expression, was achieved with the complexes formed by 2.5 µM amphiphile and 5 µg/mouse of FLuc mRNA. Additionally, these optimized conditions were further screened for the delivery of FLuc mRNA utilizing other administration routes, such as intradermal (i.d.) and subcutaneous (s.c.) (Figure 14, 5µg FLuc mRNA was used in both cases) or intravenous (i.v.) (Figure 10). For i.d. and s.c. injections, the previously optimized conditions performed less efficiently than i.m. delivery (Figure 14).

In the case of i.v. administration (tail vein), luciferase expression was evaluated over 24 h, starting at 1 h after the administration of mRNA complexes. After that time, the specimens were sacrificed, and each organ was analysed individually using BLI for quantification of the expression. In this case, preferred conditions involved the injection of 50 µL of complex solution in a suitable buffer, such as PBS, or phosphate buffer enriched with inorganic salts, comprising 2.5 µM 7.3(Leu-His) M and 1.3 µg/mouse FLuc mRNA. Under all conditions tested, a preferred spleen biodistribution of the bioluminiscence was observed, followed by liver expression and, to a lesser extent, in the lungs (Figure 10).

### Concentrated formulation

Amphiphile stock solutions were prepared in DMSO as described above, but in this case, the amphiphile was contacted directly with the solution of nucleic acid cargo in a suitable buffer, such as PBS, or phosphate buffer enriched with inorganic salts, without any predilution step of the amphiphile's DMSO solution in a suitable aqueous buffer. Delivery efficiency of mRNA by i.v. administration route was determined by bioluminescence imaging (BLI). Fluc mRNA (5 µg/mouse) was contacted with concentrated 7.3(L-H) M amphiphile-DMSO solutions at various aqueous-organic volume ratios (Fig. 16, 5 µM amphiphile concentration) for 30 min, and administered in 200 µL (5 µg mRNA/mouse) in the tail vein. Luciferase expression was evaluated over 24 h, starting at 1 h after the administration of mRNA complexes. After that time, mice were sacrificed, and each organ was analysed individually using bioluminescence imaging (BLI) for quantification of the expression. Under all conditions tested, a preferred spleen biodistribution of the bioluminescence was observed. From all volume ratios tested, complexes prepared utilizing 1:100 (DMSO:aqueous) ratios showed the highest bioluminescence values, with up to one order of magnitude luminescence enhancement as compared to 1:200 ratio. Transfection results with the optimal volume ratios (125 or 250 µM final amphiphile concentration) and expression quantification in organs for candidates 7.2 DO, 7.2 M, 9.2 DO and 9.2 M are presented in Figures 12, 11, 13, and 20 respectively.

Figure 19 shows *in vivo* transfection experiments in mice using amphiphiles comprising peptide 13.2 with oleyl aldehyde (13.2 O). This figure shows representative bioluminescence images of mice and the extracted organs following i.v. (intravenous) injection of FLuc mRNA/amphiphile 13.2 O complexes (125 and 250 µM of 13.2 O were used, as indicated) at different times (1 h, 4 h, 7 h and 24 h). The quantification of organs' bioluminescence is shown in Figure 19B. Complexes were formed as described above with 5 µg FLuc mRNA. As it can be seen in Figure 19, amphiphiles comprising peptide 13.2, i.e., with two hydrophobic tails, show very limited *in vivo* delivery as compared with the same amount of mRNA delivered with the amphiphile 7.3(Leu-His) M (Figure 16).

### Example 10: DLS measurements for complex comprising 7.3M or 7.3(L-H) M and pDNA (100 ng, in vitro conditions) or mRNA (50 ng, in vitro conditions)

The complexes formed after mixing EGFP pDNA (100 ng) or mRNA (50 ng) with 7.3 M or 7.3(L-H) M amphiphiles at a range of concentrations (2.5-10 µM) and pH values (4-10) were characterized by DLS to account for their size (mean diameter) and surface charge (ζ potential), as shown in Fig. 15. In particular, Fig. 15 shows the following:
A) Top panel: 7.3 M + pDNA complexes exhibit mean diameters from 70 to 200 nm, where results for amphiphile concentration of 2.5 and 10 µM are very similar (70-100 nm) and invariable through the whole pH range. Result for 5 µM show larger particles, whose diameter peaks at neutral pH (200 nm). These results demonstrate optimal sizes for cellular delivery.
   Bottom panel: For 2.5 and 5 µM, pH increase results in a decrease in the surface charge of the particles (-5 to -40 mV and +35 to -30 mV, respectively), whereas ζ potential result for 10 µM remains positive independently of pH value; +20 to +30). Thus, the concentration of cationic amphiphile present in the formulation is a key factor in the correct surface charge balance of the formulated pDNA/amphiphile complexes.
B) Top panel: 7.3(L-H) M + pDNA complexes increase their mean diameter when pH is increased at all concentrations tested, almost doubling their size from pH 4 to pH 10 values.
   Bottom panel: complexes formulations see their surface potential reduced when increasing the pH at all concentrations tested. The higher the amphiphile concentration, the larger the drop in observed ζ potential, going from +60 to -45 mV at 10 µM and pH 4 and 10, respectively.
C) Top panel: 7.3 M + mRNA complexes present a very low variability in their mean diameter throughout the whole range of concentrations and pH values tested. All recorded values between 50 and 100 nm makes them optimal intracellular delivery candidates.
   Bottom panel: surface potential results are positive in all cases (+5 to +30 mV) encouraging interaction with negatively charged cell membrane environment. There is a tendency to increase ζ potential with amphiphile concentration and pH value.
D) Top panel: 7.3(L-H) M + mRNA complexes present a very low variability in their mean diameter throughout the whole range of concentrations and pH values tested (100-150 nm), with the sole exception of particles formulated at pH 7 and lower amphiphile concentrations, whose mean diameter grows considerably until i.e., 500 nm.
   Bottom panel: as expected, increasing the amphiphile concentration results in an enhancement on the cationic character of the surface potential of the particles, a tendency that is also observed when rising the pH of the medium.

### Example 11: SARS-CoV-2 immunization

A codon optimized (based on a previously described sequence) mRNA (a custom RNA syntesized by TriLink, capped, polyadenylated and fully substituted with N1-methylpseudouridine) encoding a prefusion stabilized form of coronavirus SARS-CoV-2 spike protein (protein S, with the substitutions of the residues ⁹⁸⁶KV⁹⁸⁷ by ⁹⁸⁶PP⁹⁸⁷ and the elimination of the furin cleavage site ⁶⁸²RRAR⁶⁸⁵ by subtitution for ⁶⁸²GSAS⁶⁸⁵, ORF sequence in SEQ ID NO: 27) was administered i.v. formulated (concentrated formulation, Example 9) with the amphiphile 7.3(L-H) M with or without the well-known adjuvant TLR9 agonist CpG (Invivogen ODN2395, 5'-TCGTCGTTTTCGGCGCGCGCCG-3' (SEQ ID NO: 60), bases are phosphorothioates).

Amphiphilic molecule stock solutions were prepared in DMSO as described above for the concentrated formulation. Mice were injected with 5 µg of mRNA formulated with 2 µL of amphiphile (50 mM) or with 5 µg of mRNA plus 2 µg of CpG formulated with 2.8 µL of amphiphile (50 mM). Mice were vaccinated by i.v. injection and were boosted (with identical formulations) 21 days after the first injection. For i.v. administration, 200 µL of formulated mRNA/amphiphile was administered by tail vein injection. 14 and 35 days post-injection, blood was collected in Eppendorf tubes and allowed to settle for 3-4 h at room temperature. After 10 min centrifugation at 10,000 g, the supernatant was collected and frozen at-80°C until IgG quantitation was performed by ELISA.

ELISA: Nunc-Immuno microwell 96-well ELISA plates were coated overnight with 100 µL/well of 1 µg/mL SARS-CoV-2 Spike S1-His recombinant protein (from Sinobiological) in PBS buffer pH 7.2. After 3 washes in ELISA wash buffer (PBS with 0.05% Tween-20, PBS-T), wells were blocked for 1 h at rt with 200 µL of 3% nonfat dry milk diluted in PBS-T. Serum dilutions (1:50 and 1:250) were prepared in PBS-T containing 1% nonfat dry milk. The blocking solution was removed, and wells were washed two times with 200 µL of PBS-T. Then, 100 µL of each serum dilution was added to the plate for 2 h with shaking (70 rpm) and at rt. Wells were washed three times with 200 µL PBS-T and incubated with Goat Anti-Mouse IgG Fc, Human/Bovine/Horse SP ads-HRP (Southern Biotech) (1:2,000) in 1% nonfat dry milk, for 1 h at rt and with shaking (70 rpm). Wells were washed three times with PBS-T, and 100 µL of SureBlue TMB microwell peroxidase substrate was added to each well. Color was developed for 30 min, and then, the reaction was stopped with 100 µL of HCI 1M. Optical density at 450 nm (OD450) was measured using a Tecan Infinite F200Pro microplate reader (Figure 17). OD450 of serum samples collected at 14 days post-injection showed values above background (three times the blank) value just for the formulation containing the CpG adjuvant at lowest serum dilution factor. Samples from 35 days post-injection revealed positive results for formulations with and without CpG adjuvant at both serum dilution factors.

### Example 12: OVA immunization

mRNA encoding ovalbumin (OVA; from TriLink, capped, polyadenylated and fully substituted with 5-methoxyuridine, ORF in SEQ ID NO: 63) was administered i.v. formulated (concentrated formulation, example 9) with the amphiphile 7.3(L-H) M with or without the well-known adjuvant TLR9 agonist CpG (Invivogen ODN2395, 5'-TCGTCGTTTTCGGCGCGCGCCG-3' (SEQ ID NO: 60), bases are phosphorothioates). Amphiphilic molecule stock solutions were prepared in DMSO as described above for the concentrated formulation. Mice were injected with 5 µg of mRNA formulated with of amphiphile (125 µM) or with 5 µg of mRNA plus 2 µg of CpG formulated with amphiphile (175 µM). Mice were vaccinated by i.v. injection and were boosted (with identical formulations) 21 days after the first injection. For i.v. administration, 200 µL of formulated mRNA/amphiphile was administered by tail vein injection. 14 and 35 days post-injection, blood was collected in Eppendorf tubes and allowed to settle for 3-4 h at room temperature. After 10 min centrifugation at 10,000 g, the supernatant was collected and frozen at -80°C until IgG quantitation was performed by ELISA.

ELISA: Nunc-Immuno microwell 96-well ELISA plates were coated overnight with 100 µL/well of 1 µg/mL OVA (Sigma-Aldrich, CAS: 9006-59-1) in PBS buffer pH 7.2. After 3 washes in ELISA wash buffer (PBS with 0.05% Tween-20, PBS-T), wells were blocked for 1 h at rt with 200 µL of 3% nonfat dry milk diluted in PBS-T. Serum dilutions (1:50 and 1:250) were prepared in PBS-T containing 1% nonfat dry milk. The blocking solution was removed, and wells were washed two times with 200 µL of PBS-T. Then, 100 µL of each serum dilution was added to the plate for 2 h with shaking (70 rpm) at rt. Wells were washed three times with 200 µL PBS-T and incubated with Goat Anti-Mouse IgG Fc, Human/Bovine/Horse SP ads-HRP (Southern Biotech) (1:2,000) in 1% nonfat dry milk, for 1 h at rt and with shaking (70 rpm). Wells were washed three times with PBS-T, and 100 µL of SureBlue TMB microwell peroxidase substrate was added to each well. Color was developed for 10 min, and then, the reaction was stopped with 100 µL of HCl 1M. Optical density at 450 nm (OD450) was measured using a Tecan Infinite F200Pro microplate reader (Figure 21A).

Additionally, serial dilutions of sera obtained at day 35 (from 10² to 10⁹) were analyzed by ELISA using secondary Goat Anti-Mouse IgG1 or IgG2c Human/Bovine/Horse SP ads-HRP antibodies (1:2,000, Southern Biotech) to determine the titer of the different isotypes and the type of immune response (Figure 21B).

The type of immune response induced after immunisation can be classified as Th1, Th2 or mixed response, that differ on the CD4+ T-cell subsets involved, the cytokines secreted, and the antibody classes induced. A Th1 response is typically associated with delayed type hypersensitivity responses and cell-mediated immunity, while Th2 is associated with the induction of humoral responses and the production of antibodies.

The high-titers of IgG2c with respect to IgG1 indicate a Th1-biased immune response, which is generally preferred for cancer therapy and most viral infections.

The present invention provides the following items.
1. A peptide or salt thereof, preferably a cationic peptide
   - wherein the peptide or salt thereof has a length of 7 to 21 amino acids,
   - wherein the peptide or salt thereof comprises basic amino acid residues selected from the group consisting of arginine (R), histidine (H) and reactive basic amino acid residues containing a reactive group on its side chain,
   - wherein the peptide or salt thereof optionally comprises one or more hydrophobic amino acid selected from the group consisting of alanine (A), valine (V), leucine (L) and isoleucine (I), wherein, if present, the hydrophobic amino acid represent less than 50% of the total number of amino acids in the peptide,
   - wherein the number of arginines in the peptide or salt thereof is from 2 to 10,
   - wherein the number of histidines in the peptide or salt thereof is from 0 to 3,
   - wherein the number of reactive basic amino acid residues containing a reactive group in its side chain in the peptide or salt thereof is 3,
   - wherein the number of hydrophobic amino acids in the peptide or salt thereof is 0 to 8 and
   - wherein in the peptide amino acid sequence there are not more than two consecutive amino acids of the same residue, except for the reactive basic amino acid residue containing a reactive group in its side chain and the amino acid arginine, in which cases there can be a maximum of three consecutive arginines and/or a maximum of three consecutive reactive basic amino acid residues containing a reactive group in its side chain, and
   - wherein the amino acids comprised in the peptide or salt thereof are L-amino acids and/or D-amino acids, preferably wherein all of the amino acids comprised in the peptide or salt thereof are L-amino acids or all of the amino acids comprised in the peptide or salt thereof are D-amino acids.
2. The peptide or salt thereof according to item 1, wherein the three reactive basic amino acid residues containing a reactive group in its side chain are reactive lysines (K*), reactive ornithines (O*) or mixtures thereof, and/or wherein the amino acid in the *N*-terminal end of the peptide is R.
3. The peptide or salt thereof according to item 2, wherein the reactive lysine (K*) is a native lysine or a modified lysine, wherein the modified lysine is characterized in that the reactive group is connected to the ε-amino group in the reactive lysine by an amide bond, said reactive group and amide bond being connected by a spacer, preferably wherein the spacer has from 1 to 10 carbon atoms, such as from 2 to 10 carbon atoms, preferably from 2 to 6 carbon atoms, more preferably 4 carbon atoms.
4. The peptide or salt thereof according to item 2, wherein the reactive ornithine (O*) is a native ornithine or a modified ornithine, wherein the modified ornithine is characterized in that the reactive group is connected to the δ-amino group in the ornithine by an amide bond, said reactive group and amide bond being connected by a spacer, preferably wherein the spacer has from 1 to 10 carbon atoms, such as from 2 to 10 carbon atoms, preferably from 2 to 6 carbon atoms, more preferably 4 carbon atoms.
5. The peptide or salt thereof according to any one of items 3 or 4, wherein the reactive group is selected from the group consisting of a hydrazide group, an amino group, a carboxylic acid, and an aminooxy-carboxylic acid group, preferably wherein the reactive group is a hydrazide group.
6. The peptide or salt thereof according to any one of items 3-5, wherein the modified lysine or the modified ornithine is obtained by reacting the peptide with a hydrazide carboxylic acid, preferably glutaric acid monohydrazide, thereby resulting in the modified lysine or the modified ornithine, wherein the reactive group is a hydrazide group.
7. The peptide or salt thereof according to any one of items 1 to 6, wherein the peptide has a length of 7 amino acids, wherein the peptide consists of basic amino acids selected from arginines, histidines and reactive basic amino acid residues containing a reactive group, wherein the number of arginines in the peptide is at least 2, wherein the number of histidines in the peptide is 2 or less, and wherein the number of reactive basic amino acid residues containing a reactive group in its side chain in the peptide is 3.
8. The peptide or salt thereof according to item 7, wherein the peptide sequence is selected from the group consisting of Ac-RK^{*}HRK^{*}K^{*}H-NH₂ (SEQ ID NO: 1), Ac-RHK*K*K*HR-NH₂ (SEQ ID NO: 2), Ac-RK*HHK*K*R-NH₂ (SEQ ID NO: 3), Ac-RO^{*}HRO^{*}O^{*}H-NH₂ (SEQ ID NO: 52), Ac-RRK^{*}HRK^{*}K^{*-}NH₂ (SEQ ID NO: 53); Ac-RO*HRK*K*H-NH₂ (SEQ ID NO: 58) and Ac-RK*HRK*O*H-NH₂ (SEQ ID NO: 59).
9. The peptide or salt thereof according to any one of items 1 to 6, wherein the peptide has a length of at least 9 amino acids, wherein the peptide comprises a core sequence RX₁X₂R (SEQ ID NO: 9) or RX₁X₂RRX₃X₄ (SEQ ID NO: 4), and wherein any one of X₁ to X₄ is an amino acid selected from the group consisting of H, K*, O* and a hydrophobic amino acid, wherein the hydrophobic amino acid is selected from the group consisting of A, V, L and I.
10. The peptide or salt thereof according to item 9, wherein the peptide is a peptide of sequence RX₁X₂RRX₃X₄RX₅ (SEQ ID NO: 5), RRX₁X₂RX₃X₄RRX₅X₆RX₇ (SEQ ID NO: 6); RRX₁X₂RX₃X₄RRX₅X₆RX₇X₈RRX₉ (SEQ ID NO: 7), RRX₁X₂RX₃X₄RRX₅X₆RX₇X₈RRX₉RRLL (SEQ ID NO: 8) or RX₁X₂X₃RX₄X₅RRX₆X₇RX₈ (SEQ ID NO: 10), wherein any one of X₁ to X₉ is an amino acid selected from the group consisting of H, K*, O* and a hydrophobic amino acid, wherein the hydrophobic amino acid is selected from the group consisting of A, V, L and I.
11. The peptide or salt thereof according to item 10 wherein the peptide sequence is selected from the group consisting of Ac-RK*LRRK*LRK*-NH₂ (SEQ ID NO: 13), Ac-RRLK*RLK*RRLK*RL-NH₂ (SEQ ID NO: 14), Ac-RRLK*RK*LRRLK*RL-NH₂ (SEQ ID NO: 15), Ac-RRK*K*RK*LRRLLRL-NH₂ (SEQ ID NO: 16), Ac-RRHK*RLK*RRLK*RL-NH₂ (SEQ ID NO: 17), Ac-RHLK*RLK*RRLK*RL-NH₂ (SEQ ID NO: 18), Ac-RHLK*RHK*RRLK*RH-NH₂ (SEQ ID NO: 19), Ac-RRLK*RLLRRLK*RLK*RRL-NH₂ (SEQ ID NO: 21), Ac-RRLK*RLLRRLK*RLK*RRLRRLL-NH₂ (SEQ ID NO: 23).
12. The peptide or salt thereof according to any of items 1 to 6 wherein the peptide sequence is selected from the group consisting of Ac-RKLRK*K*L-NH₂ (SEQ ID NO: 11), Ac-RK*RRK*K*R-NH₂ (SEQ ID NO: 12), Ac-RK*HK*HK*R-NH₂ (SEQ ID NO: 49), Ac-RK*LRRK*LRK*-NH₂ (SEQ ID NO: 13), Ac-RRK^{*}HRK^{*}K^{*}H-NH₂ (SEQ ID NO: 54), Ac-RRK*LRK*K* (SEQ ID NO: 55), Ac-RRK^{*}LRK^{*}K^{*}L-NH₂ (SEQ ID NO: 56) and Ac-RRRK*LRK*K*L-NH₂ (SEQ ID NO: 57).
13. The peptide or salt thereof according to any of items 1 to 12 wherein the peptide is N-acylated and/or C-amidated.
14. An amphiphilic molecule comprising or, alternatively, consisting of:
   a) A peptide or salt thereof as defined in any one of items 1 to 13 and
   b) Three hydrophobic tails,
   wherein each hydrophobic tail is connected to the peptide by a bond formed by a reaction between (i) the reactive group in the side chain of the reactive basic amino acid residue and (ii) a precursor of the hydrophobic tail, wherein the precursor of the hydrophobic tail comprises the hydrophobic tail and a second reactive group which can react with the reactive group in the side chain of the reactive basic amino acid residue in the peptide.
15. The amphiphilic molecule according to item 14, wherein the amphiphilic molecule is obtained by reacting the peptide or salt thereof with a precursor of the hydrophobic tail, said precursor comprising a second reactive group which reacts with the reactive group in the reactive basic amino acid residue, forming a covalent bond.
16. The amphiphilic molecule according to items 14 or 15, wherein the bond formed by a reaction between the reactive group in the side chain of the reactive basic amino acid residue and the second reactive group in the precursor of the hydrophobic tail is an amide bond, an ester bond, a hydrazone bond or an oxime bond, preferably a hydrazone bond.
17. The amphiphilic molecule according to item 16, wherein the second reactive group in the hydrophobic tail precursor is an aldehyde, a carboxylic acid or an alcohol, preferably an aldehyde, and wherein the aldehyde is selected from the group consisting of hexanal, octanal, decanal, dodecanal, myristoleyl aldehyde, palmitoleyl aldehyde, petroselinyl aldehyde, oleyl aldehyde, linoleoyl aldehyde, eicosenoyl aldehyde and tetracosenoleyl aldehyde, preferably selected from myristoleyl aldehyde, palmitoleyl aldehyde, and petroselinyl aldehyde.
18. The amphiphilic molecule according to items 14-17, wherein the reactive basic amino acid residue containing a primary amine in its side chain is a reactive lysine (K*) or a reactive ornithine (O*) and wherein the covalent bond is formed between the second reactive group in the hydrophobic tail precursor and the native lysine or native ornithine (i.e., the primary amine in the side chain of the native lysine or native ornithine), or between the second reactive group in the hydrophobic tail precursor and the (first) reactive group in the modified lysine or modified ornithine.
19. The amphiphilic molecule according to items 17 or 18, wherein the second reactive group in the hydrophobic tail precursor is an aldehyde, and wherein the aldehyde is selected from the group consisting of myristoleyl aldehyde, palmitoleyl aldehyde, and petroselinyl aldehyde.
20. The amphiphilic molecule according to item 19, wherein
   - the peptide is Ac-RK^{*}HRK^{*}K^{*}H-NH₂ (SEQ ID NO: 1) or Ac-RKLRK*K*L-NH₂ (SEQ ID NO: 11), or salts thereof, the spacers connecting each of the reactive groups in the side chain of the peptide and each of the ε-amino groups in the lysine residues are formed by reacting the peptide with at least three molecules of glutaric acid monohydrazide per molecule of peptide, thereby obtaining a (poly)hydrazide-activated peptide, and then contacting the (poly)hydrazide-activated peptide with palmitoleyl aldehyde or myristoleyl aldehyde as hydrophobic tail precursor; or
   - the peptide is Ac-RRLK*RLK*RRLK*RL-NH₂ (SEQ ID NO: 14), or a salt thereof, the spacers connecting each of the reactive groups in the side chain of the peptide and each of the ε-amino groups in the lysine residues are formed by reacting the peptide with at least three molecules of glutaric acid monohydrazide per molecule of peptide, thereby obtaining a (poly)hydrazide-activated peptide and then contacting the (poly)hydrazide-activated peptide with myristoleyl aldehyde, palmitoleyl aldehyde or petroselinyl aldehyde as hydrophobic tail precursor; or
   - the peptide is selected from the group consisting of Ac-RO*HRO*O*H-NH₂ (SEQ ID NO: 52), Ac-RRK^{*}HRK^{*}K^{*-}NH₂ (SEQ ID NO: 53), Ac-RRK^{*}HRK^{*}K^{*}H-NH₂ (SEQ ID NO: 54), Ac-RRK^{*}LRK^{*}K^{*-}NH₂ (SEQ ID NO: 55), Ac-RRK^{*}LRK^{*}K^{*}L-NH₂ (SEQ ID NO: 56), Ac-RRRK*LRK*K*L-NH₂ (SEQ ID NO: 57), Ac-RO^{*}HRK^{*}K^{*}H-NH₂ (SEQ ID NO: 58) and Ac-RK*HRK*O*H-NH₂ (SEQ ID NO: 59), or salts thereof, the spacers connecting each of the reactive groups in the side chain of the peptide and each of the ε-amino groups in the lysine residues or δ-amino groups in the ornithine residues are formed by reacting the peptide with at least three molecules of glutaric acid monohydrazide per molecule of peptide, thereby obtaining a (poly)hydrazide-activated peptide, and then contacting the (poly)hydrazide-activated peptide with myristoleyl aldehyde as hydrophobic tail precursor.
21. A complex comprising
   a) at least one amphiphilic molecule as defined in any one of items 14 to 20, and
   b) at least one molecule of biological interest.
22. The complex according to item 21, wherein the molecule of biological interest is selected from the group consisting of a protein, a nucleic acid, a nucleoprotein, a small molecule and an immunogen.
23. The complex according to items 21 or 22 wherein the molecule of biological interest has negative net charge.
24. The complex according to item 22, wherein the nucleic acid is pDNA, siRNA, or mRNA, and wherein the nucleoprotein is a ribonucleoprotein.
25. Use of a peptide or salt thereof as defined in any one of items 1 to 13, an amphiphilic molecule as defined in any one of items 14 to 20, or a complex as defined in any one of items 21 to 24, for the *in vitro* delivery to a cell of a molecule of biological interest.
26. The use according to item 25, wherein the molecule of biological interest is selected from the group consisting of a protein, a nucleic acid, a nucleoprotein and a small molecule.
27. The use according to items 25 or 26, wherein the nucleic acid is pDNA, siRNA, or mRNA, and wherein the nucleoprotein is a ribonucleoprotein.
28. A peptide or salt thereof as defined in any one of items 1 to 13, an amphiphilic molecule as defined in any one of items 14 to 20, and/or a complex as defined in any one of items 21 to 24 for use as a medicament.
29. A vaccine comprising a peptide or salt thereof as defined in any one of items 1 to 13, an amphiphilic molecule as defined in any one of items 14 to 20 and/or a complex as defined in any one of items 21 to 24.
30. A method for the preparation of an amphiphilic molecule as defined in any one of items 14 to 20, wherein the method comprises contacting a solution of the peptide or salt thereof as defined in any of items 1 to 13 in an organic solvent with a solution of a precursor of the hydrophobic tail comprising a second reactive group which reacts with the reactive group in the side chain of the peptide under conditions adequate for the formation of a covalent bond between the second reactive group of the precursor of the hydrophobic tail and the reactive group in the peptide, wherein said conditions are the following:
   (i) acidic media, when the reactive group in the hydrophobic tail precursor is an aldehyde, or
   (ii) basic media and in the presence of a carboxylic acid activator reagent, when the reactive group in the hydrophobic tail precursor is an alcohol or a carboxylic acid.
31. A method for the preparation of a complex as defined in any one of items 21 to 24, wherein the method comprises contacting a solution of at least one amphiphilic molecule as defined in any of items 14 to 20 with a solution of at least one molecule of biological interest in a suitable medium under conditions adequate for the formation of a complex between at least one amphiphilic molecule and at least one molecule of biological interest.
32. A method for the *in vitro* delivery of a molecule of biological interest to a cell population comprising
   (i) contacting a first solution of at least one amphiphilic molecule as defined in any of items 14 to 20 in a suitable solvent and a second solution containing the molecule of biological interest in suitable solvent under conditions adequate for the formation of a complex between the at least one amphiphilic molecule and the at least one molecule of biological interest and
   (ii) adding the complex obtained in step (i) to the cell population under conditions adequate for the delivery of the molecule of biological interest to the cell population.
33. The method of item 32 wherein the contacting step (i) is carried out in the same medium in which the cells are cultured and wherein the amphiphilic molecule is provided from a stock solution in an organic solvent.
34. The method according to any of items 31 to 33 wherein the molecule of biological interest has a negative net charge at the pH at which the contacting step (i) is carried out.
35. A method for obtaining a library of amphiphilic molecules as defined in any one of items 14 to 20, the method comprising contacting "n" peptides or salts thereof as defined in items 1 to 13 in an organic solvent with at least "3n" hydrophobic tail precursors, said precursors comprising a hydrophobic tail and a second reactive group which reacts with the reactive group in the side chain of the reactive basic amino acid in the peptide or salt thereof, wherein the contacting is carried out under conditions adequate for the formation of a covalent bond between the hydrophobic tail precursors and the reactive groups within the peptide or salt thereof.
36. The method according to item 35, wherein the at least two peptides or salts thereof are independently selected from any one of SEQ ID NOs 1-19, 21, 23, 49, 52-59.
37. The method according to items 35 or 36, wherein the hydrophobic tail comprised in the at least "3n" hydrophobic tail precursors are selected from an aldehyde, a carboxylic acid or an alcohol, or mixtures thereof, preferably an aldehyde, wherein the aldehyde is selected from the group consisting of hexanal, octanal, decanal, dodecanal, myristoleyl aldehyde, palmitoleyl aldehyde, petroselinyl aldehyde, oleyl aldehyde, linoleoyl aldehyde, eicosenoyl aldehyde, tetracosenoleyl aldehyde, or mixtures thereof; wherein the carboxylic acid is selected from the group consisting of hexanoic acid, octanoic acid, decanoic acid, dodecanoic acid, myristoleic acid, palmitoleic acid, petroselinic acid, oleic acid, linoleic acid, eicosenoic acid,tetracosenoleic acid, or mixtures thereof; and wherein the alcohol is selected from the group consisting of hexanol, octanol, decanol, dodecanol, myristoleyl alcohol, palmitoleyl alcohol, petroselinyl alcohol, oleyl alcohol, linoleoyl alcohol, gondoyl alcohol tetracosenoleyl alcohol, or mixtures thereof, preferably wherein the hydrophobic tail comprised in the at least "3n" tail precursors are selected from dodecanal, myristoleyl, palmitoleyl, petroselinyl, oleyl, eicosenoyl, tetracosenoleyl aldehydes, or mixtures thereof.
38. The method according to any of items 35 to 37, wherein the reactive group in the peptide as defined in items 1 to 13 is a hydrazide group or an aminooxy-carboxylic acid group, and wherein the reactive group in the hydrophobic tail precursors is an aldehyde group.
39. A library comprising at least two different amphiphilic molecules, each amphiphilic molecule comprising
   (i) a peptide or salt thereof as defined in any one of items 1 to 13 and
   (ii) three hydrophobic tails,
   wherein the hydrophobic tails are connected to the peptide by a bond formed by a reaction between the reactive group in the side chain of the reactive basic amino acid residues of the peptide and a precursor of the hydrophobic tails, wherein each precursor of the hydrophobic tail contains said hydrophobic tail and a second reactive group which can react with the reactive group in the side chain of the reactive basic amino acid residue in the peptide, and wherein one amphiphilic molecule differs at least from another amphiphilic molecule in the peptide and/or in the hydrophobic tails.
40. The library according to item 39 wherein each amphiphilic molecule is obtained by reacting the peptide or salt thereof with at least three precursors of the hydrophobic tails, said precursors comprising a second reactive group which reacts with the reactive group in the reactive basic amino acid residue, thereby forming a covalent bond.
41. The library according to items 39 or 40, wherein the bond formed by a reaction between the reactive group in the side chain of the reactive basic amino acid residues of the peptide or salt thereof and the precursors of the hydrophobic tails which contain said hydrophobic tail and a second reactive group which can react with the reactive group in the side chain of the reactive basic amino acid residue in the peptide or salt thereof is an amide bond, an ester bond, a hydrazone bond or an oxime bond.
42. A method for the identification of an amphiphilic molecule suitable for the delivery of a molecule of biological interest into a cell, the method comprising:
   (i) contacting the molecule of biological interest with the library as defined in any of items 39 to 41 under conditions adequate for the binding of the amphiphilic molecule and the molecule of biological interest;
   (ii) optionally selecting the amphiphilic molecules that are capable of binding to the molecule of biological interest from those amphiphilic molecules which do not bind to the molecule of biological interest, and/or
   (iii) screening for an amphiphilic molecule which is suitable for the delivery of the molecule of biological interest into a cell.
43. The method according to item 42, wherein the molecule of biological interest is selected from the group consisting of a protein, a nucleic acid, a nucleoprotein and a small molecule.

## Claims

1. An amphiphilic molecule comprising:
a) A peptide or salt thereof:
▪ wherein the peptide or salt thereof has a length of 7 to 21 amino acids,
▪ wherein the peptide or salt thereof comprises basic amino acid residues selected from the group consisting of arginine (R), histidine (H) and reactive basic amino acid residues containing a reactive group on its side chain,
▪ wherein the peptide or salt thereof optionally comprises one or more hydrophobic amino acid residues selected from the group consisting of alanine (A), valine (V), leucine (L) and isoleucine (I), wherein, if present, the hydrophobic amino acid residues represent less than 50% of the total number of amino acids in the peptide,
▪ wherein the number of arginines in the peptide or salt thereof is from 2 to 10,
▪ wherein the number of histidines in the peptide or salt thereof is from 0 to 3,
▪ wherein the number of reactive basic amino acid residues containing a reactive group in its side chain in the peptide or salt thereof is 3,
▪ wherein the number of hydrophobic amino acids in the peptide or salt thereof is 0 to 8;
▪ wherein in the peptide amino acid sequence there are not more than two consecutive amino acids of the same residue, except for the reactive basic amino acid residue containing a reactive group in its side chain and the amino acid arginine, in which case there can be a maximum of three consecutive arginines and/or a maximum of three consecutive reactive basic amino acid residues containing a reactive group in its side chain;
▪ wherein the amino acids comprised in the peptide or salt thereof are L-amino acids and/or D-amino acids, preferably wherein all of the amino acids comprised in the peptide are L-amino acids or all of the amino acids comprised in the peptide are D-amino acids;
▪ wherein the reactive basic amino acid residue containing a reactive group on its side chain is as defined in Formula (I): 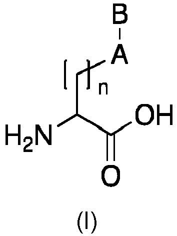
wherein:
B may be present or not; if B is not present, A is a reactive group, preferably a primary amine; if B is present, A is selected from an amide bond, a secondary amine or any other functional group, and B is a spacer linked to a reactive group; n is 1, 2, 3, 4, 5, 6, 7, 8, or more, preferably 1, 2, 3, or 4; and
b) Three hydrophobic tails,
wherein each hydrophobic tail is connected to the peptide or salt thereof by a bond formed by a reaction between (i) the reactive group in the side chain of the reactive basic amino acid residue of the peptide and (ii) a precursor of the hydrophobic tail, wherein the precursor of the hydrophobic tail comprises the hydrophobic tail and a second reactive group which can react with the reactive group in the side chain of the reactive basic amino acid residue in the peptide.

2. The amphiphilic molecule according to claim 1, wherein the three reactive basic amino acid residues containing a reactive group in its side chain comprised in the peptide or salt thereof are reactive lysines (K*), reactive ornithines (O*) or mixtures thereof.

3. The amphiphilic molecule according to claim 2, wherein:
(i) the reactive lysine (K*) comprised in the peptide or salt thereof is a native lysine or a modified lysine, wherein the modified lysine is **characterized in that** the reactive group is connected to the ε-amino group in the reactive lysine by an amide bond, said reactive group and amide bond being connected by a spacer; and
(ii) the reactive ornithine (O*) comprised in the peptide or salt thereof is a native ornithine or a modified ornithine, wherein the modified ornithine is **characterized in that** the reactive group is connected to the δ-amino group in the ornithine by an amide bond, said reactive group and amide bond being connected by a spacer.

4. The amphiphilic molecule according to any one of claims 1-3, wherein the reactive group in the side chain of the reactive basic amino acid residue comprised in the peptide or salt thereof is a hydrazide group.

5. The amphiphilic molecule according to any one of claims 1 to 4, wherein the peptide or salt thereof comprised therein has a length of 7 amino acids, wherein the peptide or salt thereof consists of basic amino acids selected from arginines, histidines and reactive basic amino acid residues containing a reactive group, wherein the number of arginines in the peptide or salt thereof is at least 2 and wherein the number of histidines in the peptide or salt thereof is 2 or less.

6. The amphiphilic molecule according to claim 5, wherein the peptide sequence is selected from the group consisting of Ac-RK*HRK*K*H-NH₂ (SEQ ID NO: 1), Ac-RHK*K*K*HR-NH₂ (SEQ ID NO: 2), Ac-RK*HHK*K*R-NH₂ (SEQ ID NO: 3), Ac-RO*HRO*O*H-NH₂ (SEQ ID NO: 52), Ac-RRK*HRK*K*-NH₂ (SEQ ID NO: 53); Ac-RO*HRK*K*H-NH₂ (SEQ ID NO: 58) and Ac-RK*HRK*O*H-NH₂ (SEQ ID NO: 59), or salts thereof.

7. The amphiphilic molecule according to any one of claims 1 to 4, wherein the peptide or salt thereof comprised therein has a length of at least 9 amino acids, wherein the peptide or salt thereof comprises a core sequence RX₁X₂R (SEQ ID NO: 9) or RX₁X₂RRX₃X₄ (SEQ ID NO: 4), and wherein any one of X₁ to X₄ is an amino acid selected from the group consisting of H, K*, O* and a hydrophobic amino acid, wherein the hydrophobic amino acid is selected from the group consisting of A, V, L and I.

8. The amphiphilic molecule according to claim 7, wherein the peptide or salt thereof comprised therein is a peptide of sequence RX₁X₂RRX₃X₄RX₅ (SEQ ID NO: 5), RRX₁X₂RX₃X₄RRX₅X₆RX₇ (SEQ ID NO: 6); RRX₁X₂RX₃X₄RRX₅X₆RX₇X₈RRX₉ (SEQ ID NO: 7), RRX₁X₂RX₃X₄RRX₅X₆RX₇X₈RRX₉RRLL (SEQ ID NO: 8) or RX₁X₂X₃RX₄X₅RRX₆X₇RX₈ (SEQ ID NO: 10), wherein any one of X₁ to X₉ is an amino acid selected from the group consisting of H, K*, O* and a hydrophobic amino acid, wherein the hydrophobic amino acid is selected from the group consisting of A, V, L and I.

9. The amphiphilic molecule according to claim 8, wherein the peptide sequence is selected from the group consisting of Ac-RK*LRRK*LRK*-NH₂ (SEQ ID NO: 13), Ac-RRLK*RLK*RRLK*RL-NH₂ (SEQ ID NO: 14), Ac-RRLK*RK*LRRLK*RL-NH₂ (SEQ ID NO: 15), Ac-RRK*K*RK*LRRLLRL-NH₂ (SEQ ID NO: 16), Ac-RRHK*RLK*RRLK*RL-NH₂ (SEQ ID NO: 17), Ac-RHLK*RLK*RRLK*RL-NH₂ (SEQ ID NO: 18), Ac-RHLK*RHK*RRLK*RH-NH₂ (SEQ ID NO: 19), Ac-RRLK*RLLRRLK*RLK*RRL-NH₂ (SEQ ID NO: 21), and Ac-RRLK*RLLRRLK*RLK*RRLRRLL-NH₂ (SEQ ID NO: 23), or salts thereof.

10. The amphiphilic molecule according to any of claims 1 to 4, wherein the peptide sequence is selected from the group consisting of Ac-RK*LRK*K*L-NH₂ (SEQ ID NO: 11), Ac-RK*RRK*K*R-NH₂ (SEQ ID NO: 12), Ac-RK*HK*HK*R-NH₂ (SEQ ID NO: 49), Ac-RK*LRRK*LRK*-NH₂ (SEQ ID NO: 13), Ac-RRK*HRK*K*H-NH₂ (SEQ ID NO: 54), Ac-RRK*LRK*K*-NH₂ (SEQ ID NO: 55), Ac-RRK*LRK*K*L-NH₂ (SEQ ID NO: 56) and Ac-RRRK*LRK*K*L-NH₂ (SEQ ID NO: 57), or salts thereof.

11. The amphiphilic molecule according to any of claims 1 to 10, wherein the peptide comprised therein is *N*-acylated and/or C-amidated.

12. The amphiphilic molecule according to any one of claims 1-11, wherein the bond formed by a reaction between the reactive group in the side chain of the reactive basic amino acid residue and the second reactive group in the precursor of the hydrophobic tail is a hydrazone bond.

13. The amphiphilic molecule according to any one of claims 1-12, wherein the second reactive group in the hydrophobic tail precursor is an aldehyde, and wherein the aldehyde is selected from hexanal, octanal, decanal, dodecanal, myristoleyl aldehyde, palmitoleyl aldehyde, petroselinyl aldehyde, oleyl aldehyde, linoleoyl aldehyde, eicosenoyl aldehyde and tetracosenoleyl aldehyde, preferably from myristoleyl aldehyde, palmitoleyl aldehyde, and petroselinyl aldehyde.

14. The amphiphilic molecule according to any one of claims 1-13, wherein
- the peptide is selected from Ac-RK*HRK*K*H-NH₂ (SEQ ID NO: 1) or Ac-RK*LRK*K*L-NH₂ (SEQ ID NO: 11) or salts thereof and the spacers connecting each of the reactive groups in the side chain of the peptide and each of the ε-amino groups in the lysine residues are formed by reacting the peptide with glutaric acid monohydrazide, thereby obtaining a hydrazide activated peptide, and then with palmitoleyl aldehyde or myristoleyl aldehyde as hydrophobic tail precursor; or
- the peptide is Ac-RRLK*RLK*RRLK*RL-NH₂ (SEQ ID NO: 14) or a salt thereof and the spacers connecting each of the reactive groups in the side chain of the peptide and each of the ε-amino groups in the lysine residues are formed by reacting the peptide with glutaric acid monohydrazide, thereby obtaining a hydrazide activated peptide and then with palmitoleyl aldehyde or petroselinyl aldehyde as hydrophobic tail precursor; or
- the peptide is selected from the group consisting of Ac-RO*HRO*O*H-NH₂ (SEQ ID NO: 52), Ac-RRK*HRK*K*-NH₂ (SEQ ID NO: 53), Ac-RRK*HRK*K*H-NH₂ (SEQ ID NO: 54), Ac-RRK*LRK*K*-NH₂ (SEQ ID NO: 55), Ac-RRK*LRK*K*L-NH₂ (SEQ ID NO: 56), Ac-RRRK*LRK*K*L-NH₂ (SEQ ID NO: 57), Ac-RO*HRK*K*H-NH₂ (SEQ ID NO: 58) and Ac-RK*HRK*O*H-NH₂ (SEQ ID NO: 59), or salts thereof, the spacers connecting each of the reactive groups in the side chain of the peptide and each of the ε-amino groups in the lysine residues or δ-amino groups in the ornithine residues are formed by reacting the peptide with glutaric acid monohydrazide, thereby obtaining a hydrazide activated peptide, and then with myristoleyl aldehyde as hydrophobic tail precursor.

15. A complex comprising
(i) at least one amphiphilic molecule as defined in any one of claims 1 to 14, and
(ii) at least one molecule of biological interest,
wherein preferably the molecule of biological interest is selected from a protein, a nucleic acid, a nucleoprotein, an immunogen and a small molecule, preferably wherein the nucleic acid is pDNA, siRNA, or mRNA, and the nucleoprotein is a ribonucleoprotein.

16. A peptide or salt thereof suitable for forming an amphiphilic molecule as defined in any one of claims 1 to 14:
▪ wherein the peptide or salt thereof has a length of 7 to 21 amino acids,
▪ wherein the peptide or salt thereof comprises basic amino acid residues selected from the group consisting of arginine (R), histidine (H) and reactive basic amino acid residues containing a reactive group on its side chain,
▪ wherein the peptide or salt thereof optionally comprises one or more hydrophobic amino acid selected from the group consisting of alanine (A), valine (V), leucine (L) and isoleucine (I), wherein, if present, the hydrophobic amino acid represent less than 50% of the total number of amino acids in the peptide,
▪ wherein the number of arginines in the peptide or salt thereof is from 2 to 10,
▪ wherein the number of histidines in the peptide or salt thereof is from 0 to 3,
▪ wherein the number of reactive basic amino acid residues containing a reactive group in its side chain in the peptide or salt thereof is 3,
▪ wherein the number of hydrophobic amino acids in the peptide is or salt thereof 0 to 8;
▪ wherein in the peptide amino acid sequence there are not more than two consecutive amino acids of the same residue, except for the reactive basic amino acid residue containing a reactive group in its side chain and the amino acid arginine, in which cases there can be a maximum of three consecutive arginines and/or a maximum of three consecutive reactive basic amino acid residues containing a reactive group in its side chain;
▪ wherein the amino acids comprised in the peptide or salt thereof are L-amino acids and/or D-amino acids, preferably wherein all of the amino acids comprised in the peptide are L-amino acids or all of the amino acids comprised in the peptide are D-amino acids;
▪ wherein the reactive basic amino acid residue containing a reactive group on its side chain is as defined in Formula (I): 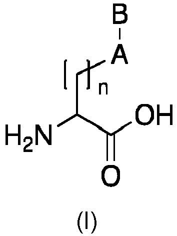 wherein:
B may be present or not; if B is not present, A is a reactive group, preferably a primary amine; if B is present, A is selected from an amide bond, a secondary amine or any other functional group and B is a spacer linked to a reactive group; n is 1, 2, 3, 4, 5, 6, 7, or 8, preferably 1, 2, 3, or 4.

17. An amphiphilic molecule as defined in any one of claims 1-14, a complex as defined in claim 15 and/or a peptide as defined in claim 16 for use as a medicament.

18. A vaccine comprising an amphiphilic molecule as defined in any one of claims 1-14, a complex as defined in claim 15 and/or a peptide as defined in claim 16.
